(19) Europäisches Patentamt
European Patent Office
Office européen des brevets

(11) **EP 4 157 439 B1**

(12) **EUROPEAN PATENT SPECIFICATION**

(45) Date of publication and mention
of the grant of the patent:
**19.11.2025 Bulletin 2025/47**

(51) International Patent Classification (IPC):
***A61N 1/39*** *(2006.01)*       *A61N 1/378* *(2006.01)*
***A61N 1/37*** *(2006.01)*       *A61N 1/362* *(2006.01)*

(21) Application number: **21735017.2**

(52) Cooperative Patent Classification (CPC):
**A61N 1/3912; A61N 1/3904; A61N 1/3937;**
**A61N 1/3981;** A61N 1/3621; A61N 1/3625;
A61N 1/3706; A61N 1/378

(22) Date of filing: **26.05.2021**

(86) International application number:
**PCT/US2021/034287**

(87) International publication number:
**WO 2021/242882 (02.12.2021 Gazette 2021/48)**

(54) **ELECTROTHERAPEUTIC WAVEFORM AND PULSE GENERATION AND DELIVERY SYSTEM**

ELEKTROTHERAPEUTISCHES WELLENFORM- UND IMPULSERZEUGUNGS- UND ABGABESYSTEM

SYSTÈME DE GÉNÉRATION ET DE DÉLIVRANCE DE FORMES D'ONDE ET D'IMPULSIONS ÉLECTROTHÉRAPEUTIQUES

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **26.05.2020   US 202062704734 P**

(43) Date of publication of application:
**05.04.2023   Bulletin 2023/14**

(73) Proprietor: **ZOLL Medical Corporation**
**Chelmsford, MA 01824-4105 (US)**

(72) Inventors:
• **BUTLER, Gideon D.H.**
**Portsmouth, New Hampshire 03801 (US)**
• **WASERMAN, Boaz Bronislav**
**Brookline, Massachusetts 02446 (US)**
• **LESNIEWSKI-LAAS, Nicholas Gryf**
**Somerville, Massachusetts 02144 (US)**

(74) Representative: **Potter Clarkson**
**Chapel Quarter**
**Mount Street**
**Nottingham NG1 6HQ (GB)**

(56) References cited:
EP-A- 0 712 321       EP-A1- 1 535 645
JP-A- H1 057 508      US-A1- 2001 031 991
US-A1- 2003 074 025

## Description

## BACKGROUND

Electrotherapies, which can include delivery of electrical energy or current to a patient, may be used, for example, in connection with treatments relating to organs such as the human heart. Normally, electro-chemical activity within a human heart causes the organ's muscle fibers to contract and relax in a synchronized manner. This synchronized action of the heart's musculature results in the effective pumping of blood from the ventricles throughout the body, including to the vital organs. Certain conditions can disrupt this normal electrochemical activity, synchronized action and effective pumping of the heart. These conditions include cardiac arrhythmias, which can cause the heart to beat irregularly or abnormally. Electrotherapies, including defibrillation and pacing, for example, can be useful or lifesaving in connection with conditions such as cardiac arrhythmias.

**In** particular, one of the most deadly cardiac arrhythmias is ventricular fibrillation, during which abnormal electrical activity within the heart causes the individual muscle fibers to contract in an unsynchronized and chaotic way. As a result of this loss of synchronization, the heart can rapidly lose its ability to effectively pump blood.

Defibrillation is a type of electrotherapy that may be used to treat conditions including ventricular fibrillation. A defibrillator may produce a large shock for delivery to a patient that disrupts the chaotic electrical activity of the heart associated with ventricular fibrillation, which can allow the heart's electro-chemical system to re-synchronize itself. Once organized electrical activity is restored, synchronized muscle contractions often follow, leading to the restoration of effective cardiac pumping.

EP 1535645 A1 disclose a method and circuit for forming an electrotherapy current wave form. A charge storage device located externally of a patient's body is charged, and is discharged through the patient's body through at least two discharge electrodes connected by electrical circuitry to opposite poles of the charge storage device. A continuous discharge of the charge storage device through the electrodes is controlled so as to produce at least one phase of a current wave form that includes a ripple. The ripple has a height less than one third of the height of the peak current of the phase. The current wave form has a sensing pulse portion that is integral with the therapeutic discharge portion of the current wave form. US 2001031991 A1 discloses a circuit for producing an arbitrary defibrillator waveform using switching techniques which reduce the usual high current or high voltage stress on the switching element. EP 0712321 A1 discloses an electrotherapy method and apparatus for delivering a multiphasic waveform from an energy source to a patient. The method comprises the steps of charging the energy source to an initial level; discharging the energy source across the electrodes to deliver electrical energy to the patient in a multiphasic waveform; monitoring a patient-dependent electrical parameter during the discharging step; shaping the waveform of the delivered electrical energy based on a value of the monitored electrical parameter, wherein the relative duration of the phases of the multiphasic waveform is dependent on the value of monitored electrical parameter. US 2003/074025 A1 discloses a defibrillation method and apparatus for delivering a desired quantity of electric charge to a patient. The method includes determining one or more intended waveform parameters based on the desired quantity of electric charge and, optionally, on one or more patient impedance values. A defibrillation waveform is then generated and applied to the patient based on the intended waveform parameters. JP H10 57508 A discloses selecting a desired waveform from those stored in a memory and providing the selected waveform to a waveform generator. The signal of the waveform generator is given to a modulator and provides a switching signal for controlling the duty cycle of the switch of a charging/discharging circuit that acts in the same way as a switching regulator. Further, the impedance value of a patient is obtained from an impedance sensor and by controlling the switching signal, the scaling of a defibrillation signal is carried out.

## **SUMMARY** OF THE INVENTION

The invention provides a system as defined in claim 1. Preferred embodiments are defined in the dependent claims. The methods disclosed herein do not form part of the claimed invention.

## SUMMARY OF THE DISCLOSURE

One example of a system for generating an electrotherapeutic pulse to be delivered to a patient, the system includes: an energy storage capacitor for providing electrotherapeutic current to the patient; a therapeutic current control network, electrically coupled to the energy storage capacitor, for controlling the electrotherapeutic current to be delivered to the patient, the therapeutic current control network comprising a resonant electrical circuit and at least one current control switch, wherein energy provided by the energy storage capacitor flows through the resonant electrical circuit; and a controller electrically coupled to the energy storage capacitor and the therapeutic current control network, wherein the controller is configured to, in connection with the energy provided by the energy storage capacitor that flows through the resonant electrical circuit, control operation of the at least one current control switch of the therapeutic current control network in delivering an electrotherapeutic waveform to the patient to correspond with a specified waveform.

In some examples, the system comprises at least one sensor configured to sense at least one electrical parameter from which current flow to the patient can be determined or estimated, wherein the controller is configured to: process a signal associated with the sensed at least one electrical parameter; compare the processed signal with a second signal associated with the specified waveform; and control operation of the at least one current control switch of the therapeutic current control network in adjusting delivery of the electrotherapeutic waveform to the patient to correspond with the specified waveform.

In some examples, the at least one current control switch comprises a plurality of switches, and wherein the control of the operation of the at least one current control switch of the therapeutic current control network in the adjusting of the delivery of the electrotherapeutic waveform to the patient to correspond with the specified waveform comprises controlling a configuration of each of the plurality of switches as open or closed. In some examples, the plurality of switches comprises at least one H-bridge. In some examples, the configuration of each of the plurality of switches as open or closed affects a level of current delivered to the patient. In some examples, the resonant electrical circuit comprises a resonant tank. In some examples, the sensed at least one electrical parameter comprises a current parameter. In some examples, the sensed at least one electrical parameter comprises a voltage parameter. In some examples, the controller comprises at least one processor. In some examples, the controller is digital. In some examples, the controller is analog.

In some examples, the therapeutic current control network comprises a rectifier, and wherein: the energy storage capacitor and the at least one current control switch are connected via a first node and a second node; the at least one current control switch and the resonant electrical circuit are connected via a third node and a fourth node; and the resonant electrical circuit and the rectifier are connected via a fifth node and a sixth node.

In some examples, the system comprises a battery and a bidirectional charging control network, wherein: the battery and the bidirectional charging control network are connected via a first node and a second node, and the bidirectional charging control network and the energy storage capacitor are connected via a third node and a fourth node, wherein: the bidirectional charging control network is for controlling energy flow from the battery to the energy storage capacitor for storage by the energy storage capacitor, and from the energy storage capacitor to the battery for storage by the battery. In some examples, the system comprises a battery and a bidirectional charging control network, wherein: the battery and the bidirectional charging control network are connected via a seventh node and a eighth node, and the bidirectional charging control network and the energy storage capacitor are connected via a ninth node and a tenth node, wherein: the bidirectional charging control network is for controlling energy flow from the battery to the energy storage capacitor for storage by the energy storage capacitor, and from the energy storage capacitor to the battery for storage by the battery. In some examples, the bidirectional charging control network comprises a second resonant electrical circuit. In some examples, the second resonant electrical circuit comprises a resonant tank. In some examples, the rectifier and the filter are connected via an eleventh node and a twelfth node. In some examples, the system comprises at least one polarity control switch, wherein the filter and the at least one polarity control switch are connected via an thirteenth node and a fourteenth node. In some examples, the at least one polarity control switch comprises an H-bridge. In some examples, the at least one sensor is connected with the at least one polarity control switch via a fifteenth node and a sixteenth node.

In some examples, the at least one current control switch comprises a plurality of switches, and wherein the control of the operation of the at least one current control switch of the therapeutic current control network in the adjusting of the delivery of the electrotherapeutic waveform to the patient to correspond with the specified waveform comprises controlling a configuration of each of the plurality of switches as open or closed. In some examples, the configuration of each of the plurality of switches as open or closed affects a level of current delivered to the patient. In some examples, the controller is configured to control operation of the at least one current control switch of the therapeutic current control network to stagger a time of initiation of current switching relative to a time of initiation of voltage switching, the voltage switching being associated with the current switching, so as to reduce switching losses relative to substantially simultaneous initiation of the current switching and the associated voltage switching. In some examples, the controller is configured to control timing of voltage switching waveforms relative to associated current switching waveforms to minimize the switching losses. In some examples, the controller is configured to control the timing of voltage switching waveforms relative to associated current switching waveforms to minimize the switching losses and to reduce electromagnetic interference (EMI). In some examples, the controller is configured to control operation of the at least one current control switch to provide substantially zero voltage switching (ZVS) and substantially zero current switching (ZCS). In some examples, the at least one current control switch comprises a semiconductor material with a band gap of between 2 and 6 eV. In some examples, the at least one current control switch comprises at least one of: silicon carbide (SiC) and gallium nitride (GaN).

In some examples, a peak energy efficiency of the system in delivering the electrotherapeutic waveform to the patient is at least 80, 85 or 90 percent. In some examples, the electrotherapeutic waveform delivered to the patient delivers an amount of energy to the patient that is no more than 15, 10 or 5 percent different than an amount of energy of the specified waveform. In some examples, the electrotherapeutic waveform delivered to the patient delivers an average amount of energy to the patient that is no more than 3 percent different than an average amount of energy of the specified waveform. In some examples, the system comprises an electrical circuit comprising the energy storage capacitor, the therapeutic current control network, the at least one sensor and the controller. In some examples, the at least one sensor comprises a current sensor and a voltage sensor. In some examples, the specified waveform is a biphasic rectilinear current waveform. In some examples, the system comprises at least one polarity control switch, wherein the at least one polarity control switch is used in controlling direction of current flow in delivering the biphasic rectilinear current waveform to the patient. In some examples, the controller is configured to receive input specifying the specified waveform. In some examples, the specified waveform is a current waveform. In some examples, the controller is configured to accept data specifying the specified waveform. In some examples, the controller is configured to store data specifying the specified waveform.

In some examples, the system is configured to provide closed-loop control of the delivery of the electrotherapeutic waveform to the patient. In some examples, the system is configured to monitor, and provide continuous adjustment over time, of the delivery of the electrotherapeutic waveform to the patient. In some examples, the system is configured to monitor impedance of the patient over time during the delivery of the electrotherapeutic waveform to the patient.

In some examples, the resonant electrical circuit is a two element resonant tank. In some examples, the two element resonant tank comprises an inductor and a capacitor. In some examples, the inductor is connected in series and the capacitor is connected in parallel. In some examples, the resonant electrical circuit is a three element resonant tank. In some examples, the resonant electrical circuit is a four element resonant tank. In some examples, the resonant electrical circuit is an LCC resonant tank. In some examples, the resonant electrical circuit is a CLL resonant tank. In some examples, the resonant electrical circuit is an LLC resonant tank. In some examples, the resonant electrical circuit is an LCLC resonant tank. In some examples, the resonant electrical circuit comprises two inductors and one capacitor.

In some examples, the rectifier comprises a switching network configured to be controlled to perform synchronous rectification. In some examples, the rectifier comprises a diode network configured to be controlled to perform passive rectification. In some examples, the controller comprises a field-programmable gate array (FPGA). In some examples, the controller comprises a digital signal processor (DSP). In some examples, the controller comprises a voltage controlled oscillator. In some examples, the controller comprises a switch timing latch. In some examples, the controller comprises a reference waveform signal generator. In some examples, the controller comprises a current sense amplifier. In some examples, the therapeutic current control network comprises a zero current detector. In some examples, the zero current detector is used in increasing an accuracy of the determined or estimated current flow to the patient. In some examples, the at least one current control switch is configured to be operated for use in adjusting a level of electrotherapeutic current being delivered to the patient.

In some examples, the resonant electrical circuit is configured for use in storing at least a portion of energy received by the therapeutic current control network from the energy storage capacitor. In some examples, the rectifier is configured for use in conversion of alternating current to direct current. In some examples, the filter is configured for use in electrical signal frequency filtering. In some examples, system comprises a polarity control network, wherein the polarity control network is configured for use in controlling direction of current flow. In some examples, the specified waveform is a predetermined current waveform. In some examples, the electrotherapeutic waveform is an electrotherapeutic current waveform. In some examples, the electrotherapeutic waveform is for defibrillation. In some examples, the electrotherapeutic waveform is for pacing. In some examples, the system comprises a driver circuit for use in control of operation of the at least one current control switch, wherein the driver circuit is connected to ground. In some examples, the control of the operation of the at least one current control switch of the therapeutic current control network in the adjusting of the delivery of the electrotherapeutic waveform to the patient to correspond with the specified waveform comprises controlling a switching frequency of the at least one current control switch.

In some examples, the energy storage capacitor and a first portion of the therapeutic current control network comprising a patient load and are connected via a first node; the first portion of the therapeutic current control network and a second portion of the therapeutic current control network comprising the resonant electrical circuit are connected via a second node; and the second portion of the therapeutic current control network and a third portion of the therapeutic current control network comprising the at least one current control switch are connected via a third node. In some examples, the third portion of the therapeutic current control network and the at least one sensor are connected via a fourth node. In some examples, the first portion of the therapeutic current control network comprises a filter and at least one polarity control switch. In some examples, the at least one polarity control switch comprises an H-bridge. In some examples, the at least one polarity control switch and the patient load are connected via a fifth node and a sixth node. In some examples the at least one current control switch comprises a high voltage MOSFET switch. In some examples, the driver circuit is configured as a cascode switch driver circuit.

In some examples, the system comprises: a battery; and a bidirectional charging control network, comprising: a first circuit connected with the battery, a second circuit connected with the energy storage capacitor, and a transformer, connected with the first circuit and the second circuit, for use in storing first energy from the battery and in transferring at least a portion of the stored first energy to the energy storage capacitor, and for use in storing second energy from the energy storage capacitor and in transferring at least a portion of the stored second energy to the battery. In some examples, at least one of the first circuit and the second circuit comprises at least one clamp circuit. In some examples, the at least one clamp circuit comprises an active clamp circuit. In some examples, the at least one clamp circuit comprises a passive clamp circuit.

In some examples, the system comprises: a battery; a bidirectional charging control network, comprising: a first circuit connected with the battery, a second circuit connected with the energy storage capacitor, and a transformer, connected with the first circuit and the second circuit, for use in storing first energy from the battery and in transferring at least a portion of the stored first energy to the energy storage capacitor, and for use in storing second energy from the energy storage capacitor and in transferring at least a portion of the stored second energy to the battery. In some examples, at least one of the first circuit and the second circuit comprises at least one clamp circuit. In some examples, the at least one clamp circuit comprises an active clamp circuit. In some examples, the at least one clamp circuit comprises a passive clamp circuit.

In some examples, the system comprises: a battery; and a bidirectional charging control network, comprising: a first circuit connected with the battery, a second circuit connected with the energy storage capacitor, and a transformer, connected with the first circuit and the second circuit, for use in storing first energy from the battery and in transferring at least a portion of the stored first energy to the energy storage capacitor, and for use in storing second energy from the energy storage capacitor and in transferring at least a portion of the stored second energy to the battery. In some examples, at least one of the first circuit and the second circuit comprises at least one clamp circuit. In some examples, the at least one clamp circuit comprises an active clamp circuit. In some examples, the at least one clamp circuit comprises a passive clamp circuit.

In some examples, the therapeutic current control network comprises a patient relay circuit used in control of allowing current flow through a patient load, wherein the patient relay circuit comprises at least one patient relay switch comprising a wide bandgap material. In some examples, the wide bandgap material comprises at least one of silicon carbide (SiC) and gallium nitride (GaN). In some examples, the at least one patient relay switch comprises at least one MOSFET. In some examples, the patient relay circuit comprises a plurality of driver circuits. In some examples, the plurality of driver circuits are connected in series. In some examples, the at least one patient relay switch comprises a plurality of patient relay switches. In some examples, the patient relay circuit has a maximum leakage current of less than 50uA, 40uA or 30uA.

In In some examples, the system comprises: a bidirectional charging control network, comprising: a first circuit, connected with a battery, and a transformer circuit, connected with the first circuit, wherein the transformer circuit is connected with the energy storage capacitor, wherein the bidirectional charging control network is configured to operate in a forward mode in a buck capacity, and in a reverse mode in a boost capacity, and wherein: in the forward mode, energy flows from the battery to the energy storage capacitor for storage by the energy storage capacitor, and in the reverse mode, energy flows from the energy storage capacitor to the battery for storage by the battery.

In some examples, the system comprises: a bidirectional charging control network, comprising: a first circuit, connected with a battery, and a transformer circuit, connected with the first circuit, wherein the transformer circuit is connected with the energy storage capacitor, wherein the bidirectional charging control network is configured to operate in a forward mode in a buck capacity, and in a reverse mode in a boost capacity, and wherein: in the forward mode, energy flows from the battery to the energy storage capacitor for storage by the energy storage capacitor, and in the reverse mode, energy flows from the energy storage capacitor to the battery for storage by the battery.

In some examples, the system comprises: a bidirectional charging control network, comprising: a first circuit, connected with a battery, and a transformer circuit, connected with the first circuit, wherein the transformer circuit is connected with the energy storage capacitor, wherein the bidirectional charging control network is configured to operate in a forward mode in a buck capacity, and in a reverse mode in a boost capacity, and wherein: in the forward mode, energy flows from the battery to the energy storage capacitor for storage by the energy storage capacitor, and in the reverse mode, energy flows from the energy storage capacitor to the battery for storage by the battery.

In some examples, the system includes at least one patient relay circuit comprising at least one MOSFET comprising a wide bandgap material. In some examples, the wide bandgap material comprises silicon carbide (SiC). In some examples, the at least one patient relay circuit comprises at least one MOSFET comprising a wide bandgap material. In some examples, the wide bandgap material comprises silicon carbide (SiC).

One example of a system for generating an electrotherapeutic pulse to be delivered to a patient, the system includes: an energy storage capacitor for providing electrotherapy current to be delivered to the patient; a therapeutic current control network, electrically coupled to the energy storage capacitor, for controlling the electrotherapy current to be delivered to the patient, the therapeutic current control network comprising: at least one current control switch, and a resonant electrical circuit comprising at least one inductor and at least one capacitor, wherein energy provided by the energy storage capacitor flows through the resonant electrical circuit; and a controller electrically coupled to the energy storage capacitor and the therapeutic current control network, wherein the controller is configured to, in connection with the energy provided by the energy storage capacitor that flows through the resonant electrical circuit, control operation of the at least one current control switch of the therapeutic current control network in delivering an electrotherapeutic waveform to the patient to correspond with a specified waveform.

In some examples, the system comprises at least one sensor, electrically coupled with the controller, configured to sense at least one electrical parameter from which current flow to the patient can be determined or estimated, and wherein the controller is configured to: process a signal associated with the sensed at least one electrical parameter; compare the processed signal with a second signal associated with the specified waveform; and control operation of the at least one current control switch of the therapeutic current control network in adjusting delivery of the electrotherapeutic waveform to the patient to correspond with the specified waveform. In some examples, the controller comprises at least one processor.

In some examples, the resonant electrical circuit stores at least a portion of energy received by the therapeutic current control network from the energy storage capacitor. In some examples, the resonant electrical circuit comprises a resonant tank. In some examples, the resonant electrical circuit is a two element resonant tank. In some examples, the two element resonant tank comprises an inductor and a capacitor. In some examples, the inductor is connected in series and the capacitor is connected in parallel. In some examples, the resonant electrical circuit is a three element resonant tank. In some examples, the resonant electrical circuit is a four element resonant tank. In some examples, the resonant tank is an LCC resonant tank. In some examples, the resonant electrical circuit is a CLL resonant tank. In some examples, the resonant electrical circuit is an LLC resonant tank. In some examples, the resonant electrical circuit is an LCLC resonant tank. In some examples, the resonant electrical circuit comprises a first inductor connected in series, a second inductor connected in parallel, and a capacitor connected in series.

In some examples, the system comprises a driver circuit for use in the control of the operation of the at least one current control switch, wherein the driver circuit is connected to ground. In some examples, the control of the operation of the at least one current control switch of the therapeutic current control network in the adjusting of the delivery of the electrotherapeutic waveform to the patient to correspond with the specified waveform comprises controlling a switching frequency of the at least one current control switch. In some examples, the energy storage capacitor and a first portion of the therapeutic current control network comprising a patient load and are connected via a first node; the first portion of the therapeutic current control network and a second portion of the therapeutic current control network comprising the resonant electrical circuit are connected via a second node; and the second portion of the therapeutic current control network and a third portion of the therapeutic current control network comprising the at least one current control switch are connected via a third node. In some examples, the third portion of the therapeutic current control network and the at least one sensor are connected via a fourth node. In some examples, the therapeutic current control network comprises a rectifier, and wherein: the energy storage capacitor and the at least one current control switch are connected via a first node and a second node; the at least one current control switch and the resonant electrical circuit are connected via a third and a fourth node; and the resonant electrical circuit and the rectifier are connected via a fifth node and a sixth node.

In some examples, the system comprises a battery and a bidirectional charging control network, wherein: the battery and the bidirectional charging control network are connected via a first node and a second node, and the bidirectional charging control network and the energy storage capacitor are connected via a third node and a fourth node, wherein: the bidirectional charging control network is for controlling energy flow from the battery to the energy storage capacitor for storage by the energy storage capacitor, and from the energy storage capacitor to the battery for storage by the battery.

In some examples, the system comprises: a battery; and a bidirectional charging control network, comprising: a first circuit connected with the battery, a second circuit connected with the energy storage capacitor, and a transformer, connected with the first circuit and the second circuit, for use in storing first energy from the battery and in transferring at least a portion of the stored first energy to the energy storage capacitor, and for use in storing second energy from the energy storage capacitor and in transferring at least a portion of the stored second energy to the battery. In some examples, at least one of the first circuit and the second circuit comprises at least one clamp circuit.

In some examples, the system comprises: a bidirectional charging control network, comprising: a first circuit, connected with a battery, and a transformer circuit, connected with the first circuit, wherein the transformer circuit is connected with the energy storage capacitor, wherein the bidirectional charging control network is configured to operate in a forward mode in a buck capacity, and in a reverse mode in a boost capacity, and wherein: in the forward mode, energy flows from the battery to the energy storage capacitor for storage by the energy storage capacitor, and in the reverse mode, energy flows from the energy storage capacitor to the battery for storage by the battery.

In some examples, the therapeutic current control network comprises a patient relay circuit used in control of allowing current flow through a patient load, wherein the patient relay circuit comprises at least one patient relay switch comprising a wide bandgap material. In some examples, the wide bandgap material comprises at least one of silicon carbide (SiC) and gallium nitride (GaN). In some examples, the at least one patient relay switch comprises at least one MOSFET. In some examples, the patient relay circuit comprises a plurality of driver circuits. In some examples, the plurality of driver circuits are connected in series.

One example of a method for generating an electrotherapeutic pulse for delivery to a patient includes: specifying a desired electrotherapeutic waveform for delivery to the patient, wherein energy for electrotherapy current is provided by an energy storage capacitor; and operating a resonant therapeutic current control network, comprising at least one current control switch and a resonant tank, in controlling an electrotherapeutic waveform being delivered to the patient to correspond to the desired electrotherapeutic waveform, wherein energy provided by the energy storage capacitor flows through the resonant tank.

In some examples, the method includes determining a current at a point in the resonant therapeutic current control network from which a current flow to the patient can be determined or estimated; and comparing the current flow to the patient to the desired electrotherapeutic waveform; wherein the operating of the resonant therapeutic current control network comprises operating the at least one current control switch in adjusting the electrotherapeutic waveform being delivered to the patient to match the desired electrotherapeutic waveform. In some examples, the method is performed by a defibrillator. In some examples, the operating of the resonant therapeutic current control network comprises staggering a time of initiation of current switching relative to a time of initiation of voltage switching, the voltage switching being associated with the current switching, so as to reduce switching losses relative to substantially simultaneous initiation of the current switching and the associated voltage switching. In some examples, timing of voltage switching waveforms relative to associated current switching waveforms is controlled so as to minimize switching losses. In some examples, operating the resonant therapeutic current control network to employ substantially zero voltage switching (ZVS) and substantially zero current switching (ZCS). In some examples, operating the resonant therapeutic current control network to employ closed-loop control of the electrotherapeutic waveform being delivered to the patient. In some examples, operating the resonant therapeutic current control network to continuously adjust, over time, the delivery of the electrotherapeutic waveform to the patient. In some examples, specifying the desired electrotherapeutic waveform for delivery to the patient comprises specifying a current waveform.

In some examples, the method includes charging the energy storage capacitor using a battery electrically coupled with the energy storage capacitor, and comprising returning at least a portion of energy stored by the energy storage capacitor to the battery for storage by the battery using a bidirectional charging control network, the bidirectional charging control network being electrically coupled to the battery and the energy storage capacitor. In some examples, the method includes using a bidirectional charging control network in transferring energy from the energy storage capacitor to a battery, and from the battery to the energy storage capacitor, wherein the battery and the energy storage capacitor are electrically coupled, wherein the energy storage capacitor is for providing energy for the electrotherapeutic waveform being delivered to the patient. In some examples, using the bidirectional charging control network comprises using the bidirectional charging control network comprising a resonant tank. In some examples, generating the electrotherapeutic pulse for delivery to a patient is for defibrillation. In some examples, generating the electrotherapeutic pulse for delivery to a patient is for pacing.

In some examples, the at least one current control switch comprises a plurality of switches, and comprising controlling a configuration of each of the switches of the plurality of switches as open or closed in adjusting delivery of the electrotherapeutic waveform being delivered to the patient to match the desired electrotherapeutic waveform. In some examples,
The method includes controlling a switching frequency of the at least one current control switch in adjusting delivery of the electrotherapeutic waveform being delivered to the patient to match the desired electrotherapeutic waveform.

One example of a computerized mobile device configured to provide an electrotherapeutic pulse to a patient includes: a housing; and an electronic system, disposed within the housing, configured for generating an electrotherapeutic waveform to be delivered to the patient, the electronic system comprising: an energy storage capacitor for providing current to be delivered to the patient, a therapeutic current control network, electrically coupled to the energy storage capacitor, comprising at least one current control switch and a resonant electrical circuit, wherein energy provided by the energy storage capacitor flows through the resonant electrical circuit, and a controller, electrically coupled to the therapeutic current control network, for, in connection with the energy provided by the energy storage capacitor that flows through the resonant electrical circuit, controlling operation of the at least one current control switch of the therapeutic current control network in delivering the electrotherapeutic waveform to the patient to result in a desired waveform being delivered to the patient; wherein the computerized mobile device has an energy density, given by an energy of the electrotherapeutic waveform to be delivered to the patient relative to a volume defined by the housing, of between 0.50 - 4.00 joules per cubic centimeter.

In some examples, the device includes an electrical parameter sensor configured to produce a signal from which a current provided to the patient can be determined or estimated, and wherein the controller is configured to: process the signal from which the current provided to the patient can be determined or estimated; compare the processed signal with a second signal associated with the desired waveform; and control operation of the at least one current control switch of the plurality of switches of the therapeutic current control network in adjusting delivery of the electrotherapeutic waveform to the patient such that the electrotherapeutic waveform reflects the desired waveform. In some examples, the resonant electrical circuit comprises a resonant tank. In some examples, the electrotherapeutic waveform is for defibrillation. In some examples, the electrotherapeutic waveform is for pacing.

In some examples, the computerized mobile device is configured to deliver the electrotherapeutic waveform to the patient, wherein the electrotherapeutic waveform has an energy of between 1 - 400 joules. In some examples, the computerized mobile device is configured to deliver the electrotherapeutic waveform to the patient, wherein the electrotherapeutic waveform has an energy of between 50 - 200 joules. In some examples, the computerized mobile device has an energy density, given by an energy of the electrotherapeutic waveform to be delivered to the patient relative to a volume defined by the housing, of between 0.5 - 1.00 joules per cubic centimeter. In some examples, the computerized mobile device has an energy density, given by an energy of the electrotherapeutic waveform to be delivered to the patient relative to a volume defined by the housing, of between 1.00 - 2.00 joules per cubic centimeter. In some examples, the computerized mobile device has an energy density, given by an energy of the electrotherapeutic waveform to be delivered to the patient relative to a volume defined by the housing, of between 2.00 - 3.00 joules per cubic centimeter. In some examples, the computerized mobile device has an energy density, given by an energy of the electrotherapeutic waveform to be delivered to the patient relative to a volume defined by the housing, of between 3.00 - 4.00 joules per cubic centimeter. In some examples, the computerized mobile device has a volume, defined by the housing, of between 400 - 1,200 cubic centimeters. In some examples, the computerized mobile device has a volume, defined by the housing, of between 200 - 800 cubic centimeters. In some examples, the computerized mobile device has a volume, defined by the housing, of between 100 - 400 cubic centimeters. In some examples, the electronic system comprises at least one planar transformer of between 2,500 - 4,000 volts. In some examples, the electronic system comprises at least one planar transformer of between 2,750 - 3,500 volts. In some examples, the electronic system comprises at least one planar transformer of between 3,000 - 3,300 volts.

In some examples, the electronic system comprises at least one switching device comprising a wide bandgap material and with a switching speed of between 150 - 500 kilohertz. In some examples, the at least one switching device comprises at least one of silicon carbide (SiC) or gallium nitride (GaN). In some examples, the electronic system comprises at least one switching device comprising a wide bandgap material and with a switching speed of between 200 - 600 kilohertz. In some examples, the at least one switching device comprises at least one of silicon carbide (SiC) or gallium nitride (GaN).

In some examples, the electronic system comprises at least one switching device comprising a wide bandgap material and with a switching speed of between 300 - 500 kilohertz. In some examples, the electronic system comprises at least one switching device comprising at least one of silicon carbide (SiC) or gallium nitride (GaN).

In some examples, the electronic system comprises at least one switching device comprising a wide bandgap material and with a switching speed of between 375 - 500 kilohertz. In some examples, the at least one switching device comprises at least one of silicon carbide (SiC) or gallium nitride (GaN).

In some examples, the controller is configured to control operation of the therapeutic current control network to stagger a time of initiation of current switching relative to a time of initiation of voltage switching, associated with the current switching, so as to reduce switching losses relative to substantially simultaneous initiation of the current switching and the associated voltage switching. In some examples, the at least one current control switch comprises a plurality of switches, and wherein at least a portion of the switches comprises a semiconductor material with a band gap of between 2 and 6 ev. In some examples, at least one switch of the plurality of switches comprises at least one of: silicon carbide (SiC) and gallium nitride (GaN). In some examples, the controller is configured to control timing voltage switching waveforms relative to associated current switching waveforms to minimize switching losses. In some examples, the controller is configured to control operation of the at least one current control switch to provide substantially zero voltage switching (ZVS) and substantially zero current switching (ZCS). In some examples, the electrotherapeutic waveform delivered to the patient delivers an amount of energy to the patient that is no more than 15 percent different than an amount of energy of the desired waveform. In some examples, a peak energy efficiency of the computerized mobile device in providing the electrotherapeutic waveform to the patient is at least 80, 85 or 90 percent.

In some examples, the computerized mobile device comprises a driver circuit for use in control of operation of the at least one current control switch, wherein the driver circuit is connected to ground. In some examples, the controlling of the operation of the at least one current control switch of the therapeutic current control network in the adjusting of the delivery of the electrotherapeutic waveform to the patient to result in the desired waveform being delivered to the patient comprises controlling a switching frequency of the at least one current control switch.

In some examples, the energy storage capacitor and a first portion of the therapeutic current control network comprising a patient load and are connected via a first node; the first portion of the therapeutic current control network and a second portion of the therapeutic current control network comprising the resonant electrical circuit are connected via a second node; and the second portion of the therapeutic current control network and a third portion of the therapeutic current control network comprising the at least one current control switch are connected via a third node. In some examples, the third portion of the therapeutic current control network and the electrical parameter sensor are connected via a fourth node.

In some examples, the energy storage capacitor and the at least one current control switch are connected via a first node and a second node; the at least one current control switch and the resonant electrical circuit are connected via a third and a fourth node; and the resonant electrical circuit and the rectifier are connected via a fifth node and a sixth node.

In some examples, the computerized mobile device comprises a battery and a bidirectional charging control network, wherein: the battery and the bidirectional charging control network are connected via a first node and a second node, and the bidirectional charging control network and the energy storage capacitor are connected via a third node and a fourth node, wherein: the bidirectional charging control network is for controlling energy flow from the battery to the energy storage capacitor for storage by the energy storage capacitor, and from the energy storage capacitor to the battery for storage by the battery.

In some examples, the computerized mobile device comprises a battery; and a bidirectional charging control network, comprising: a first circuit connected with the battery, a second circuit connected with the energy storage capacitor, and a transformer, connected with the first circuit and the second circuit, for use in storing first energy from the battery and in transferring at least a portion of the stored first energy to the energy storage capacitor, and for use in storing second energy from the energy storage capacitor and in transferring at least a portion of the stored second energy to the battery. In some examples, at least one of the first circuit and the second circuit comprises at least one clamp circuit.

In some examples, the computerized mobile device comprises: a first circuit, connected with a battery, and a transformer circuit, connected with the first circuit, wherein the transformer circuit is connected with the energy storage capacitor, wherein the bidirectional charging control network is configured to operate in a forward mode in a buck capacity, and in a reverse mode in a boost capacity, and wherein: in the forward mode, energy flows from the battery to the energy storage capacitor for storage by the energy storage capacitor, and in the reverse mode, energy flows from the energy storage capacitor to the battery for storage by the battery.

In some examples, the therapeutic current control network comprises a patient relay circuit used in control of allowing current flow through a patient load, wherein the patient relay circuit comprises at least one patient relay switch comprising a wide bandgap material. In some examples, the wide bandgap material comprises at least one of silicon carbide (SiC) and gallium nitride (GaN). In some examples, the computerized mobile device of claim 202, wherein the at least one patient relay switch comprises at least one MOSFET. In some examples, the patient relay circuit comprises a plurality of driver circuits. In some examples, the plurality of driver circuits are connected in series.

One example of a system for generating an electrotherapeutic pulse to be delivered to a patient includes: a battery; an energy storage capacitor for providing electrotherapeutic current to the patient; a bidirectional charging control network, comprising: a first circuit connected with the battery, and a transformer circuit, connected with the first circuit, wherein the transformer circuit is connected with the energy storage capacitor, wherein the bidirectional charging control network is configured to operate in a forward mode in a buck capacity, and in a reverse mode in a boost capacity, and wherein: in the forward mode, energy flows from the battery to the energy storage capacitor for storage by the energy storage capacitor, and in the reverse mode, energy flows from the energy storage capacitor to the battery for storage by the battery; a therapeutic current control network, electrically coupled to the energy storage capacitor, for controlling the electrotherapeutic current provided to the patient, the therapeutic current control network comprising at least one current control switch; and a controller electrically coupled to the energy storage capacitor and the therapeutic current control network, wherein the controller is configured to control operation of the at least one current control switch of the therapeutic current control network in delivering an electrotherapeutic waveform to the patient to correspond with a specified waveform.

In some examples, the therapeutic current control network comprises a resonant tank, and wherein energy provided by the energy storage capacitor flows through the resonant tank. In some examples, the first circuit is configured as a synchronous converter to operate in the forward mode as a synchronous buck converter, and to operate in the reverse mode as a synchronous boost converter. In some examples, the first circuit comprises at least two switches and at least one inductor. In some examples, the first circuit comprises a full bridge and at least one inductor. In some examples, the first circuit is connected to the transformer circuit via a first node and a second node. In some examples, the transformer circuit comprises: a full bridge; a transformer connected to the full bridge via a third node and a fourth node; a portion, comprising a half bridge and two capacitors, connected to the transformer via a fifth node and a sixth node; and a capacitor connected to the portion via a seventh node and a eighth node. In some examples, during operation in the forward mode, current flows in a first direction and wherein, during operation in the reverse mode, current flows in a second direction. In some examples, the at least one sensor configured to sense at least one electrical parameter from which current flow to the patient can be determined or estimated.

In some examples, the controller is configured to: process a signal associated with the sensed at least one electrical parameter, compare the processed signal with a second signal associated with the specified waveform, and based at least in part on the comparison, control operation of the at least one current control switch of the therapeutic current control network in adjusting delivery of the electrotherapeutic waveform to the patient to correspond with the specified waveform.

One example of a system for generating an electrotherapeutic pulse to be delivered to a patient, the system includes: a battery; an energy storage capacitor for providing electrotherapeutic current to the patient; a bidirectional charging control network, comprising: a first circuit connected with the battery, a second circuit connected with the energy storage capacitor, and a transformer, connected with the first circuit and the second circuit, for use in storing first energy from the battery and in transferring at least a portion of the stored first energy to the energy storage capacitor, and for use in storing second energy from the energy storage capacitor and in transferring at least a portion of the stored second energy to the battery; a therapeutic current control network, electrically coupled to the energy storage capacitor, for controlling the electrotherapeutic current to be delivered to the patient, the therapeutic current control network comprising at least one current control switch; and a controller electrically coupled to the energy storage capacitor and the therapeutic current control network, wherein the controller is configured to control operation of the at least one current control switch of the therapeutic current control network in delivering an electrotherapeutic waveform to the patient to correspond with a specified waveform.

In some examples, the system comprises at least one sensor configured to sense at least one electrical parameter from which current flow to the patient can be determined or estimated. In some examples, the therapeutic current control network comprises a resonant tank, and wherein energy provided by the energy storage capacitor flows through the resonant tank. In some examples, the first circuit comprises at least one clamp circuit.

In some examples, the second circuit comprises at least one clamp circuit. In some examples, the first circuit comprises a first at least one clamp circuit, and wherein second circuit comprises a second at least one clamp circuit. In some examples, the at least one clamp circuit comprises at least one active clamp circuit. In some examples, the at least one clamp circuit comprises at least one passive clamp circuit. In some examples, the at least one clamp circuit comprises at least one active clamp circuit. In some examples, the at least one clamp circuit comprises at least one passive clamp circuit. In some examples, the first at least one clamp circuit of the first circuit and the second at least one clamp circuit of the second circuit are used in fixing signal peaks. In some examples, the first at least one clamp circuit of the first circuit and the second at least one clamp circuit of the second circuit are used in increasing energy efficiency of operation of the system. In some examples, the first at least one clamp circuit of the first circuit and the second at least one clamp circuit of the second circuit are used in reducing energy dissipation as heat during operation of the system.

In some examples, the controller is configured to: process a signal associated with the sensed at least one electrical parameter, compare the processed signal with a second signal associated with the specified waveform, and based at least in part on the comparison, control operation of the at least one current control switch of the therapeutic current control network in adjusting delivery of the electrotherapeutic waveform to the patient to correspond with the specified waveform. In some examples, the bidirectional charging control network is configured to operate using at least one of substantially zero current switching (ZVS) and substantially zero voltage switching (ZVS). In some examples, the bidirectional charging control network comprises at least one switch comprising at least one wide bandgap material. In some examples, the at least one wide bandgap material comprises at least one of silicon carbide (SiC) and gallium nitride (GaN). In some examples, the system is configured to have operating modes comprising a ZVS mode, a continuous current mode and a discontinuous current mode, and wherein a mode of operation used at a particular time is optimized, with regard to energy efficiency, based at least in part on a charging cycle state at the particular time.

One example of a system for generating an electrotherapeutic pulse to be delivered to a patient, the system comprising: a battery; an energy storage capacitor for providing electrotherapeutic current to the patient; a bidirectional charging control network for controlling energy flow from the battery to the energy storage capacitor for storage by the energy storage capacitor, and from the energy storage capacitor to the battery for storage by the battery; a therapeutic current control network, electrically coupled to the energy storage capacitor, for controlling the electrotherapeutic current to be delivered to the patient, the therapeutic current control network comprising at least one current control switch; at least one sensor configured to sense at least one electrical parameter from which current flow to the patient can be determined or estimated; and a controller electrically coupled to the energy storage capacitor, the therapeutic current control network and the at least one sensor, wherein the controller is configured to: process a signal associated with the sensed at least one electrical parameter, compare the processed signal with a second signal associated with a specified waveform, and based at least in part on the comparison, control operation of the at least one current control switch of the therapeutic current control network in adjusting delivery of an electrotherapeutic waveform to the patient to correspond with a specified waveform.

In some examples, the therapeutic current control network comprises a resonant tank. In some examples, the bidirectional charging control network comprises: a first circuit connected with the battery, a second circuit connected with the energy storage capacitor, and a transformer, connected with the first circuit and the second circuit, for use in storing first energy from the battery and in transferring at least a portion of the stored first energy to the energy storage capacitor, and for use in storing second energy from the energy storage capacitor and in transferring at least a portion of the stored second energy to the battery.

In some examples, the first circuit comprises at least one clamp circuit. In some examples, the second circuit comprises at least one clamp circuit. In some examples, the first circuit comprises a first at least one clamp circuit, and wherein second circuit comprises a second at least one clamp circuit. In some examples, the at least one clamp circuit comprises at least one active clamp circuit. In some examples, the at least one clamp circuit comprises at least one passive clamp circuit. In some examples, the at least one clamp circuit comprises at least one active clamp circuit. In some examples, the at least one clamp circuit comprises at least one passive clamp circuit. In some examples, the first at least one clamp circuit of the first circuit and the second at least one clamp circuit of the second circuit are used in fixing signal peaks. In some examples, the first at least one clamp circuit of the first circuit and the second at least one clamp circuit of the second circuit are used in increasing energy efficiency of operation of the system. In some examples, the first at least one clamp circuit of the first circuit and the second at least one clamp circuit of the second circuit are used in reducing energy dissipation as heat during operation of the system.

In some examples, the system is configured to have operating modes comprising a ZVS mode, a continuous current mode and a discontinuous current mode, and wherein a mode of operation used at a particular time is optimized, with regard to energy efficiency, based at least in part on a charging cycle state at the particular time. In some examples, the bidirectional charging control network comprises a resonant electrical circuit. In some examples, the bidirectional charging control network is configured to operate using at least one of substantially zero current switching (ZVS) and substantially zero voltage switching (ZVS). In some examples, the bidirectional charging control network comprises at least one switch comprising at least one wide bandgap material. In some examples, the at least one wide bandgap material comprises at least one of silicon carbide (SiC) and gallium nitride (GaN).

In some examples, the bidirectional charging control network comprises: a first circuit, connected with the battery, and a transformer circuit, connected with the first circuit, wherein the transformer circuit is connected with the energy storage capacitor, wherein the bidirectional charging control network is configured to operate in a forward mode in a buck capacity, and in a reverse mode in a boost capacity, and wherein: in the forward mode, energy flows from the battery to the energy storage capacitor for storage by the energy storage capacitor, and in the reverse mode, energy flows from the energy storage capacitor to the battery for storage by the battery.

In some examples, the bidirectional charging control network is configured such that, in the control of the energy flow from the energy storage capacitor to the battery for storage by the battery, at least one of: (1) estimated or anticipated charge to the battery is limited to less than 100% of a charge limit relating to the battery to avoid or minimize risk of overcharging the battery, and (2) a rate of the energy flow from the energy storage capacitor to the battery for storage by the battery is limited to avoid or minimize risk of overcharging the battery.

In some examples, the bidirectional charging control network is configured such that, in the control of the energy flow from the energy storage capacitor to the battery for storage by the battery, at least one of: estimated or anticipated charge to the battery is limited to less than 100% of a charge limit relating to the battery to avoid or minimize risk of overcharging the battery, and a rate of the energy flow from the energy storage capacitor to the battery for storage by the battery is limited to avoid or minimize risk of overcharging the battery. In some examples, the bidirectional charging control network is configured such that, in the control of the energy flow from the energy storage capacitor to the battery for storage by the battery, estimated or anticipated charge to the battery is limited to less than 100% of a charge limit relating to the battery to avoid or minimize risk of overcharging the battery. In some examples, the bidirectional charging control network is configured such that, in the control of the energy flow from the energy storage capacitor to the battery for storage by the battery, estimated or anticipated charge to the battery is limited to an amount that is at or greater than 80%, and less than 100%, of a charge limit relating to the battery.

In some examples, the bidirectional charging control network is configured such that, in the control of the energy flow from the energy storage capacitor to the battery for storage by the battery, a rate of the energy flow from the energy storage capacitor to the battery for storage by the battery is limited to avoid or minimize risk of overcharging the battery. In some examples, the bidirectional charging control network is configured such that, in the control of the energy flow from the energy storage capacitor to the battery for storage by the battery, a rate of the energy flow from the energy storage capacitor to the battery for storage by the battery is limited based at least in part based on a rate of energy consumption resulting from operation of the therapeutic current control network.

One example system for generating an electrotherapeutic pulse to be delivered to a patient includes a battery; an energy storage capacitor for providing electrotherapeutic current to the patient; a bidirectional charging control network for controlling energy flow from the battery to the energy storage capacitor for storage by the energy storage capacitor, and from the energy storage capacitor to the battery for storage by the battery; a therapeutic current control network, electrically coupled to the energy storage capacitor, for controlling the electrotherapeutic current to be delivered to the patient, the therapeutic current control network comprising at least one current control switch and a resonant electrical circuit, wherein energy provided by the energy storage capacitor flows through the resonant electrical circuit; a controller electrically coupled to the energy storage capacitor and the therapeutic current control network, wherein the controller is configured to control operation of the at least one current control switch of the therapeutic current control network in delivering an electrotherapeutic waveform to the patient to correspond with a specified waveform.

In some examples, the system includes at least one sensor, electrically coupled with the controller, configured to sense at least one electrical parameter from which current flow to the patient can be determined or estimated, and wherein the controller is configured to: process a signal associated with the sensed at least one electrical parameter; compare the processed signal with a second signal associated with the specified waveform; and control operation of the at least one current control switch of the therapeutic current control network in adjusting delivery of the electrotherapeutic waveform to the patient to correspond with the specified waveform.

In some examples, the bidirectional charging control network comprises: a first circuit connected with the battery, a second circuit connected with the energy storage capacitor, and a transformer, connected with the first circuit and the second circuit, for use in storing first energy from the battery and in transferring at least a portion of the stored first energy to the energy storage capacitor, and for use in storing second energy from the energy storage capacitor and in transferring at least a portion of the stored second energy to the battery.

In some examples, the bidirectional charging control network comprises: a first circuit, connected with the battery, and a transformer circuit, connected with the first circuit, wherein the transformer circuit is connected with the energy storage capacitor, wherein the bidirectional charging control network is configured to operate in a forward mode in a buck capacity, and in a reverse mode in a boost capacity, and wherein: in the forward mode, energy flows from the battery to the energy storage capacitor for storage by the energy storage capacitor, and in the reverse mode, energy flows from the energy storage capacitor to the battery for storage by battery.

In some examples, the bidirectional charging control network is configured such that, in the control of the energy flow from the energy storage capacitor to the battery for storage by the battery, at least one of: (1) estimated or anticipated charge to the battery is limited to less than 100% of a charge limit relating to the battery to avoid or minimize risk of overcharging the battery, and (2) a rate of the energy flow from the energy storage capacitor to the battery for storage by the battery is limited to avoid or minimize risk of overcharging the battery.

In some examples, the bidirectional charging control network is configured such that, in the control of the energy flow from the energy storage capacitor to the battery for storage by the battery, estimated or anticipated charge to the battery is limited to less than 100% of a charge limit relating to the battery to avoid or minimize risk of overcharging the battery. In some examples, the bidirectional charging control network is configured such that, in the control of the energy flow from the energy storage capacitor to the battery for storage by the battery, estimated or anticipated charge to the battery is limited to an amount that is at or greater than 80%, and less than 100%, of a charge limit relating to the battery. In some examples, the bidirectional charging control network is configured such that, in the control of the energy flow from the energy storage capacitor to the battery for storage by the battery, a rate of the energy flow from the energy storage capacitor to the battery for storage by the battery is limited to avoid or minimize risk of overcharging the battery.

In some examples, the bidirectional charging control network is configured such that, in the control of the energy flow from the energy storage capacitor to the battery for storage by the battery, a rate of the energy flow from the energy storage capacitor to the battery for storage by the battery is limited based at least in part based on a rate of energy consumption resulting from operation of the therapeutic current control network.

An example, according to some embodiments of the disclosure, of a system for generating an electrotherapeutic pulse to be delivered to a patient includes: an energy storage capacitor for providing electrotherapeutic current to the patient; a therapeutic current control network, electrically coupled to the energy storage capacitor, for controlling the electrotherapeutic current to be delivered to the patient, the therapeutic current control network including a switch network, a resonant electrical circuit and a rectifier, wherein: the energy storage capacitor and the switch network are connected via a first node and a second node,

the switch network and the resonant electrical circuit are connected via a third node and a fourth node, and the resonant electrical circuit and the rectifier are connected via a fifth node and a sixth node; at least one sensor configured to sense at least one electrical parameter indicative of current flow to the patient; and a controller electrically coupled to the energy storage capacitor, the therapeutic current control network and the at least one sensor, wherein the controller is configured to: process a signal associated with the sensed at least one electrical parameter, compare the processed signal with a second signal associated with a specified waveform, and, based at least in part on the comparison, control, at least, operation of at least a portion of the switch network of the therapeutic current control network to adjust control of an electrotherapeutic waveform being delivered to the patient to correspond with the specified waveform.

An example, according to some embodiments of the disclosure, of a system for generating an electrotherapeutic pulse to be delivered to a patient includes: an energy storage capacitor for providing electrotherapy current to be delivered to the patient; a therapeutic current control network, electrically coupled to the energy storage capacitor, for controlling the electrotherapy current to be delivered to the patient, the therapeutic current control network including: a switch network, a resonant electrical circuit including at least one inductor and at least one capacitor, and a rectifier, wherein: the energy storage capacitor and the switch network are connected via a first node and a second node, the switch network and the resonant electrical circuit are connected via a third node and a fourth node, and the resonant electrical circuit and the rectifier are connected via a fifth node and a sixth node; an electrical parameter sensor configured to produce a signal associated with a current provided to the patient; and a controller electrically coupled to the energy storage capacitor, the therapeutic current control network and the electrical parameter sensor, wherein the controller is configured to:
process the signal associated with the current provided to the patient, compare the processed signal with a second signal associated with a specified waveform, and based at least in part on the comparison, control, at least, operation of at least a portion of the switch network of the therapeutic current control network to adjust control of an electrotherapeutic waveform being delivered to the patient to correspond with the specified waveform.

An example, according to some embodiments of the disclosure, of a method for generating an electrotherapeutic pulse for delivery to a patient incudes: specifying a desired electrotherapeutic waveform for delivery to the patient, wherein energy for electrotherapy current is provided by an energy storage capacitor; determining or estimating a current output delivered to the patient; comparing the current output to the desired electrotherapeutic waveform; and operating a therapeutic current control network, including a switch network, a resonant tank and a rectifier, to control an electrotherapeutic waveform being delivered to the patient to match the desired electrotherapeutic waveform, wherein the energy storage capacitor and the switch network are electrically connected, the switch network and the resonant tank are electrically connected, and the resonant tank and the rectifier are electrically connected.

An example, according to some embodiments of the disclosure, of a system for generating an electrotherapeutic pulse to be delivered a patient includes: an energy storage capacitor for providing electrotherapy current to be delivered to the patient; a therapeutic current control network, electrically coupled to the energy storage capacitor, for controlling the electrotherapy current to be delivered to the patient, wherein the therapeutic current control network includes a resonant electrical circuit and a plurality of switches, wherein at least one of the plurality of switches includes a semiconductor material with a band gap of between 2 and 6 ev; an electrical parameter sensor configured to produce a signal associated with a current provided to the patient; and a controller, electrically coupled to the therapeutic current control network, configured to: process the signal associated with the current provided to the patient, compare the processed signal with a second signal associated with a specified waveform, and based at least in part on the comparison, control, at least, operation of at least a portion of the plurality of switches of the therapeutic current control network to adjust control of an electrotherapeutic waveform being delivered to the patient such that the electrotherapeutic waveform reflects the specified waveform.

An example, according to some embodiments of the disclosure, of a computerized mobile device configured to provide an electrotherapeutic pulse to a patient includes: a housing; an electronic system, disposed within the housing, configured for generating an electrotherapeutic waveform to be delivered to the patient, the electronic system including: an energy storage capacitor for providing current to be delivered to the patient, a therapeutic current control network, electrically coupled to the energy storage capacitor, including a switch network and a resonant electrical circuit, and a controller, electrically coupled to the therapeutic current control network, for controlling operation, at least, of at least a portion of the switch network of the therapeutic current control network to adjust control of the electrotherapeutic waveform being delivered to the patient to result in a desired waveform being delivered to the patient; wherein the computerized mobile device has an energy density, given by an energy of the electrotherapeutic waveform to be delivered to the patient relative to a volume defined by the housing, of between 0.50 - 4.00 joules per cubic centimeter.

Some implementations may include one or more of the following features. A resonant electrical circuit may include a resonant tank. A sensed at least one electrical parameter may include a current sensor and/or a voltage sensor. A controller may be digital or analog. A controller may include at least one processor.

A system may include a battery and a bidirectional charging control network, wherein: the battery and the bidirectional charging control network are connected via a seventh node and an eighth node, and the bidirectional charging control network and the energy storage capacitor are connected via a ninth node and a tenth node, wherein: the bidirectional charging control network is for controlling energy flow from the battery to the energy storage capacitor for storage by the energy storage capacitor, and from the energy storage capacitor to the battery for storage by the battery. The bidirectional charging control network may include a second resonant electrical circuit. The second resonant circuit may include a resonant tank.

A system may include a filter, wherein the rectifier and the filter are connected via an eleventh node and a twelfth node. The system may include a polarity control network, wherein the filter and the polarity control network are connected via an thirteenth node and a fourteenth node. The polarity control may include an H-bridge. The system may include at least one sensor that is connected with the polarity control network via a fifteenth node and a sixteenth node.

A controller may be configured to control operation of the at least a portion of the switch network of the therapeutic current control network to stagger a time of initiation of current switching relative to a time of initiation of voltage switching, the voltage switching being associated with the current switching, so as to reduce switching losses relative to substantially simultaneous initiation of the current switching and the associated voltage switching. A controller may be configured to control timing of voltage switching waveforms relative to associated current switching waveforms to minimize switching losses. A controller may be configured to control timing voltage switching waveforms relative to associated current switching waveforms to minimize switching losses and to reduce electromagnetic interference (EMI). A controller may be configured to control operation of the at least a portion of the switch network of the therapeutic current control network to provide substantially zero voltage switching (ZVS) and substantially zero current switching (ZCS).

A switch network may include a plurality of switches, wherein at least one of the switches includes a semiconductor material with a band gap of between 2 and 6 ev. A switch network may include a plurality of switches wherein at least one of the switches includes at least one of: silicon carbide (SiC) and gallium nitride (GaN).

A system may have a peak energy efficiency of the system in delivering the electrotherapeutic waveform to the patient that is at least 80, 85 or 90 percent, or that is between 80 - 95 percent. A system may deliver an electrotherapeutic waveform delivered to the patient that delivers an amount of energy to the patient that is no more than 15, 10, 5 or 3 percent different than an amount of energy of the specified waveform.

A system may include an electrical circuit that includes the energy storage capacitor, the therapeutic current control network, the at least one sensor and the controller. The at least one sensor may include a current sensor and a voltage sensor. The specified waveform may be a biphasic rectilinear current waveform.

A system may include a polarity control network, wherein the polarity control network is used in controlling direction of current flow in delivering a biphasic rectilinear current waveform to the patient. A controller may be configured to receive input specifying a specified waveform. The specified waveform may be a current waveform desired to be delivered to the patient. The controller may be configured to store data specifying the specified waveform. The system may be configured to provide closed-loop control of the electrotherapeutic waveform being delivered to the patient. The system may be configured to monitor impedance of the patient over time during delivery of the electrotherapeutic waveform to the patient.

A resonant electrical circuit may be a two element, three element or four element resonant tank. A resonant electrical circuit may be an LCC, CLL, LLC, or LCLC resonant tank. A resonant tank may include two inductors and one capacitor. A rectifier may be configured for use in conversion of alternating current to direct current. A rectifier may include a switching network configured to be controlled to perform synchronous or active rectification. A rectifier may include a switching network configured to be controlled to perform passive rectification. A controller may include an field-programmable gate array (FPGA). A controller may include a digital signal processor (DSP). A switch network may be configured to be operated for use in adjusting a level of electrotherapeutic current being delivered to the patient. A resonant electrical circuit may be configured for use in storing at least a portion of energy received by the therapeutic current control network from the energy storage capacitor. A filter may be configured for use in electrical signal frequency filtering. A polarity control network may be configured for use in controlling direction of current flow. A specified waveform may be an electrotherapeutic current waveform. An electrotherapeutic waveform may be for defibrillation or pacing.

A resonant electrical circuit may store at least a portion of energy received by the therapeutic current control network from the energy storage capacitor. A resonant electrical circuit may include a first inductor connected in series, a second inductor connected in parallel, and a capacitor connected in series.

A method may include operating a therapeutic current control network to continuously adjust, over time, the electrotherapeutic waveform being delivered to the patient. A method may include charging an energy storage capacitor, the energy storage capacitor being electrically coupled with the therapeutic current control network and used in providing electrotherapeutic current for delivery to the patient, using a battery electrically coupled with the energy storage capacitor, and including returning at least a portion of energy stored by the energy storage capacitor to the battery for storage by the battery using a bidirectional charging control network, the bidirectional charging control network being electrically coupled to the battery and the energy storage capacitor. Using the bidirectional charging control network may include using the bidirectional charging control network including a resonant tank. A method may include generating an electrotherapeutic pulse for delivery to a patient for defibrillation or pacing.

A computerized mobile device configured to provide an electrotherapeutic pulse to a patient may include one or more of the following features. A device may include a resonant electrical circuit that includes a resonant tank. A device may be configured to provide an electrotherapeutic waveform for defibrillation or pacing. A device may be configured to deliver an electrotherapeutic waveform to the patient, wherein the electrotherapeutic waveform has an energy of between, for example, 1 - 400 or 50 - 200 joules. A device may have an energy density, given by an energy of the electrotherapeutic waveform to be delivered to the patient relative to a volume defined by the housing, for example, of between 0.5 - 1.00, 1.00 - 2.00, 2.00 - 3.00 or 3.00 - 4.00 joules per cubic centimeter. A device may have an energy density of 4.00 - 6.00 joules per cubic centimeter. A device may have an energy density of over 4.00 joules per cubic centimeter. A device may have an energy density of over 6.00 joules per cubic centimeter. A device may have an energy density of less than 0.5 joules per cubic centimeter. A device may have a volume defined by the housing, for example, of between 400 - 1,200, 200 - 800 or 100 - 400 cubic centimeters. A device may have a volume, defined by the housing, of less than 100 cubic centimeters. A device may have a volume, defined by the housing, of greater than 1,200 cubic centimeters. A device may have at least one planar transformer of, for example, between 2,500 - 4,000, 2,750 - 3,500 or 3,000 - 3,300 volts. An electronic system may include at least one switching device including a wide bandgap material. A device may include at least one switching device that may include at least one of silicon carbide (SiC) or gallium nitride (GaN). A switch network may include a plurality of switches, and wherein at least a portion of the switches includes a semiconductor material with a band gap of, for example, between 2 and 6 ev. An electronic system may include at least one switching device with a switching speed, for example, of between 150 - 500, 200 - 600, 300 - 500 or 375 - 500 kilohertz. An electronic system may include at least one switching device with a switching speed of less than 150 kilohertz. An electronic system may include at least one switching device with a switching speed of greater than 500 kilohertz. A device may include a controller that is configured to control operation of the therapeutic current control network to stagger a time of initiation of current switching relative to a time of initiation of voltage switching, associated with the current switching, so as to reduce switching losses relative to substantially simultaneous initiation of the current switching and the associated voltage switching. A controller may be configured to control timing voltage switching waveforms relative to associated current switching waveforms to minimize switching losses. A controller may be configured to control operation of the switch network of the therapeutic current control network to provide substantially zero voltage switching (ZVS) and substantially zero current switching (ZCS). An electrotherapeutic waveform delivered to the patient may deliver an amount of energy to the patient that is no more than 15 percent different than an amount of energy of the specified current waveform. A peak energy efficiency of the device in providing the electrotherapeutic waveform to the patient may be, for example, at least 80, 85 or 90 percent.

## BRIEF DESCRIPTION OF THE DRAWINGS

Various aspects of embodiments of the present disclosure are discussed below with reference to the accompanying figures, which are not intended to be drawn to scale. The figures are included for illustrative purposes and a further understanding of the various aspects and examples, and are incorporated in and constitute a part of this specification, but are not intended to limit the scope of the disclosure. The drawings, together with the remainder of the specification, serve to explain principles and operations of the described and claimed aspects and examples. In the figures, identical or nearly identical components that are illustrated in various figures may be represented by like numerals. For purposes of clarity, not every component may be labeled in every figure.

FIG. 1A illustrates an example of use of a defibrillator incorporating a therapeutic current control system, in accordance with embodiments of the present disclosure.
FIG. 1B illustrates an example of use of a handheld defibrillator incorporating a therapeutic current control system, in accordance with embodiments of the present disclosure.
FIG. 2A is a block diagram of an illustrative example of a therapeutic current control system, in accordance with embodiments of the present disclosure.
FIG. 2B is a block diagram of an illustrative example of a therapeutic current control system, in accordance with embodiments of the present disclosure.
FIG. 2C is a block diagram of an illustrative example of a therapeutic current control system, including a battery and a bidirectional charging control network, in accordance with embodiments of the present disclosure.
FIG. 3 is a schematic diagram of an illustrative example of a therapeutic current control system, in accordance with embodiments of the present disclosure.
FIG. 4A is a schematic diagram including an illustrative example of a therapeutic current control network of a therapeutic current control system, in accordance with embodiments of the present disclosure.
FIG. 4B is a schematic diagram including an illustrative example of a therapeutic current control network of a therapeutic current control system, in accordance with embodiments of the present disclosure.
FIG. 4C is a schematic diagram of an illustrative example of a controller of a therapeutic current control system, in accordance with embodiments of the present disclosure.
FIG. 5 is a flow diagram of an illustrative example of a method for therapeutic current control, in accordance with embodiments of the present disclosure.
FIGS. 6A - 6Q are exemplary schematic diagrams of three-element resonant tank configurations that can be used in a therapeutic current control network, in accordance with embodiments of the present disclosure.
FIGS. 6R - 6U are exemplary schematic diagrams of two-element resonant tank configurations that can be used in a therapeutic current control network, in accordance with embodiments of the present disclosure.
FIG. 6V is a schematic exemplary diagram of a four-element resonant tank configuration that can be used in a therapeutic current control network, in accordance with embodiments of the present disclosure.
FIG. 7A is a conceptual exemplary diagram illustrating switch configurations for generating no requested current during a time period, in accordance with embodiments of the present disclosure.
FIG. 7B is a conceptual exemplary diagram illustrating switch configurations for generating full requested current during a time period, in accordance with embodiments of the present disclosure.
FIG. 7C is a graphical illustration of signaling associated with generating partial requested current during a time period, in accordance with embodiments of the present disclosure.
FIG. 7D is an example graphical illustration associated with generating partial requested current during a time period, in accordance with embodiments of the present disclosure.
FIG. 7E is an example graphical illustration associated with generating no requested current during a time period, in accordance with embodiments of the present disclosure.

FIG. 7F is an example graphical illustration associated with generating full requested current during a time period, in accordance with embodiments of the present disclosure.

FIG. 8 is a simplified graphical illustration of signaling associated with hard switching.

FIG. 9 is a simplified graphical illustration of signaling associated with soft switching.

FIG. 10A includes flow diagrams illustrating two different charging related methods, in accordance with embodiments of the present disclosure.

FIG. 10B is a schematic diagram of an example bidirectional charging control network, in accordance with embodiments of the present disclosure.

FIG. 10C is a schematic diagram of an example full bridge switching mode of the secondary side of the bidirectional charging control network of FIG. 10B.

FIG. 10D is a schematic diagram of an example half bridge switching mode of the secondary side of the bidirectional charging control network of FIG. 10B.

FIGS. 11A-K are graphical illustrations of various generated current waveforms matching specified waveforms, in accordance with embodiments of the present disclosure.

FIG. 12 is a block diagram of an example quasi-resonant therapeutic current control system, in accordance with embodiments of the present disclosure.

FIG. 13 is a flow diagram of a method implemented using a quasi-resonant therapeutic current control system, in accordance with embodiments of the present disclosure.

FIG. 14 is an exemplary schematic diagram of a quasi-resonant therapeutic current control system, in accordance with embodiments of the present disclosure.

FIG. 15 a flow diagram of a method implemented using a quasi-resonant therapeutic current control system, in accordance with embodiments of the present disclosure, which can be associated with the system of FIG. 14 and the timing diagram of FIG. 16.

FIG. 16 is an exemplary timing diagram relating to operation of a quasi-resonant therapeutic current control system, in accordance with embodiments of the present disclosure, which can be associated with the flow diagram of FIG. 15.

FIG. 17 is an exemplary schematic diagram of a quasi-resonant therapeutic current control system, in accordance with embodiments of the present disclosure.

FIG. 18 is a graphical illustration of a biphasic rectilinear waveform that can be produced by a quasi-resonant therapeutic current control system, in accordance with embodiments of the present disclosure.

FIG. 19 is an exemplary schematic diagram of a patient relay with a single driver circuit, in accordance with embodiments of the present disclosure.

FIG. 20 is an exemplary schematic diagram of a patient relay with a dual driver circuits, in accordance with embodiments of the present disclosure.

FIG. 21 is an exemplary schematic diagram of a bidirectional charging network, in accordance with embodiments of the present disclosure.

FIG. 22 is a block diagram of a bidirectional charging network, in accordance with embodiments of the present disclosure.

FIG. 23 is an exemplary schematic diagram of a transformer circuit, which can be associated with the network depicted in FIG. 22, in accordance with embodiments of the present disclosure.

FIG. 24 is an exemplary schematic diagram of a primary side switch, which can be associated with the network depicted in FIG. 22, in accordance with embodiments of the present disclosure.

FIG. 25 is an exemplary schematic diagram of a primary side active clamp, which can be associated with the network depicted in FIG. 22, in accordance with embodiments of the present disclosure.

FIG. 26 is an exemplary schematic diagram of a primary side switch driver, which can be associated with the network depicted in FIG. 22, in accordance with embodiments of the present disclosure.

FIG. 27 is an exemplary schematic diagram of a secondary side gate driver, which can be associated with the network depicted in FIG. 22, in accordance with embodiments of the present disclosure.

FIG. 28 is an exemplary schematic diagram of a secondary side gate driver, which can be associated with the network depicted in FIG. 22, in accordance with embodiments of the present disclosure.

FIG. 29 is an exemplary schematic diagram of a secondary side passive clamp, in accordance with embodiments of the present disclosure, which can be associated with the network depicted in FIG. 22, in accordance with embodiments of the present disclosure.

FIG. 30 is an exemplary schematic diagram of a bidirectional charging control network, showing a forward charging path, in accordance with embodiments of the present disclosure.

FIG. 31 is an exemplary schematic diagram of a bidirectional charging control network, showing a forward discharging path, which can be associated with the network depicted in FIG. 22, in accordance with embodiments of the present disclosure.

FIG. 32 is an exemplary schematic diagram of a bidirectional charging control network, showing a reverse charging path, which can be associated with the network depicted in FIG. 22, in accordance with embodiments of the present disclosure.

FIG. 33 is an exemplary schematic diagram of a bidirectional charging control network, which can be associated with the network depicted in FIG. 20, showing a reverse discharging path, in accordance with embodiments of the present disclosure.

FIG. 34 is a graphical illustration of idealized charging waveforms, which can be associated with the network depicted in FIG. 22, in accordance with embodiments of the present disclosure.

FIG. 35 is a graphical illustration of a continuous control mode (CCM) waveform and a boundary conduction mode (BCM) waveform, in accordance with embodiments of the present disclosure.

FIG. 36 is a graphical illustration of a continuous control mode (CCM) waveform, in accordance with embodiments of the present disclosure.

FIG. 37 is an exemplary schematic diagram of an average input current sense circuit for use with CCM, in accordance with embodiments of the present disclosure.

FIG. 38 is an exemplary schematic diagram of a bidirectional charging control network, including a first circuit connected with a transformer circuit, in accordance with embodiments of the present disclosure.

FIG. 39 is an exemplary schematic diagram of an example transformer circuit as depicted in FIG. 38, including an illustration of an apparent capacitance, in accordance with embodiments of the present disclosure.

FIG. 40 is an exemplary schematic diagram of the bidirectional charging control network of FIG. 38, including an illustration of a first forward mode current flow, in accordance with embodiments of the present disclosure.

FIG. 41 is an exemplary schematic diagram of the bidirectional charging control network of FIG. 38, including an illustration of a second forward mode current flow, in accordance with embodiments of the present disclosure.

FIG. 42 is an exemplary schematic diagram of the bidirectional charging control network of FIG. 38, including an illustration of a first reverse mode current flow, in accordance with embodiments of the present disclosure.

FIG. 43 is an exemplary schematic diagram of the bidirectional charging control network of FIG. 38, including an illustration of a second reverse mode current flow, in accordance with embodiments of the present disclosure.

FIG. 44 includes graphical illustrations of current and voltage waveforms associated with FIGS. 40-41, in accordance with embodiments of the present disclosure.

FIG. 45 includes graphical illustrations of voltage waveforms associated with FIGS. 42-43, in accordance with embodiments of the present disclosure.

FIG. 46 includes graphical illustrations of voltage waveforms associated with the bidirectional charging control network of FIG. 38, in accordance with embodiments of the present disclosure.

FIG. 47 is a schematic diagram illustrating components of a system of various devices that may incorporate aspects of a therapeutic current control system, in accordance with embodiments of the present disclosure.

FIG. 48 is a schematic diagram illustrating components of a system, including a patient monitor and a therapeutic medical device, which may incorporate aspects of a therapeutic current control system, in accordance with embodiments of the present disclosure.

## DETAILED DESCRIPTION

Systems, apparatus and methods are presented herein for generating and delivering an electrotherapeutic pulse according to a specified current waveform to a patient, such as for defibrillation or pacing.

Embodiments described herein provide systems, methods, devices and circuits for generating electrotherapeutic pulses and waveforms for delivery to patients with high safety, reliability, accuracy and precision. Moreover, embodiments of the present disclosure provide for electrical circuit topologies that are built to generate and deliver a wide variety of desired electrotherapy waveforms to the patient, allowing great flexibility in the therapy that may be delivered. This can also provide an ability to explore use of a wide range of different electrotherapeutic waveforms for various potential uses. Still further, embodiments described herein provide or enable use of devices, such as defibrillators, that, in addition to being higher performing, may also be more efficient, more powerful, lighter, smaller, more portable and less expensive than previously existing systems and devices. In some embodiments, with higher performance and efficiency at less size, weight or cost relative to previously existing systems, various previously unachievable combinations and balances of features can be struck as needed for particular uses or implementations. Such balances can include, for example, a balance between minimum necessary particular performance or power parameters for a certain use, and maximum size or weight that is practical for the use. In some embodiments, such uses may include use of portable, wearable or handheld defibrillators or other devices.

Many advantages of embodiments described herein can be further appreciated in part by comparison with previously existing systems and techniques. Previously existing defibrillators, for example, may operate as follows. In an electrical circuit, a power converter such as a flyback converter is used in charging a high voltage capacitor using a battery. Since capacitor current leakage generally must be kept low in such a system to assure that the intended energy reaches the patient, the capacitor must be large and heavy enough to sufficiently minimize or account for such leakage. Some embodiments allow for a significant reduction in the maximum voltages and capacitance imposed on the high voltage capacitor thus allowing for a significant reduction in the size and weight of the high voltage capacitor. Since the high voltage capacitor occupies a significant percentage of the overall volume of a typical defibrillator as well as imposing minimum cross-sectional dimensions on the overall defibrillator package due to the capacitor bulk, the size reductions in the high voltage capacitor made possible by the invention can result in size reductions exceeding a 50% volumetric decrease.

One example of previously existing defibrillators includes those including a circuit including a number of resistors, a set or subset of which can be engaged and used according to a particular selected resistor schedule. A resistance is engaged and initial patient impedance is determined. Based on parameters including the initially determined patient impedance and a desired amount of energy to be delivered to the patient over a time period of milliseconds, an appropriate series of resistors is selected and utilized to deliver a defibrillation pulse to the patient. Energy is provided by discharge of the high voltage capacitor. The generated waveform of the defibrillation pulse is largely governed by the available and selected series of resistors. The circuit requires a substantial amount of electronic components, such as silicon controlled rectifiers (SCRs), gate drivers and resistors. Excess energy may be offloaded or "dumped" into high voltage resistors, producing heat.

Embodiments of the present disclosure provide various advantages. For example, some embodiments allow for generation and delivery of a wide range and variety of waveforms with high accuracy and precision. Some embodiments optionally utilize closed loop control of a therapeutic waveform, which can include continuous, repeated or substantially real-time monitoring and adjustment of a waveform, for example, throughout delivery to a patient. Since the waveform can be monitored during delivery, such as based on a specified waveform of any of a wide variety of shapes and forms, for example, and also tracked or matched very accurately, a great variety and range of waveforms can be generated and delivered. Some embodiments optionally utilize non-closed or open loop control of a therapeutic waveform, which can include a preset schedule or configuration of electrical execution that is predetermined through extensive testing, verification and validation procedures, so as to result in delivery of the desired therapeutic waveform to the patient. In some embodiments, a resonant electrical circuit based system or circuit topology allows for a high degree of control and accuracy. In some embodiments, wide bandgap materials, such as silicon carbide (SiC) or gallium nitride (GaN), further enhance performance, including enhancing the accuracy and precision of the provided waveform. Since a selected particular waveform to be provided to a patient can be critical in optimizing both therapeutic effect and reduction of risk to the patient, being able to provide a selected waveform with exactness can likewise be critical.

Some embodiments further provide greater overall performance and efficiency compared to many previously existing systems. For example, in aspects of closed or non-closed loop control, for example, use of a resonant tank or other resonant electrical circuit, use of soft switching, and use of wide bandgap materials may contribute to system performance and energy efficiency. Moreover, embodiments described herein may provide greater performance and efficiency at a smaller size or weight, or greater performance and efficiency for a given size, weight or cost. These attributes, in turn, can provide great practical advantages in terms of aspects such as portability, availability or fast availability to a potential user, often in contexts where rapid availability, even down to seconds, can be critical or even life-saving.

In some embodiments, when closed loop operation of a system is employed, such operation may enhance reducing patient risk by allowing monitoring and control during generation and delivery of a waveform. For example, if some unexpected condition causes an unplanned and undesired alteration in the waveform during delivery, then monitoring, control and adjustment can effectively allow for detection and prevention, mitigation or fast/immediate correction of any such deviation. Moreover, use of resonant electrical circuit based systems and circuit topologies, in closed loop current control embodiments, can allow unexpected developments to be detected and addressed, or as well addressed, during delivery of a waveform. However, with sufficient verification and validation design controls in place, embodiments described herein also allow for non-closed or open loop operation of the system to be implemented in accordance with aspects of the present disclosure.

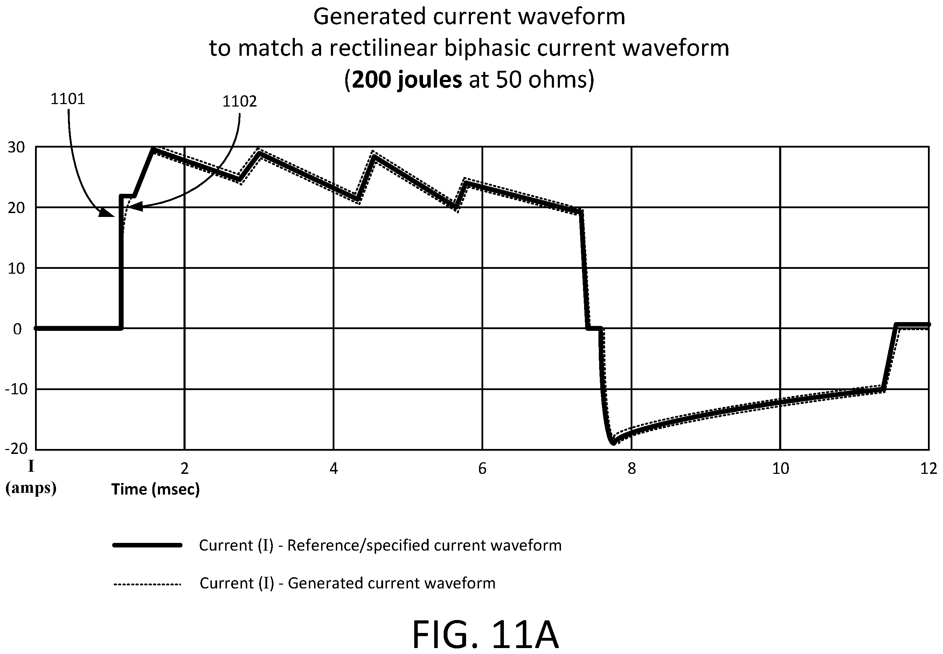

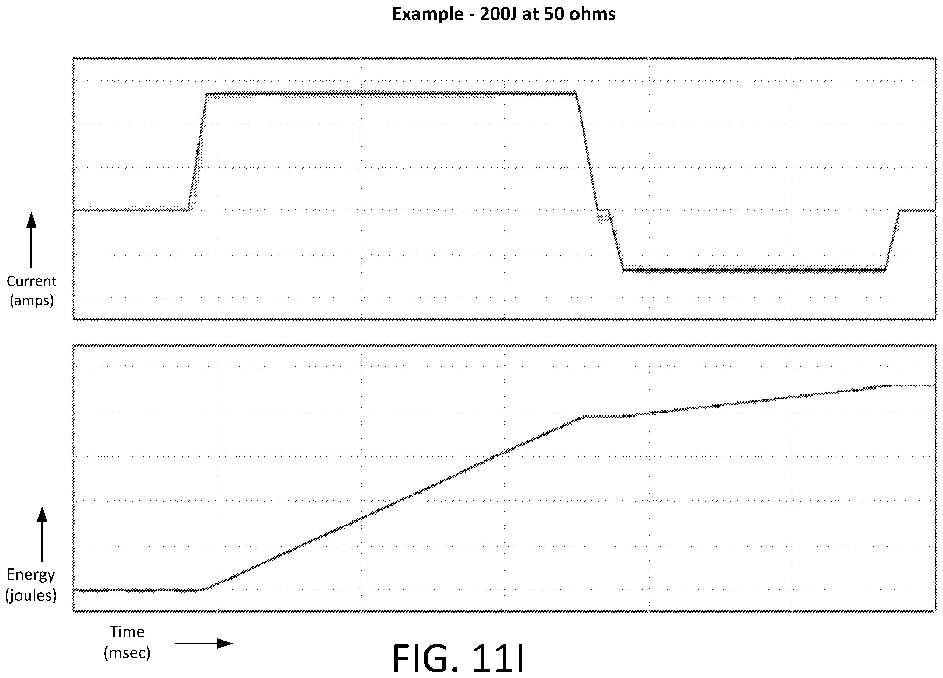

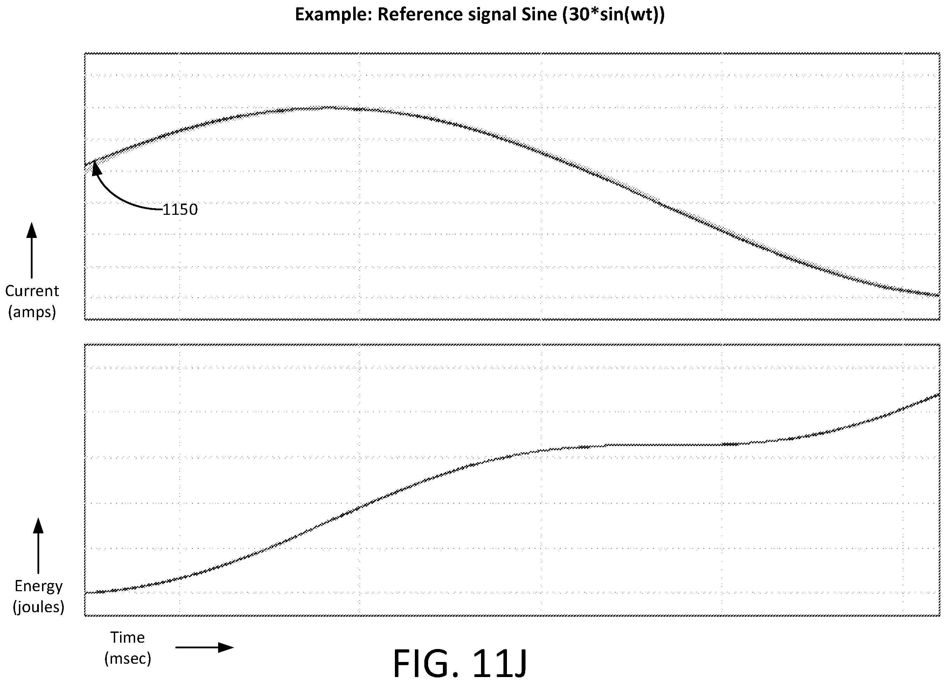

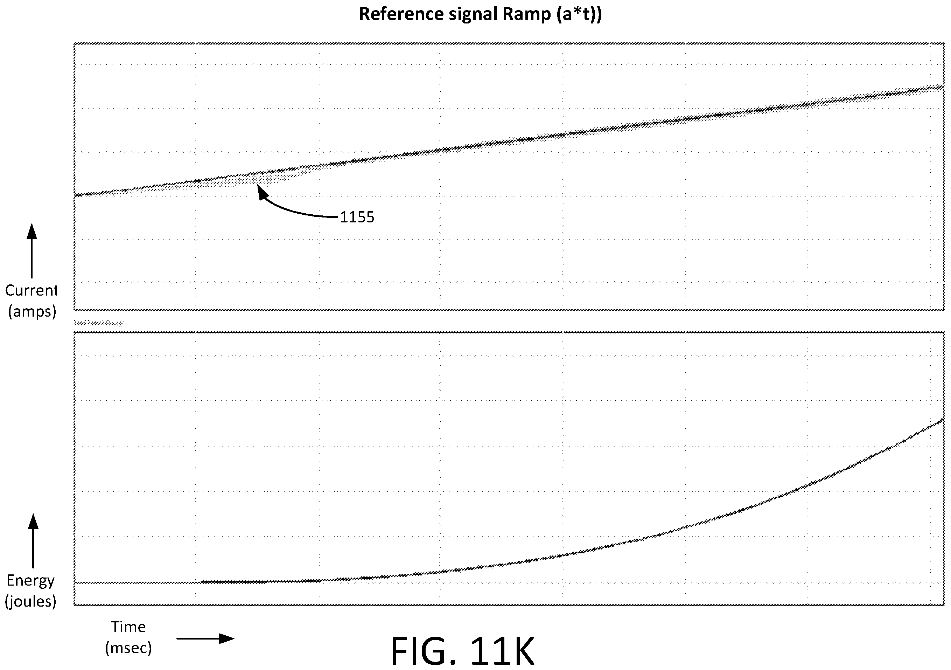

Also, some embodiments as disclosed herein are inherently stable. Still further, some embodiments provide higher bandwidth and higher dynamic range for electrotherapeutic delivery to the patient than previous implementations. Various high performance attributes, such as those mentioned above, can contribute to these aspects, including the use of a resonant electrical circuit based electrical circuit topology, use of soft switching, and use of wide bandgap materials. For example, FIGS. 11A - 11D, described in detail herein, illustrate the ability of some embodiments to track and match a waveform with great accuracy over a wide range of energy levels (for example, as depicted in FIGS. 11A - 11D, a rectilinear biphasic waveform is matched at energy levels of 200 joules and just 1 joule, respectively).

Some embodiments provide a system that provides a peak efficiency of 80, 85 or 90 percent or more in delivering an electrotherapeutic waveform to a patient, which may be measured based on energy used by the system relative to energy delivered to a patient. Furthermore, some embodiments deliver an amount of energy to a patient that is no more than 15, 10, 5 or 3 percent different, or from 0.1% to no more than any of 15, 10, 5 or 3 percent different, than an amount of energy of the specified waveform, or, for example, no more than 3 joules more or less than a specified amount of energy. Furthermore, some embodiments provide devices that, for a given required level of performance or efficiency, can be implemented in a device that is smaller, lighter, with generally fewer and smaller electrical components, and less expensive than would be possible with previously existing systems. Still further, less precise or "strict" system calibration may not be necessary, or as necessary, in embodiments of the present disclosure, relative to open loop systems, for instance, due to the system's ability to correct deviations by monitoring and adjusting a waveform during generation and delivery.

In some embodiments, previously known electrotherapy circuits can be improved, thereby significantly reducing printed board (PCB) area and thus reducing the overall volume and weight of the defibrillator.

Some embodiments of the present disclosure utilize a circuit topology and technique for optional "closed loop" control of an electrotherapeutic waveform being delivered to a patient. For example, in some embodiments, the waveform being delivered can be controlled or adjusted during its delivery to match a specified waveform with high accuracy and precision. Hence, the therapeutic current control network may actively sense or otherwise measure the current as it is being applied to the patient, and subsequently control (e.g., increase or decrease) the level of current so as to substantially track with the specified waveform. The closed loop topology may include a controller, such as a field programmable gate array (FPGA) or a digital signal processor (DSP) based controller, which controls operation of at least a portion of a therapeutic current control network, potentially among other things. A therapeutic current control network according to some embodiments includes a resonant tank, or other resonant electrical circuit, as described further herein. The controller may control, potentially among other things, operation of at least a portion of at least one switch of the therapeutic current control network to adjust the waveform over the time of delivery to the patient, which time is typically on the order of milliseconds, to match a specified waveform. The controller may also control other components of the therapeutic current control network, such as a rectifier, or other components of the overall system, in adjusting the waveform or otherwise. In some embodiments, use of a resonant tank or other resonant electrical circuit, in the therapeutic current control network, as described further herein, allows for high performance and efficiency, including by allowing faster switching for a given amount of switching loss. Closed loop topologies of embodiments described herein may offer many advantages over previously existing topologies, such as higher overall performance and efficiency, greater flexibility in terms of waveforms that can be produced or reproduced, less produced heat and thus a smaller required cooling system, fewer and smaller electrical components (which can increase system reliability), smaller footprint, greater portability, longer battery life and reduced cost. Though, as discussed above, embodiments of the present disclosure may implement non-closed loop control topologies as well, which may be sufficiently effective and safe provided that the appropriate verification and validation design controls are put in place for the therapeutic waveform to be predetermined.

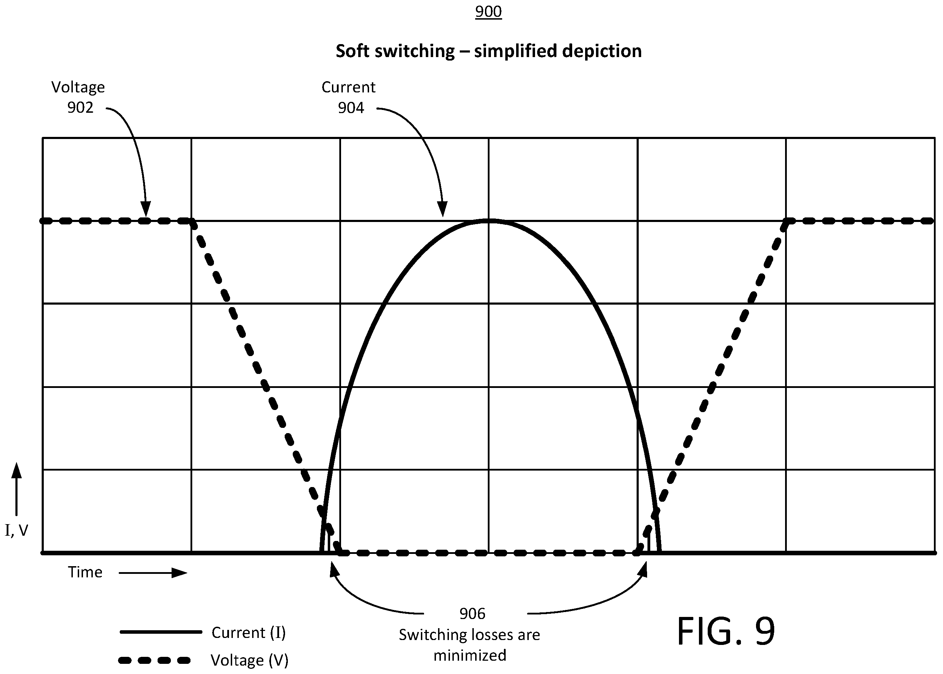

Additionally, some embodiments utilize soft switching, substantially zero voltage switching (ZVS) or substantially zero current switching (ZCS), as further described herein, such as with reference to Fig. 9, further reducing switching losses and increasing efficiency. In general, soft switching can include controlling timing of device turn-on and turn off at near zero current or near zero voltage, thus minimizing intersection of the current and voltage waveforms, which in turn minimizes energy losses associated with the switching. Additionally, in some embodiments, utilization of soft switching allows for reduced or low electromagnetic interference (low EMI) for higher system performance.

Furthermore, some embodiments use wide bandgap switch component materials such as SiC or GaN, or others, increasing performance and efficiency by enabling higher bandwidth and faster switching, resulting in minimized switching loss,, and enabling use of higher voltage devices.

The following table, Table 1, provides a general, non-limiting, example comparison of examples of some existing defibrillator aspects relative to aspects of examples of defibrillators according to some embodiments of the present disclosure.

Table 1 - comparison of examples of types of defibrillators/systems

| Row | Aspect | (1) Defibrillator using some previously existing therapeutic control systems | (2) Defibrillator using therapeutic control system according to some embodiments herein, using regular bandgap materials | (3) Miniaturized defibrillator using therapeutic control system according to some embodiments herein, using wide bandgap materials |
|---|---|---|---|---|
| 1 | Switches | SCRs | IGBT | SiC and/or GaN |
| 2 | Number of H bridge switches | 12 (3 per arm x 4) | 4 | 4 |
| 3 | High voltage component device avail-ability | No | Yes | Yes |

(continued)

| Row | Aspect | (1) Defibrillator using some previously existing therapeutic control systems | (2) Defibrillator using therapeutic control system according to some embodiments herein, using regular bandgap materials | (3) Miniaturized defibrillator using therapeutic control system according to some embodiments herein, using wide bandgap materials |
| --- | --- | --- | --- | --- |
| 4 | Usage of dump resistors | Yes | No | No |
| 6 | Energy efficiency | Lowest of the three | Middle of the three | Highest of the three |
| 7 | Filter size | Largest of the three | Middle of the three | Smallest of the three |
| 8 | Total defibrillator size | Largest of the three | Middle of the three | Smallest of the three |
| 9 | Energy storage capacitor or high voltage capacitor size | Largest of the three | Smaller than (1) | Smaller than (1) |

It is noted that Table 1 is not intended to be limiting, but rather provides a comparison of example defibrillators and systems. System (1) is an example of a previously existing therapeutic control system. System (2) is an example therapeutic control system according to some embodiments described herein, in which regular (not wide bandgap) materials are used. System (3) is an example therapeutic control system according to some embodiments described herein, in which wide bandgap materials are used.

Table 1 provides a general comparison of various aspects of the example systems, relative to each other. Specifically, Row 1 compares the types of switches used. Row 2 compares the number of H bridge switches utilized. Row 3 indicates whether high voltage component devices may be utilized. Row 4 indicates whether resistors are used to dump at least a portion of excess or unused energy from an energy storage capacitor or a high voltage capacitor. Row 5 indicates relative estimated price. Row 6 compares relative energy efficiency. Row 7 compares relative filter size. Row 8 compares the relative size of a defibrillator utilizing each system. Row 9 compares the relative size of an energy storage capacitor or a high voltage capacitor used in each system.

Additionally, some embodiments may use one or more planar transformers. By using flat windings, which may, in some embodiments, be printed on a printed circuit board (PCB), instead of copper wire coils, the height of the associated device may be reduced, and the size or footprint of the overall device may therefore also potentially be reduced. In some embodiments, planar transformers may also offer advantages, for example, in terms of providing precise electrical characteristics such as capacitance, output and aspect ratio.

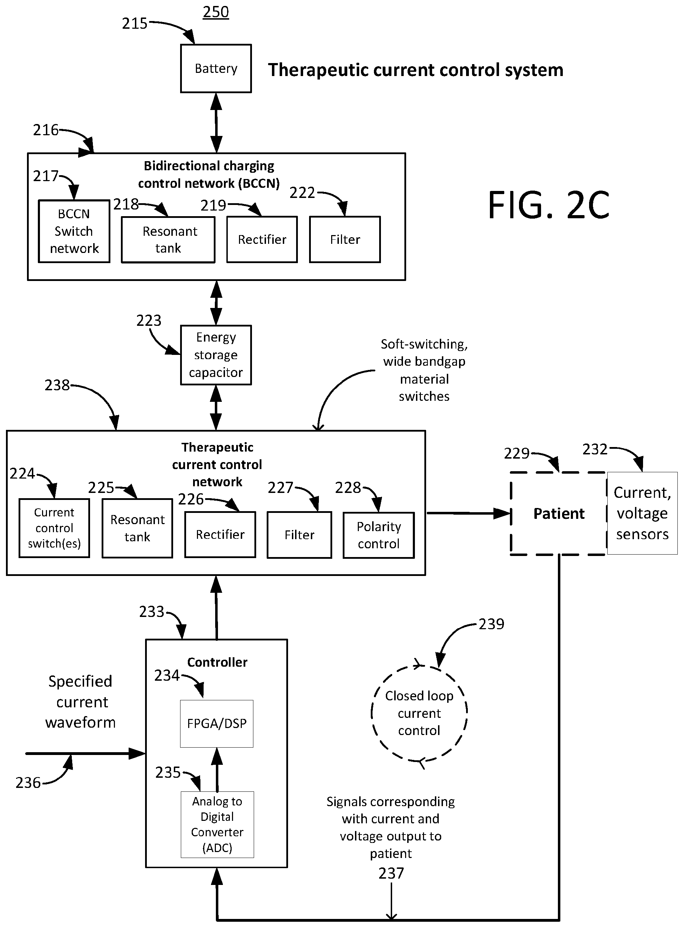

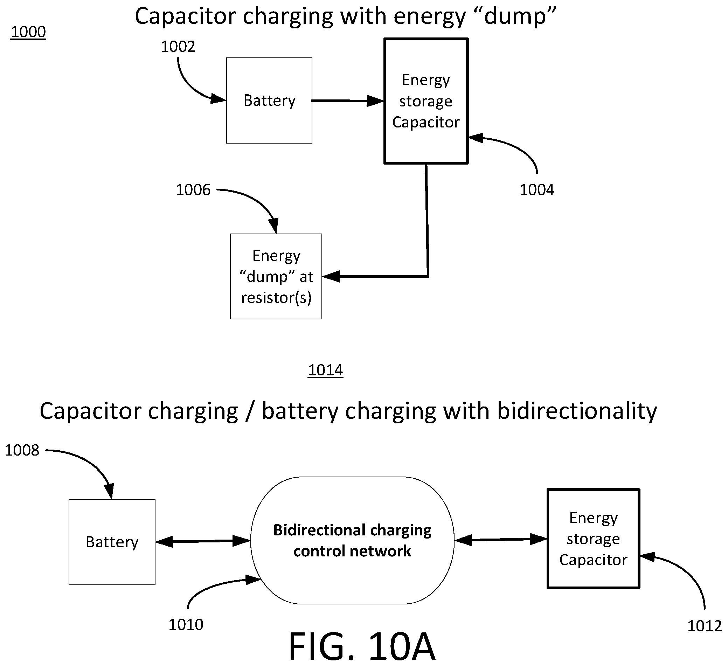

Still further, some embodiments utilize a bidirectional power supply. A battery may be used to charge an energy storage device, such as an energy storage capacitor, that is then used to provide current for an electrotherapeutic waveform. However, instead of dumping excess energy into one or more resistors, the bidirectional power supply may enable return of at least a portion of the stored capacitor energy back to the battery for storage in the battery. Relative to energy dumping at one or more resistors, this can provide for less wasted energy, less produced heat, and greater battery life, or more available defibrillation cycles or other uses before recharging of the battery is required. In some embodiments, a network including a resonant tank, or other resonant electrical circuit, may be utilized for efficient bidirectional energy transfer between the battery and the energy storage device, as further described herein. For example, some embodiments described herein may utilize a bidirectional charging control network, such as described with reference to FIGS. 2C and 10A-10D herein.

In some embodiments described herein, many SCRs may be replaced by four insulated gate bipolar transistors (IGBTs), allowing for significantly reduced printed circuit board (PCB) layout area, thereby reducing overall volume of the defibrillator. Furthermore, some embodiments described herein generally may allow use of high voltage devices, increasing the overall level of system performance. Some embodiments of systems and devices described herein may be produced at a lower cost than previously existing systems and devices. Utilization of wide bandgap materials may increase cost, but still allow a cost that is lower than the cost of previously existing systems. Some embodiments include advantages over previously existing systems including higher energy efficiency (which may be highest with use of wide bandgap materials), and smaller total size, filter size and energy storage capacitor size (all of which may be smallest with use of wide bandgap materials). Furthermore, in some embodiments, signal tracking is most accurate and precise with use of fast switching facilitated by use of wide bandgap materials

**In** some embodiments, monitoring and control of a waveform during delivery, continuously and substantially in real time, provides safety advantages over previously existing therapeutic control systems. For example, when closed loop control is employed according to some embodiments described herein, more accurate and precise matching of a delivered waveform to a desired waveform may occur. Since a desired waveform may be selected or determined for optimal positive effect and safety, better matching can mean greater positive effect and greater safety. Additionally, particular safety hazards can sometimes arise in previously existing therapeutic control systems that can be avoided in embodiments of therapeutic control systems described herein. Herein, the term "patient" is broadly intended to include any individual, whether a person or an animal, or simulated patient in the form of a defibrillation test equipment, to whom an electrotherapeutic pulse or waveform may be delivered according to embodiments described herein, whether or not such individual is within a medical or healthcare context or environment. It is noted that embodiments are included in which a waveform may be generated to match a specified waveform, but not delivered to a patient, or may be used for test or simulation purposes, such as in experimental/laboratory, training, and/or manufacturing settings. It is further noted that, herein, the term "pulse" is intended to be broadly construed, and can include, for example, among other things, any of various types of bursts, as well as any type or shape of, or particular, waveform.

According to embodiments disclosed herein, devices, or components of devices, may be provided with higher energy densities than may be achievable using previously existing systems. Herein, the term "energy density" refers to the amount of energy delivered or deliverable to a patient, such as via an electrotherapeutic pulse or waveform, by a device, relative to the size of the device. The device may be, for example, a defibrillator or mobile defibrillator. Various aspects and features of embodiments disclosed herein may contribute to allowing high energy densities. These aspects may include, for example, as described herein, device or component specifics and configurations, use of planar transformers, use of high voltage devices, use of methods and systems for generation and delivery of electrotherapeutic pulses or waveforms, overall circuit topologies, optional use of closed loop therapeutic current control systems, use of controllers within therapeutic current control systems, use of therapeutic current control networks, use of resonant electrical circuits or resonant tanks within therapeutic current control networks, use of bidirectional power supplies, use of resonant electrical circuits within bidirectional power supplies, use of synchronous or active rectification, use of fast switching, use of soft switching, use of zero current switching (ZCS), use of zero voltage switching (ZVS), and use of wide bandgap switching materials such as silicon carbide (SiC) or gallium nitride (GaN).

Some embodiments provide a computerized mobile device configured to provide an electrotherapeutic pulse to a patient may include one or more of the following features. A device may include a resonant electrical circuit that includes a resonant tank. A device may be configured to provide an electrotherapeutic waveform for defibrillation or pacing. A device may be configured to deliver an electrotherapeutic waveform to the patient, wherein the electrotherapeutic waveform has an energy of between, for example, 1 - 400 or 50 - 200 joules. A device may have an energy density, given by an energy of the electrotherapeutic waveform to be delivered to the patient relative to a volume defined by the housing, for example, of between 0.5 - 1.00, 1.00 - 2.00, 2.00 - 3.00 or 3.00 - 4.00 joules per cubic centimeter. A device may have an energy density of 4.00 - 6.00 joules per cubic centimeter. A device may have an energy density of over 4.00 joules per cubic centimeter. A device may have an energy density of over 6.00 joules per cubic centimeter. A device may have an energy density of less than 0.5 joules per cubic centimeter. A device may have a volume defined by the housing, for example, of between 400 - 1,200, 200 - 800 or 100 - 400 cubic centimeters. A device may have a volume, defined by the housing, of less than 100 cubic centimeters. A device may have a volume, defined by the housing, of greater than 1,200 cubic centimeters. A device may have at least one planar transformer of, for example, between 2,500 - 4,000, 2,750 - 3,500 or 3,000 - 3,300 volts. An electronic system may include at least one switching device including a wide bandgap material. A device may include at least one switching device that may include at least one of silicon carbide (SiC) or gallium nitride (GaN). At least one switch or a switch network may include a plurality of switches, and wherein at least a portion of the switches includes a semiconductor material with a band gap of, for example, between 2 and 6 ev. An electronic system may include at least one switching device with a switching speed, for example, of between 150 - 500, 200 - 600, 300 - 500 or 375 - 500 kilohertz. An electronic system may include at least one switching device with a switching speed of less than 150 kilohertz. An electronic system may include at least one switching device with a switching speed of greater than 500 kilohertz.

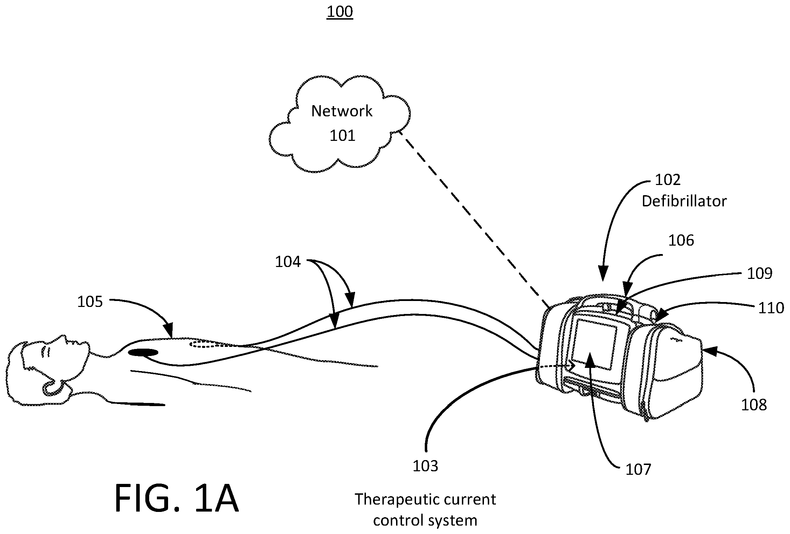

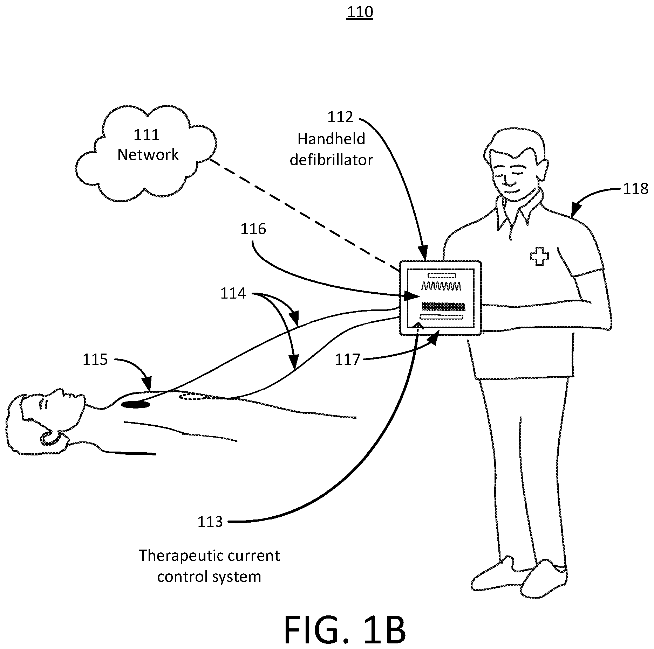

FIGS. 1A and 1B illustrate examples of uses 100, 110 of defibrillators 102, 112, employing therapeutic current control systems 103, 113 to provide electrotherapy defibrillation current to patients 105, 115. Use of a resonant electrical circuit and/or closed loop therapeutic current control may allow delivery of an electrotherapeutic waveform to a patient with greater safety, accuracy and precision than previously existing systems, while also permitting use of a smaller, lighter, more portable or less expensive defibrillator. Additionally, therapeutic current control systems may enable generation and delivery of a wide variety of waveforms, including waveforms that may be desired to be delivered to a patient. It is noted that, while not shown in FIGS. 1A and 1B, in some circumstances, electrotherapy may be provided in tandem or combination with other therapies, such as chest compressions, ventilation, or drug therapies. For example, during a cardiac arrest protocol, a rescuer may deliver cardiopulmonary resuscitation (CPR), such as in the form of chest compressions and, in some cases, ventilations, followed by a period of ECG analysis to determine whether the heart is in need of a defibrillation shock. Embodiments described herein provide for a compact, efficient electrical circuit topology for generating such a defibrillation shock, in accordance with a desired current waveform.

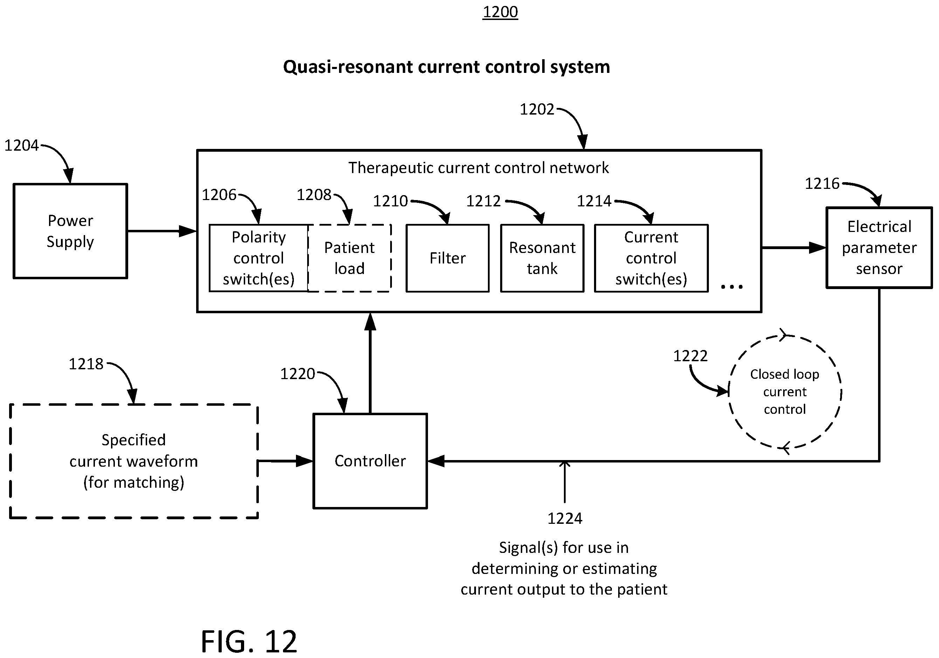

More particularly, FIG. 1A illustrates an example 100 of use of a defibrillator 102 incorporating a therapeutic current control system 103. While various methods of use are possible, in the method depicted, two electrodes 104 are placed and oriented on the torso of the patient 105 for delivery of defibrillation current through the heart of the patient 115. The defibrillator 102 includes a carrying case 110 with a handle 106 and side storage pockets 108 that may be used to store peripherals such as tablets, sensor accessories (e.g., blood pressure cuff, electrodes) and/or other electronic devices that may be adapted for use in coordination with the defibrillator 102. The defibrillator 102 also includes a monitor 107 for use by a health care provider or other user who is using the defibrillator to provide defibrillation electrotherapy to the patient 105. The defibrillator 102 and any peripheral electronic devices may be connected to one or more networks 101 via one or more wired or wireless connections. Details of medical devices, such as a defibrillator as well as other devices that may be connected to one or more networks, are provided with reference to FIGS. 12-13.

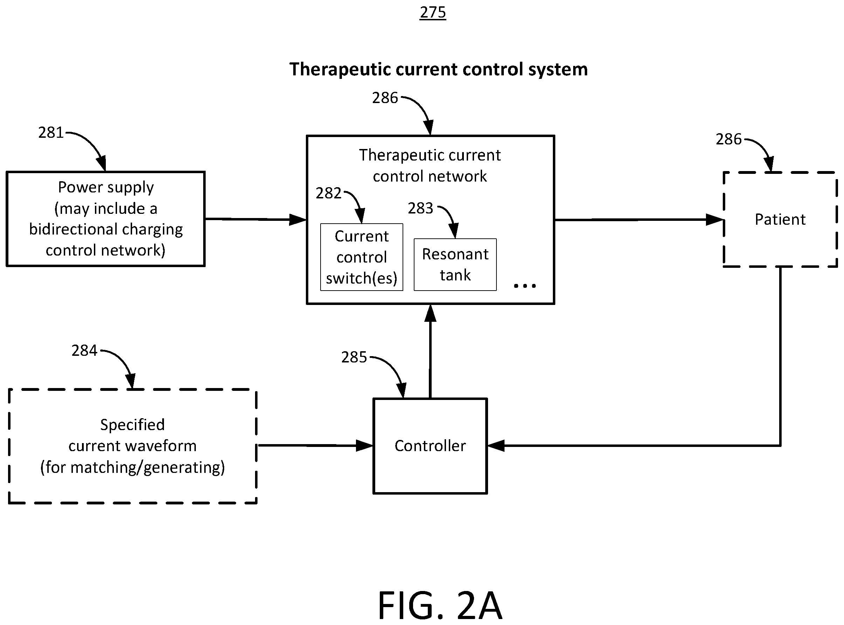

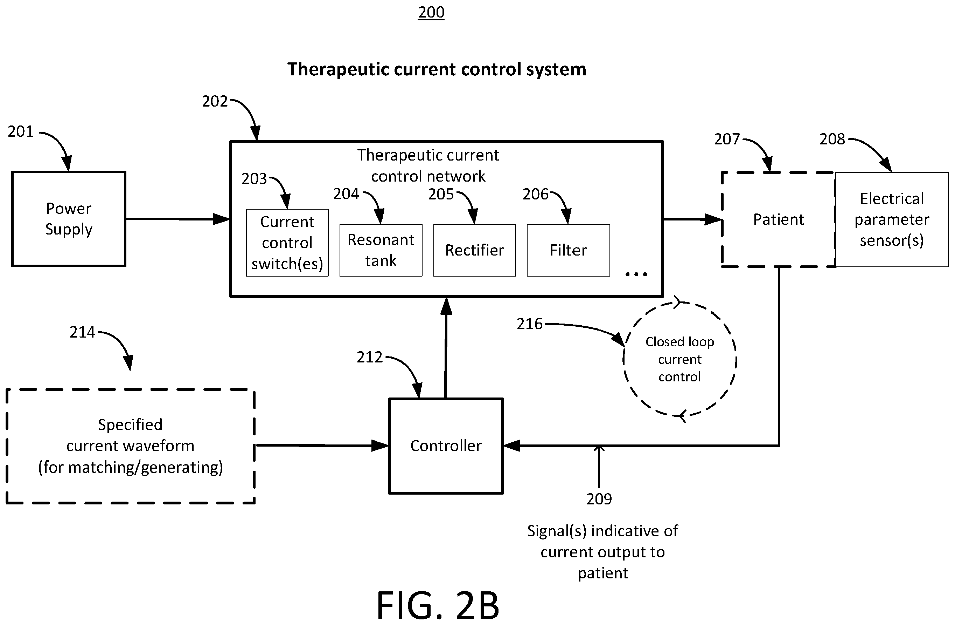

The therapeutic current control system 103 may include electronic circuitry and components partially or fully contained within a housing 109 of the defibrillator 102. Details of example closed loop therapeutic current control systems are provided herein, including with reference to FIGS. 2-5 and 12-17. The defibrillator 102 and therapeutic current control system 103 may be used in delivering an electrotherapeutic defibrillation waveform to the patient 105.

FIG. 1B illustrates an example of use 110 of a handheld defibrillator 112, with a monitor 116, incorporating a therapeutic current control system 113 (in various embodiments of the present disclosure, non-closed loop and closed loop therapeutic current control systems may be employed), being used by a health care provider or other user 118. As depicted, two electrodes 114 are placed on the torso of the patient 115 for delivery of defibrillation current through the heart of the patient 115. The defibrillator 102 and any associated peripheral electronic devices may be connected to one or more networks 111 via wired or wireless connections. As discussed herein, embodiments of circuit topologies of the present disclosure provide for the ability to integrate safe and effective defibrillation components into a mobile computing device, such as a tablet, phone, or other portable device. Such a system may allow for greater access of such devices to the general public, as well as emergency medical services (EMS). Further, during a cardiac medical event, it is of utmost importance for the rescuer to begin treatment on the patient immediately. Accordingly, embodiments described may help to save precious time for EMS personnel to reach the patient, not having to carry heavy and sometimes awkward equipment to the patient right away. In addition, as such embodiments may be integrated into a portable computing device as small as a tablet or phone, it is possible for lay users to be equipped with, or may otherwise have easy access to, a handheld defibrillator 112. This would allow people who are in close proximity to the medical emergency to begin treatment well before EMS or other trained personnel arrive at the scene.

The therapeutic current control system 113 may include electronic circuitry and components partially or fully contained within a housing 117 of the handheld defibrillator 112. The handheld defibrillator 112 and the therapeutic current control system 113 are used in delivering an electrotherapeutic defibrillation waveform to the patient 115. In some embodiments, use of a therapeutic current control system can allow for a smaller, lighter associated device, such as a defibrillator, for a given set of required performance or power parameters. For example, as described further herein, embodiments of the present disclosure would not require bulky components, such as large resistors or considerably sized high voltage capacitors, which are commonly associated with conventional defibrillators. In some cases, this can allow for a portable, wearable or handheld defibrillator or other electrotherapy device with sufficient performance or power parameters, such as might not be practical or possible with previously existing systems.

FIGS. 2A, 2B and 2C are block diagrams of example therapeutic current control systems 200, 250, 275 of the present disclosure that can be used, for example, in defibrillators, pacemakers or other electrotherapy devices, which may include portable devices.

In FIG. 2A, the therapeutic current control system 275, which may, in various embodiments, be closed loop or non-closed loop, includes a power supply 281 electrically coupled with a therapeutic current control network 202. The power supply 201 may be or include an energy storage device such as a high voltage capacitor, which may be charged, such as by a battery or other power source, prior to delivery of electrotherapy current to the patient 207. Various types of capacitors can be used in various embodiments, which may include, for example, film-foil capacitors or metallized film capacitors, as well as capacitors utilizing various materials, including, for example, aluminum electrolytic capacitors, ceramic capacitors, polycarbonate, polypropylene, polystyrene, tantalum or niobium, among others.

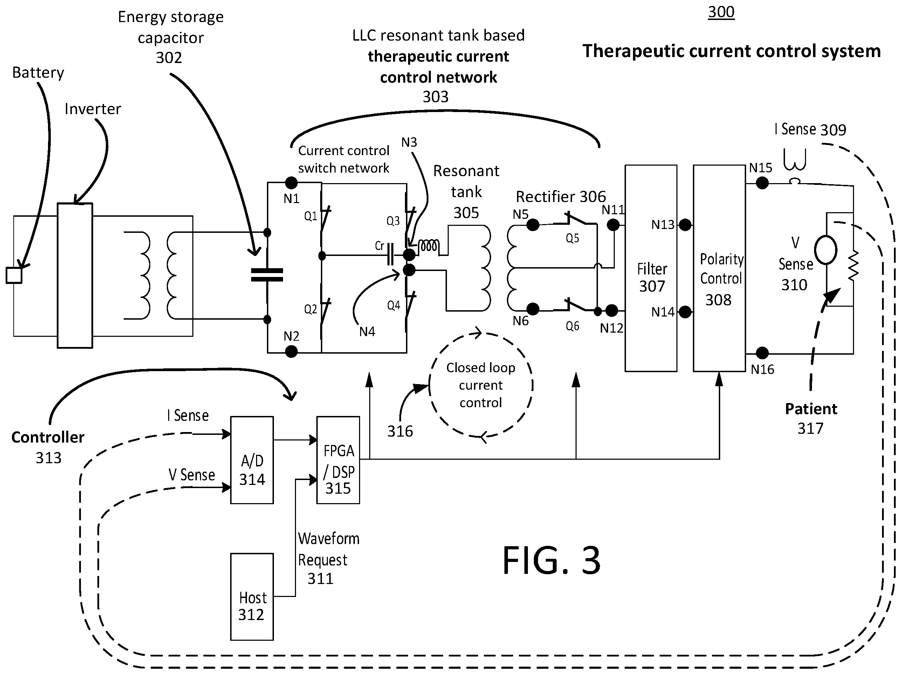

The therapeutic current control network 286 may include at least one current control switch 282 (e.g., one or more switches and/or a switch network for controlling the therapeutic current), a resonant tank or other resonant electrical circuit 283 and a controller 285. Input to the controller 285 may include a specified current waveform 284. Examples of embodiments of controllers are provided, for example, with reference to various later figures, including FIGS. 2C and 3. In various embodiments, the therapeutic current control network 202 may include closed or non-closed loop current control, such as with regard to an electrotherapeutic waveform generated and delivered to a patient. Some non-closed loop systems may utilize a selected pre-set schedule (e.g., according to a predetermined switching schedule or look up table when switches are used to control therapeutic current delivery) and/or series of resistors to shape the delivered waveform to correspond with a specified waveform or type of waveform. For example, in a non-closed loop therapeutic current control network with a resonant electrical circuit or resonant tank, current provided by an energy storage capacitor, to be delivered to the patient via the therapeutic current control network, may flow through the resonant electrical circuit or resonant tank.

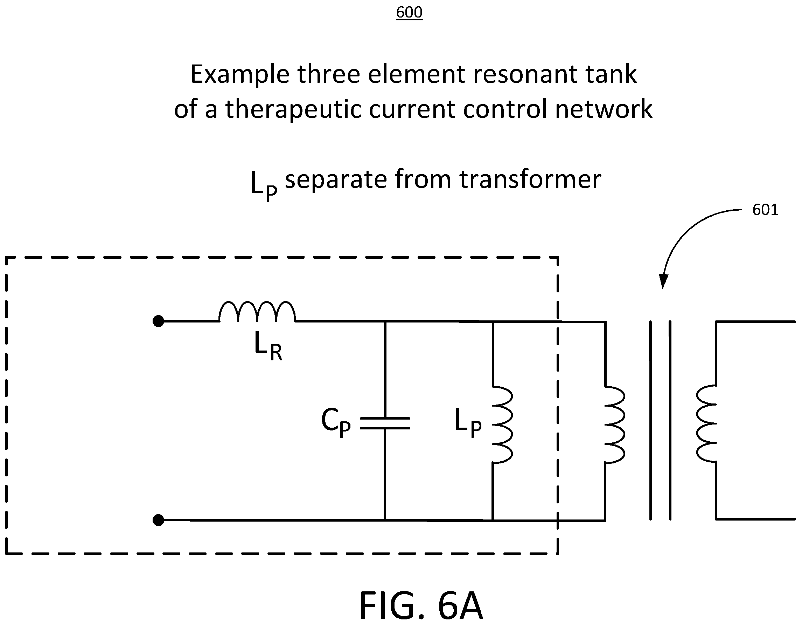

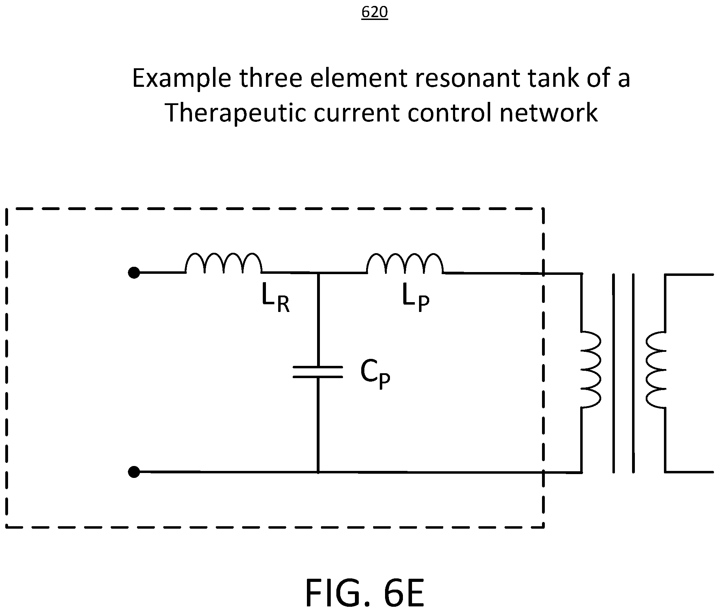

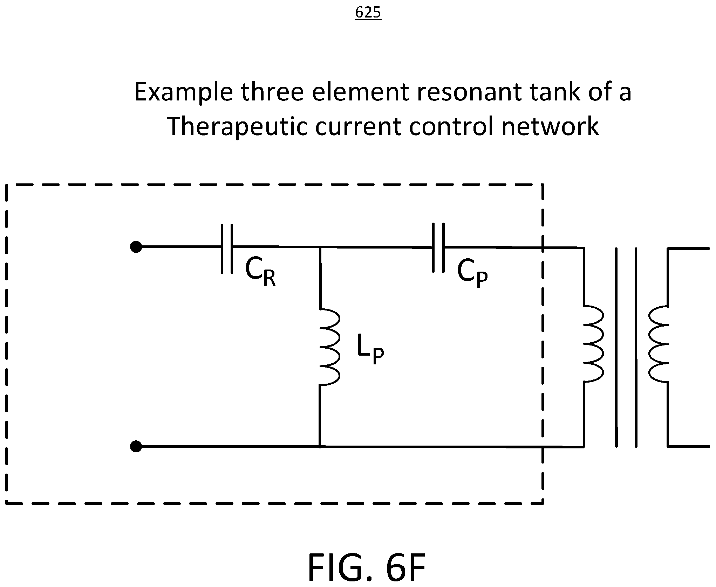

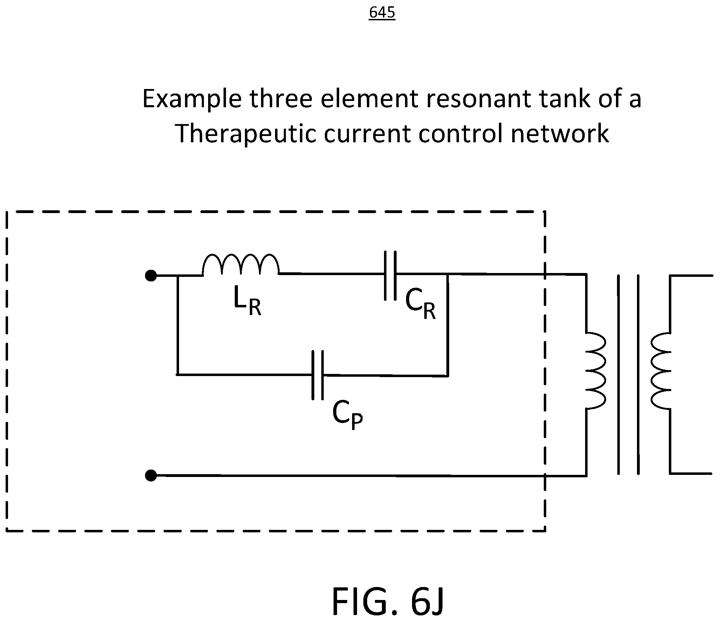

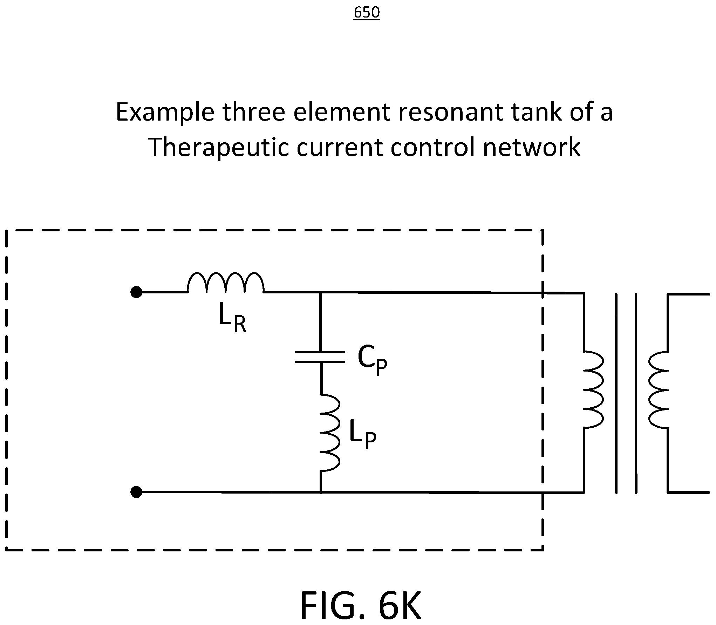

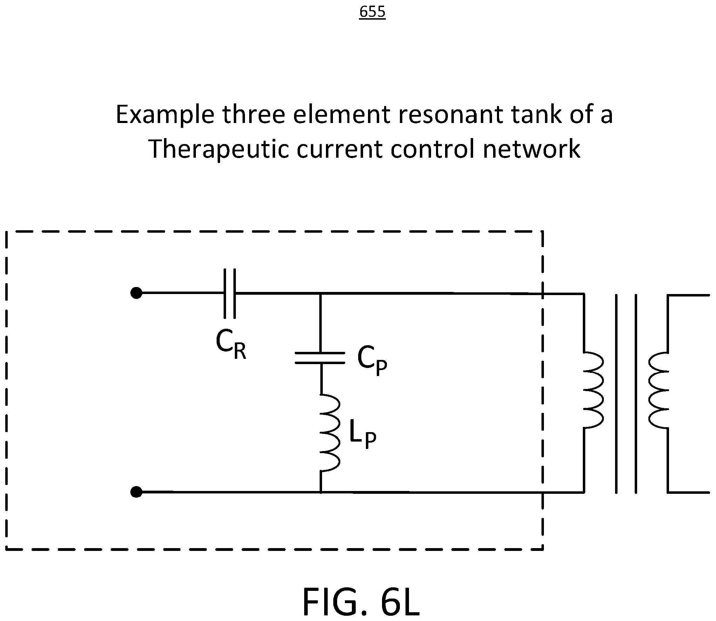

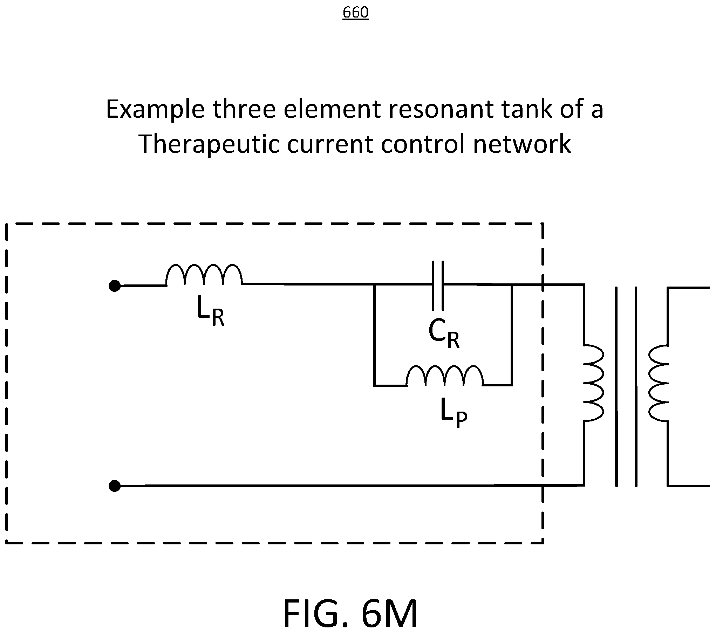

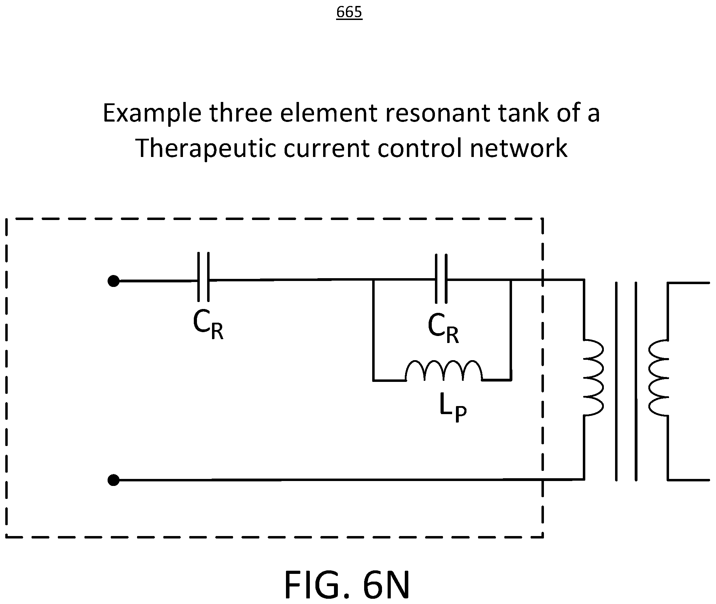

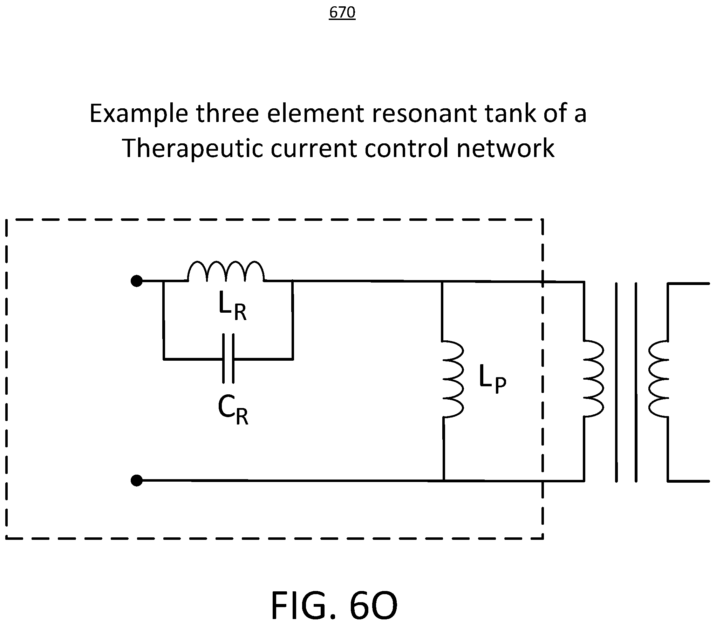

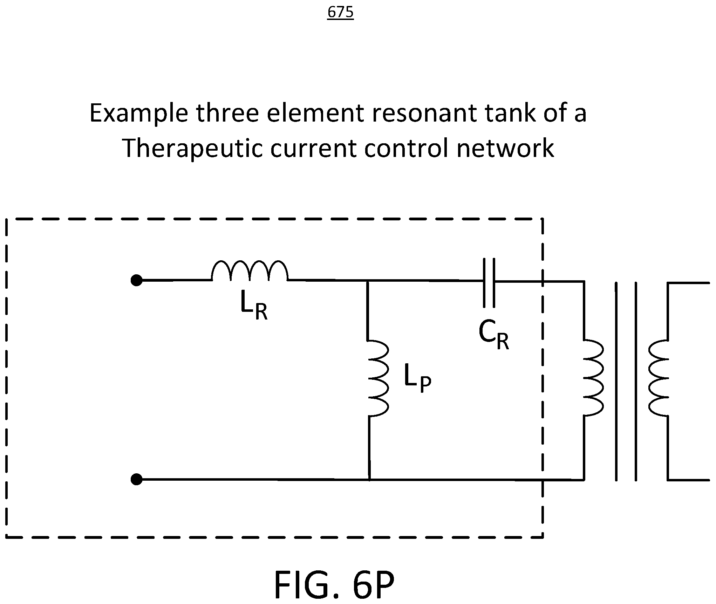

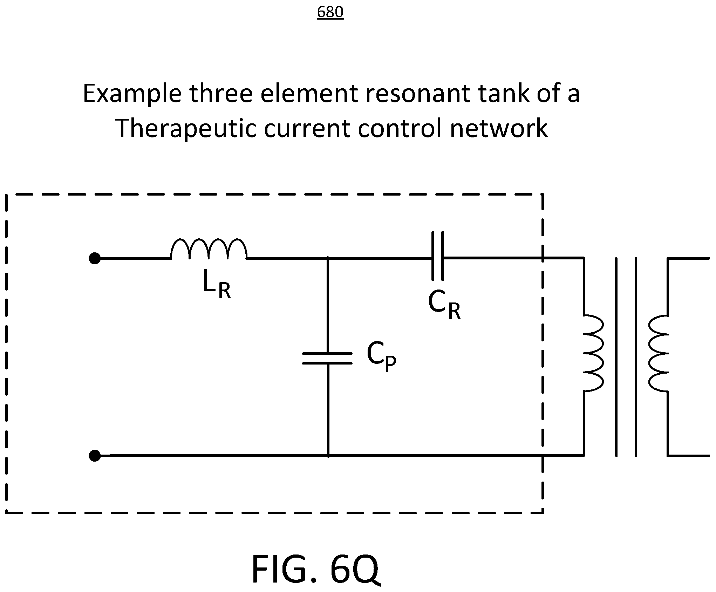

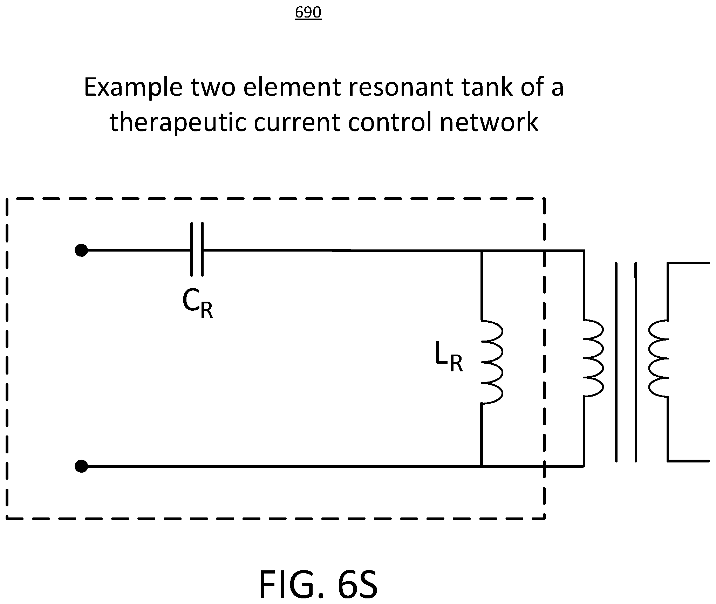

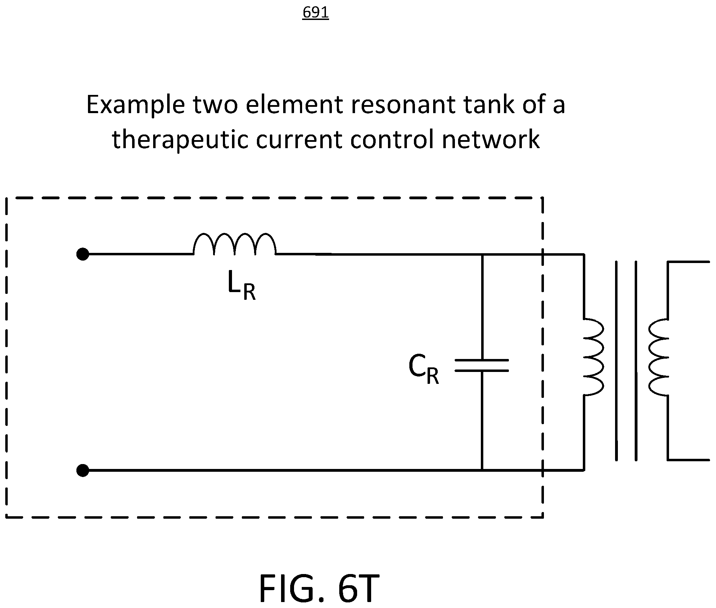

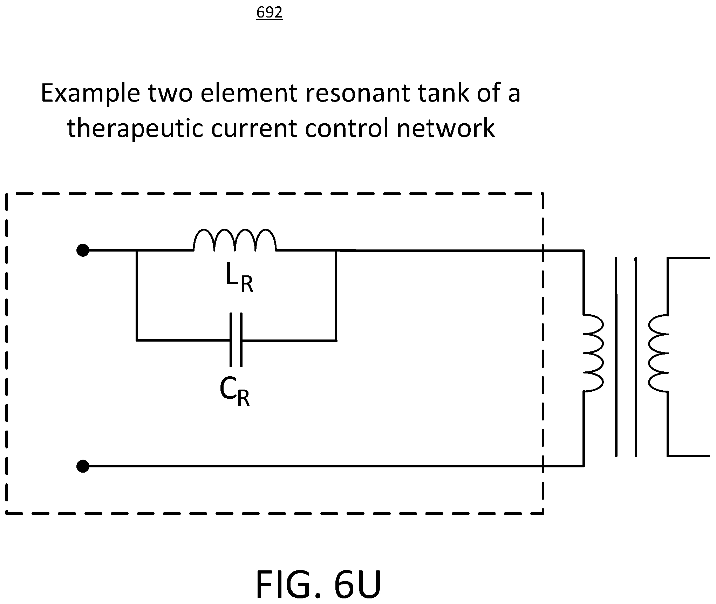

The resonant tank 283 may, in some embodiments, include one or more inductors and one or more capacitors that act as an electrical resonator, storing energy oscillating at the resonant tank's resonant frequency or frequency range. Non-limiting examples of resonant tank configurations that may be used in a therapeutic current control network are provided in FIGS. 6A - 6V. In various embodiments, a resonant tank may be beneficial to effectively store energy in a manner that minimizes or otherwise reduces the overall loss or dissipation of energy that may occur in other energy storage configurations. This may be advantageous in the context of defibrillation where a substantial amount of energy is stored and released over a short period of time. Other aspects and advantages of resonant tanks, as well as other components and aspects of therapeutic current control systems and controllers, including various electrical components and configurations, are described with reference to other figures, including, for example, FIG. 2B and FIG. 12.

In various embodiments, power supply 281 of the therapeutic current control network 202 may or may not include or make use of a bidirectional charging control network, embodiments of which are described in detail with regard to figures including FIGS. 2C and 10A-D, such as may be used in connection with a battery and an energy storage capacitor of the power supply, for example.

FIG. 2B is a block diagram of an illustrative example of a therapeutic current control system 200. The therapeutic current control system 200 includes a power supply 201 electrically coupled with a therapeutic current control network 202. The power supply 201 may be or include an energy storage device such as a high voltage capacitor, which may be charged, such as by a battery or other power source, prior to delivery of electrotherapy current to the patient 207. Various types of capacitors can be used in various embodiments, examples of which are described with reference to FIG. 2A.

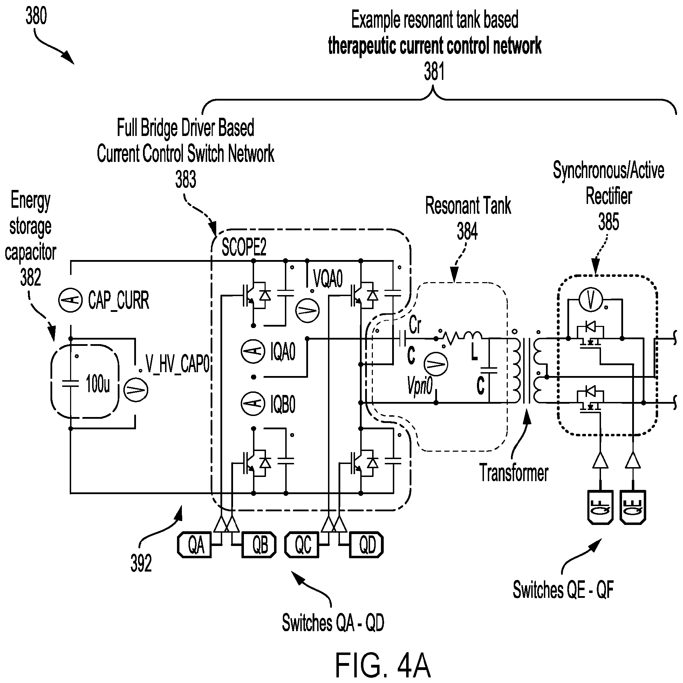

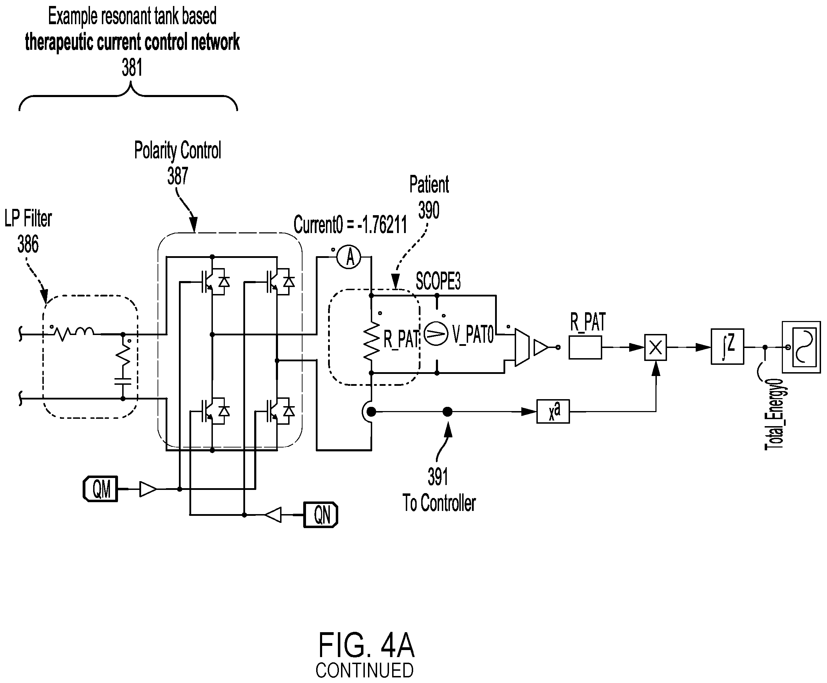

The therapeutic current control network 202 may include at least one current control switch 203, a resonant tank 204, a rectifier 205 and a filter 206. Components of an example corresponding therapeutic current control network are described in detail with reference to FIG. 4A herein.

Generally, by way of example and without limitation, components of the therapeutic current control network 202 may be configured or function as follows. The at least one current control switch 203 may include a switch network including one or more bridge circuits, such as a half bridge or a full bridge that control the flow of current from the power supply 201 through sections of the therapeutic current control network 202, and ultimately to the patient 207. As described below, a controller 212 may control operation of at least a portion of the at least one current control switch 203 of the therapeutic current control network 202, and may control other components of the therapeutic current control network including the rectifier 205 or filter 206, or other components of the system 200, to control or adjust a level of current being delivered to the patient 207.

In some embodiments, the resonant tank 204 may include one or more inductors and one or more capacitors that act as an electrical resonator, storing energy oscillating at the resonant tank's resonant frequency or frequency range. Non-limiting examples of resonant tank configurations that may be used in a therapeutic current control network are provided in FIGS. 6A - 6V. In various embodiments, a resonant tank may be beneficial to effectively store energy in a manner that minimizes or otherwise reduces the overall loss or dissipation of energy that may occur in other energy storage configurations. This may be advantageous in the context of defibrillation where a substantial amount of energy is stored and released over a short period of time.

The rectifier 205 may be used in converting alternating current to direct current. The rectifier may be a passive rectifier, or it may be an active or synchronous rectifier for performing active or synchronous rectification, which may be more efficient than a passive rectifier. Diodes may be used in passive rectification, where current is effectively permitted to flow in a single direction, once a threshold potential has been reached. Alternatively, in active or synchronous rectification, diodes may be replaced by actively controlled switches, which permit current to flow upon actuation of the switch(es) according to controlled timing.

The filter 206 may be used in electrical signal frequency filtering, for example, by allowing certain frequencies to pass through the network, thereby altering the bandwidth characteristic of the current control loop.

In the illustrative embodiment of Fig. 2B, the therapeutic current control network 202 is electrically coupled with the patient 207 for delivery of current to the patient 207, and to an electrical parameter sensor 208 (or more than one) configured to sense one or more electrical parameters of the circuit that may be, alone or in combination with other available information, indicative of current flow to the patient. In some examples, the electrical parameter sensor(s) 208 is configured for sensing a current output to the patient 207 during and over a period of delivery of current to the patient. In other examples, the electrical parameter sensor 208 is configured to sense voltage.

A signal 209 corresponding with the sensed electrical parameter, which sensed signal may vary over the period of delivery of the current to the patient, is received by the controller 212, which is electrically coupled with the electrical parameter sensor 208. It can be appreciated that various other types of sensors for measuring or sensing a signal indicative of current delivered to the patient may be employed. The signal(s) 209 indicative of the sensed electrical parameter may take the form of sensing the current and/or voltage across the output circuit nodes to the patient (e.g., current/I sense 309 or voltage/V Sense 310). In some examples, the controller 212 may then determine current output based at least in part on the current and/or voltage measurement. In various embodiments, a current and/or a voltage sensor(s) may also be included and coupled with the patient, for sensing a current and/or a voltage associated with a current output to the patient 207, and one or more signals corresponding with the sensed current and/or voltage may be received an utilized by the controller 212.

In some embodiments, a controller may utilize and compare signals, and/or, in some embodiments, parameters associated with signals may be derived, calculated, generated or obtained, such as at least in part based on associated signals, and utilized or compared, or some combination of both signals and parameters may be utilized and compared. In some embodiments, a controller may derive, calculate or generate such parameters, whereas in some embodiments, such parameters may be derived, calculated or generated, at least in part, outside of the controller. Furthermore, in some embodiments, such parameters (or signals) may be obtained within the controller and/or may be obtained from outside the controller, such as by the controller requesting them and/or by the parameters (or signals) being input to the controller. Furthermore, in some embodiments, during delivery of current to the patient 207, the impedance of the patient 207, which may vary slightly during delivery of the waveform, may be monitored and associated information may be utilized by the controller 212 to appropriately adjust, if and as necessary, the delivery of electrical therapy, to ensure that the desired waveform is as accurately as possible matched and provided to the patient. This can be an advantage of some embodiments of the present disclosure, in that many previously existing systems are not capable of monitoring and utilizing information regarding variations in patient impedance that may occur during delivery of a waveform to a patient, and therefore cannot adjust the waveform accordingly. Additionally, in some embodiments, other physiological patient parameters may be sensed and/or monitored, and obtained information may be utilized by a controller in determining or optimizing a waveform generated and delivered to the patient. It is noted that, in open loop systems, patient impedance is not actively monitored and utilized during delivery of a waveform on a continuous basis, but rather measured at a single instance to calibrate the waveform.

A specified waveform 214 is input to the controller 212, prior to or during delivery of the electrotherapy current to the patient 207. As conceptually indicated by broken circle 216 the controller 212 may be used in optionally providing closed loop control of an electrotherapeutic waveform as it is being delivered to the patient 207. When used in this context, the controller 212 may in effect monitor (by utilizing the received signal 209, for instance) and adjust the current waveform being delivered to the patient during and over the period of delivery. In a cyclical, repeating fashion over a time of delivery (typically on the order of milliseconds) of the electrotherapy current to the patient 207, based at least in part on the received signal 209, which may vary during delivery, the controller 212 may continually adjust the current being delivered to the patient to as closely as possible match the specified waveform 214. In particular, the controller 212 may control, potentially among other things, operation of at least a portion of the at least one current control switch 203 of the therapeutic current control network 202 to control or adjust a level of current or waveform being delivered to the patient 207. In various embodiments, the controller 212 operates in a non-closed loop control fashion, for example, where the electrotherapeutic waveform is predetermined according to a preset schedule such as in a look up table where switch control is prearranged so as to result in current delivery according to the desired electrotherapeutic waveform.

FIG. 2C is a block diagram of an example therapeutic current control system 250, including a battery 215 and bidirectional charging control network 216. In this illustrative embodiment, the bidirectional charging control network 216 includes a bidirectional charging control switch network 217, a resonant tank 218 or other resonant electrical circuit, a rectifier 219 and a filter 222. Details of an example bidirectional charging control network are provided with reference to FIGS. 10A-D herein. Although a bidirectional charging control network 216 including a resonant tank 218 is shown, in other embodiments, other types of power converters without resonant tanks can be used in the bidirectional charging, such as a buck-boost or flyback converter, for instance. However, a resonant tank based bidirectional charging control network 216 may provide more efficiency in the manner in which energy is transferred between the battery 215 and the energy storage capacitor 223.

The bidirectional charging control network 216 is used both in facilitating charging of the energy storage capacitor 223 by the battery 215 or potentially a different power source, as well as in returning energy from the energy storage capacitor 223 to the battery 215, providing charge to the battery 215 to prolong its storage life. In various embodiments, bidirectional charging control networks are provided that may be used with or in closed or non-closed loop current control systems. The energy storage capacitor 223 is charged prior to use in providing energy for generating and delivering an electrotherapeutic waveform to a patient. The bidirectional charging control network 216 may be used in returning at least a portion of such excess energy back from the energy storage capacitor 223 to the battery 215 to be stored at the battery 215. This can reduce waste and produced heat, as well as increase battery life, increase battery uses available before recharge of the battery, or allow for use of a smaller battery. As such, it can contribute to increased system efficiency and potentially smaller and lighter system size, a smaller cooling system, and smaller battery size, for example, as may be appropriate or needed for a particular use or a given set of performance requirements or minimum performance requirements. For example, systems where unused energy is dumped from the energy storage capacitor require circuit components (e.g., resistors, heat sinks) dedicated to address the effects of this action in a manner that does not detrimentally affect other parts of the system. Hence, a bidirectional charging control network may alleviate the necessity to build in such additional components, which could otherwise lead to inefficiencies and space constraints.

A therapeutic current control network 238 is electrically coupled with the energy storage capacitor 223 and includes at least one current control switch 224, a resonant tank 225, a rectifier 226, a filter 227 and a polarity control element 228. The polarity control element 228 may be for use in control of direction of current flow. It may be therapeutically beneficial for an electrical current to pass through a patient in two directions. For example, in the case of defibrillation, the polarity control element 228 may provide for a biphasic shock where the direction of current flow is reversed during electrical discharge. In some embodiments, the polarity control element 228 employs an H-bridge switch configuration. Details of example resonant tank configurations that can be used in various embodiments of a therapeutic current control network 238 are described herein with reference to FIGS. 6A-6V.

The therapeutic current control network 238 is electrically coupled with the patient 229 as well as current and/or voltage sensors 232 for sensing a current level and/or voltage level associated with or output to the patient 229.

A controller 233 receives signals 237 corresponding with the sensed current and/or voltage. Various types of controllers are contemplated in different embodiments. In the embodiment depicted in FIG. 2C, the controller 233 may be field programmable gate array (FPGA) based or digital signal processor (DSP) based, as depicted by block 234. As depicted, the controller 233 also includes an analog to digital converter (ADC) 235 that receives the signals 237 corresponding with current and voltage output to the patient 229. The controller 233 may also include various other components that are not depicted.

The controller 233 controls at least a portion of the at least one current control switch 224 of the therapeutic current control network 238, and may control other aspects of operation of the therapeutic current control network 238 or of the system 250, to adjust current being delivered to a patient or for other purposes. The controller 233, when optionally used in a closed loop fashion as conceptually depicted by broken circle 239, adjusts a current level being delivered to the patient 229 to match a specified current waveform 236 input to the controller 233, and may perform other control operations within the system 250 as well. In various embodiments, the controller 233 is used in a non-closed loop control fashion, as discussed herein, for example, where the electrotherapeutic waveform is predetermined according to a preset schedule such as in a look up table where switch control is prearranged so as to result in current delivery according to the desired electrotherapeutic waveform.

Various types and embodiments of controllers are contemplated in various embodiments of the present disclosure. For example, a controller may be digital or analog, including fully analog, and various embodiments may or may not use components such as a CPU, a processor or a microprocessor. It is noted that, in some embodiments, including some embodiments using non-closed loop control, a controller may not need to, for example, process a signal associated with, for example, at least one sensed electrical parameter, or compare such a processed signal with a second signal, for example, associated with a specified waveform.

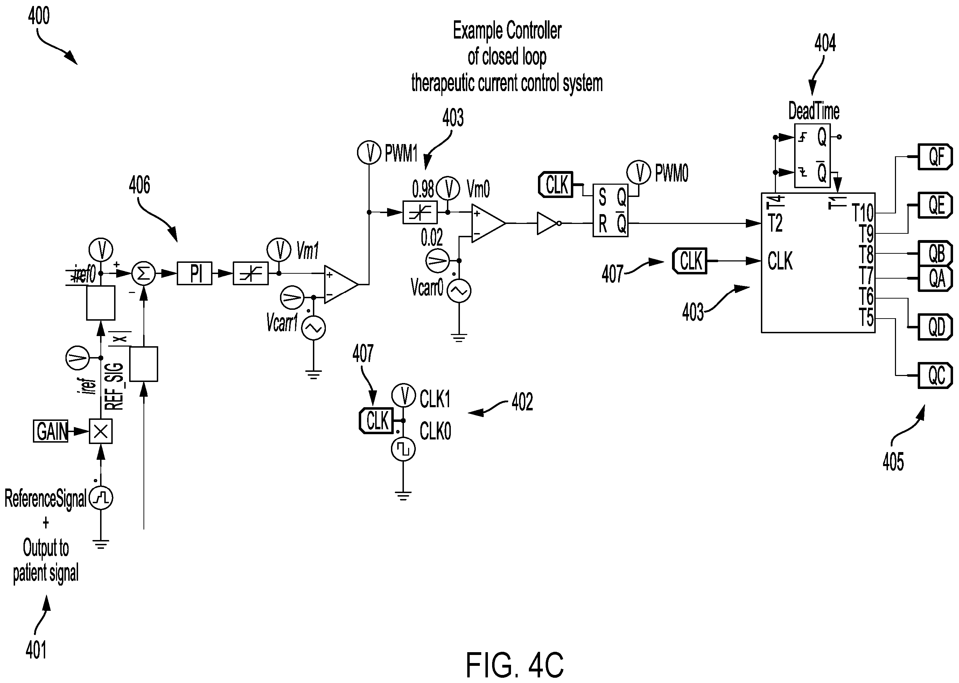

In some embodiments, the controller 233 may effectively or actually implement or execute a control algorithm or control loop. For example, the controller 233 may be or include a proportional-integral-derivative (PID) controller, a proportional-integral (PI) controller, or be or include an error amplifier (implemented as digital or analog), or another form of controller, digital or analog. Generally, the controller 233, based on input including the signals 237 corresponding with the sensed current and voltage and the specified current waveform 236, outputs one or more signals to control operation of at least a portion of the at least one current control switch 224 of the therapeutic current control network 238, as well as potentially other components, such as the rectifier 219 in the case of synchronous rectification, or active rectification, where active switch components rather than passive diodes are used, for example. In some embodiments, the controller 233 processes the signals 237 at least in the sense of receiving them and utilizing them as input. Details of an example controller are provided with reference to FIG. 4C herein.

In some embodiments, a controller processes a signal relating to at least one sensed electrical parameter indicative of current flow to a patient, such as a current parameter and/or a voltage parameter. The controller may then compare the signal, and/or an obtained parameter associated with the signal, with a second signal or parameter associated with a specified waveform (or may generate, utilize or compare parameters associated with signals, as described above with reference to FIG. 2B, for example). Based at least in part on the comparison, the controller may control, at least, operation of at least a portion of at least one current control switch of a therapeutic current control network to adjust control of an electrotherapeutic waveform being delivered to the patient to correspond with the specified waveform.

FIG. 3 is a schematic diagram of an illustrative example therapeutic current control system 300. It is to be understood that, with reference to diagrams that depict circuit components, for instance, additional circuit components beyond those that are depicted may be included. An "electrical circuit" includes one or more electrical components. A "resonant electrical circuit" is a type of electrical circuit that includes inductance and capacitance. A "resonant tank" is a type of resonant electrical circuit.

A battery 301 is shown electrically coupled with an energy storage capacitor 302. Although not shown in FIG. 3, in some embodiments, a bidirectional charging control network, such as the bidirectional charging control network 216 depicted in FIG. 2C, may be included between the battery and the energy storage capacitor 302.

The energy storage capacitor 302 is electrically coupled with a current control switch network 304 of a resonant tank based therapeutic current control network 303 that also includes a resonant tank 305 or other resonant electrical circuit, a rectifier 306 that includes switches Q5 and Q6, a filter 307 and a polarity control element 308. A current sensor 309 and a voltage sensor 310 sense current and voltage output to a patient 317 and provide signals to be input to an ADC 314 of an FPGA or DSP 315 based controller 313. As depicted, a host 312 electronic element sends a waveform request 311, including a specified requested waveform, to be processed as input to the FGPA or DSP element 315 of the controller 313. As conceptually indicated by broken circle 316, the controller 313 may optionally be used in providing closed loop control of an electrotherapeutic waveform as it is being delivered to the patient 317. Though, in various embodiments, the controller 313 may be used in a non-closed loop control fashion, for example, where the electrotherapeutic waveform is predetermined according to a preset schedule such as in a look up table where switch control is prearranged so as to result in current delivery according to the desired electrotherapeutic waveform.

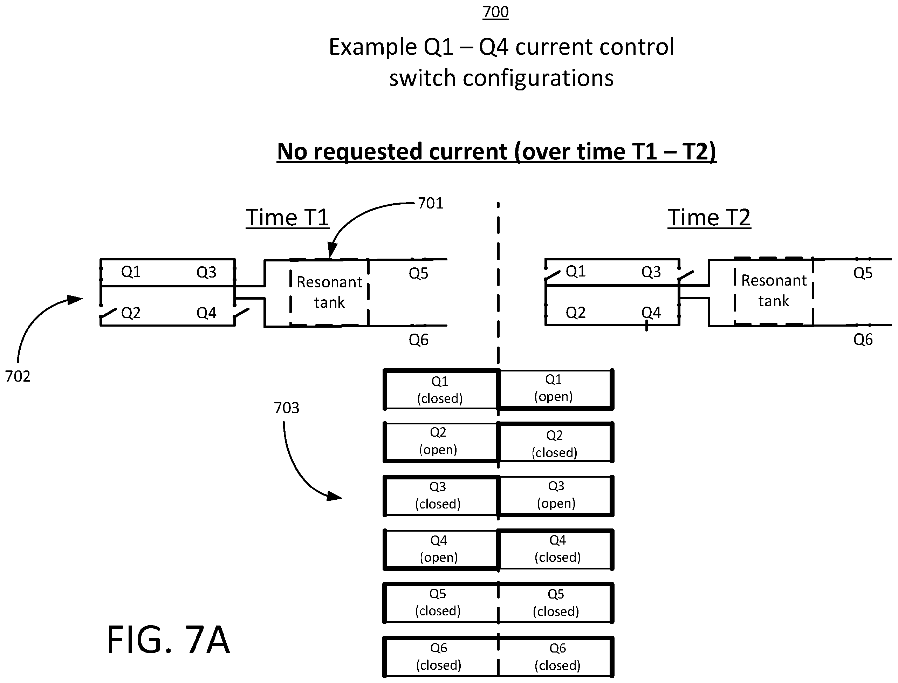

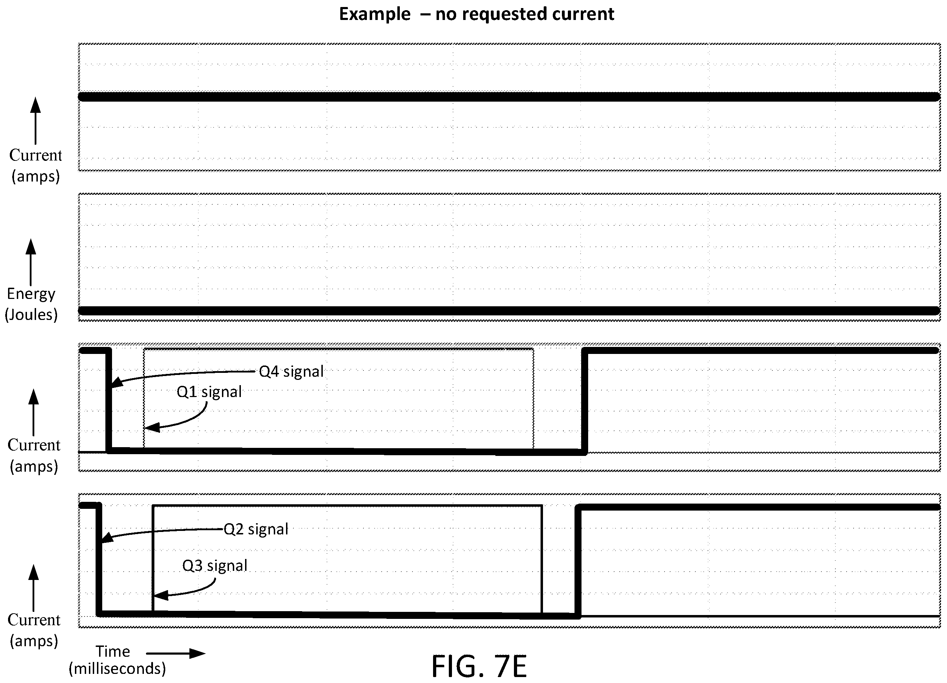

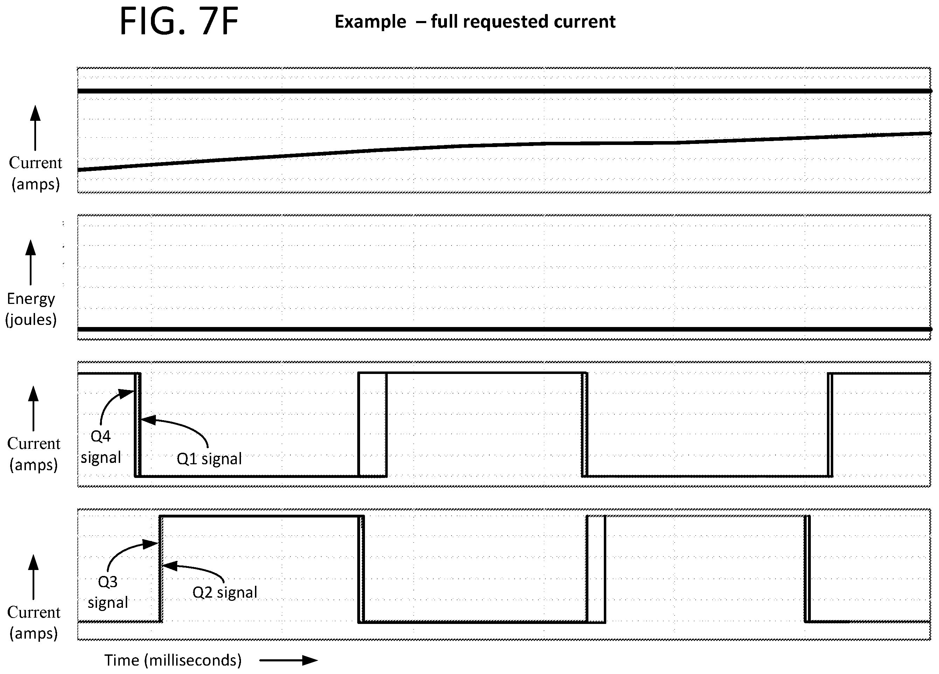

While various current control switch network configurations and components are contemplated, including, for example, H-bridge, half bridge and full bridge configurations, in the embodiment depicted in FIG. 3, the current control switch network 304 includes a full bridge configuration including four switches, specifically, Q1 - Q4. The controller 313 controls operation of the four switches Q1 - Q4 in order to control or adjust electrotherapy current being delivered to the patient 317 to match, conform with, reflect or track a waveform described or defined in the waveform request 311 from the host 312. Details of operation of the current control switch network 304 are provided with reference to FIGS. 7A-F herein.

Various components depicted in FIG. 3 are connected via nodes of the circuit. The nodal connections are described as follows. However, it is to be understood that that other embodiments may use other configurations, topologies and nodal connections or electrical couplings, as well as other components.

In the embodiment depicted in FIG. 3, the energy storage capacitor 302 and the current control switch network 304 are connected at nodes represented as N1 and N2, the current control switch network 304 and the resonant tank 305 or other resonant electrical circuit are connected at nodes represented at N3 and N4, and the resonant tank 305 or other resonant electrical circuit and the rectifier 306 are connected at nodes represented as N5 and N6.

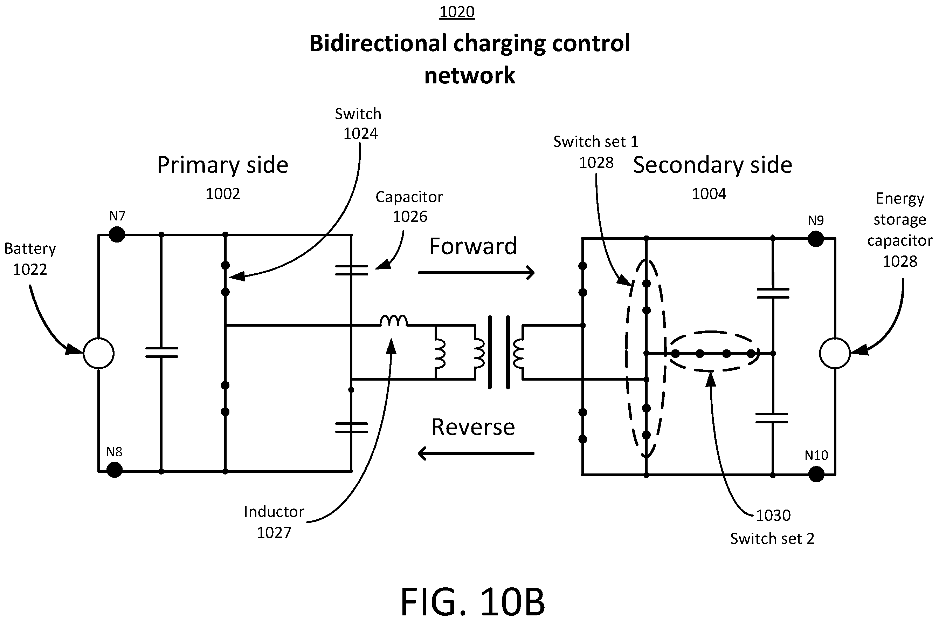

Although not depicted in FIG. 3, in some embodiments, a bidirectional charging control network may be between, and nodally connected with, a battery, such as the battery 301, and an energy storage capacitor, such as the energy storage capacitor 302. For example, as depicted in FIG. 10B, the battery 1022 is connected with the bidirectional charging control network 1020 at nodes represented as N7 and N8, and the bidirectional charging control network 1020 is connected with the energy storage capacitor 1028 at nodes represented as N9 and N10.

Turning back to FIG. 3, the rectifier 306 and the filter 307 are connected at nodes represented as N11 and N12, the filter 307 and the polarity control 308 are connected at nodes represented as N13 and N14, and the polarity control and the sensors 309, 310 are connected at nodes represented as N15 and N16.

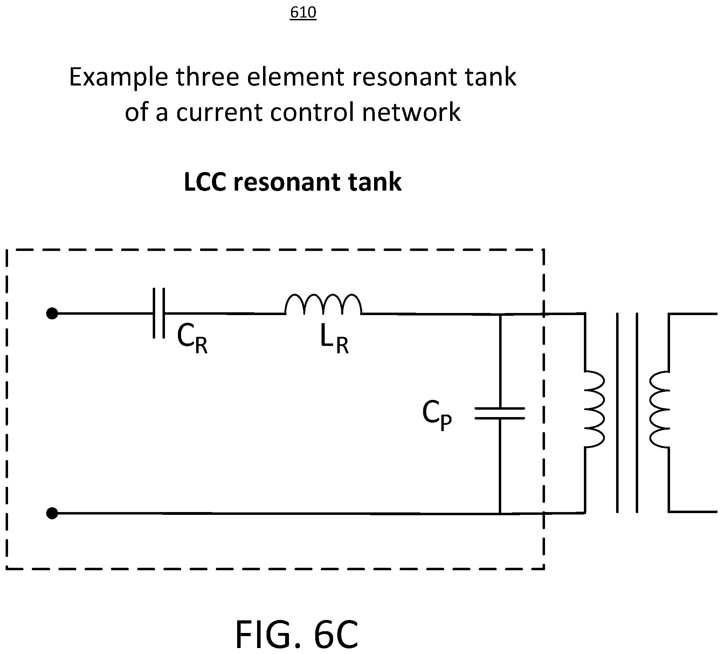

FIG. 4A is a schematic diagram 380 including an example resonant tank based therapeutic current control network 381 of a therapeutic current control system. A circuit 392 includes an energy storage capacitor 382 electrically coupled with a full bridge driver based current control switch network 383 of the therapeutic current control network 381. The therapeutic current control network 381 also includes a resonant tank 384, a synchronous or active rectifier 385, a low pass filter 386 and a polarity control element 387. As depicted, the resonant tank is an LCC resonant tank. An example of an implementation of an LCC resonant tank is provided with reference to FIG. 6C herein. The therapeutic current control network 381 is electrically coupled with a patient 390. Signals 391 corresponding with sensed current and voltage output to the patient 390 are received by a controller. While a controller is not shown in FIG. 4A, details of an example controller are described with reference to FIG. 4C herein.

As further depicted in Figs. 4A, the current control switch network 383 incudes switches QA, QB, QC and QD, and the synchronous or active rectifier 385 includes switches QE and QF. Examples of operation of the current control switch network 383 and the rectifier 385 are provided with reference to FIGS. 7A - 7F herein. It is noted that, in some embodiments, passive rectification is utilized, rather than synchronous or active rectification. In such embodiments where passive rectification is employed, the rectifier may utilize diodes, and switches QE and QF will not be included, or necessary, in the system.

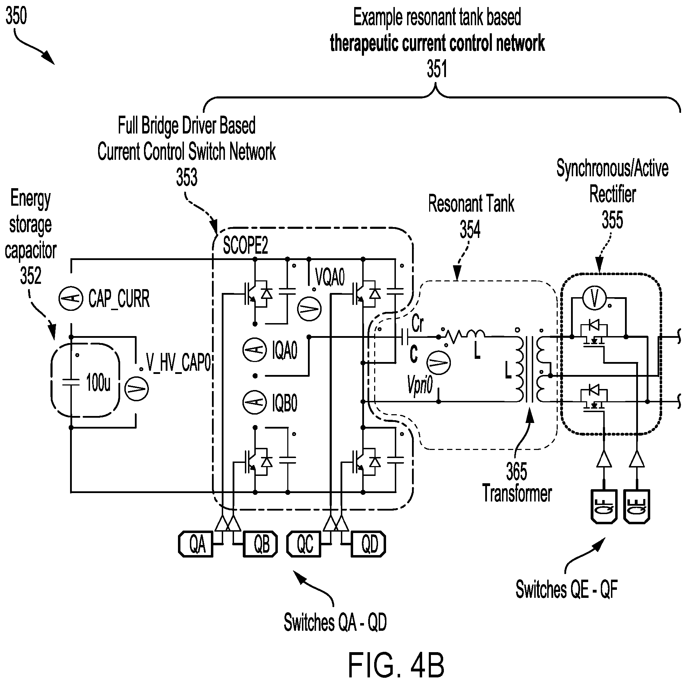

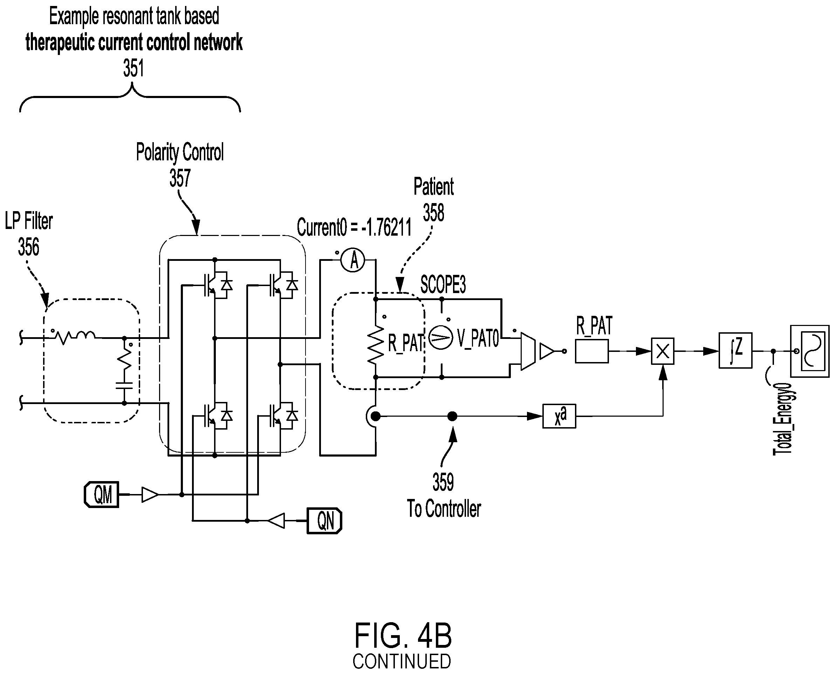

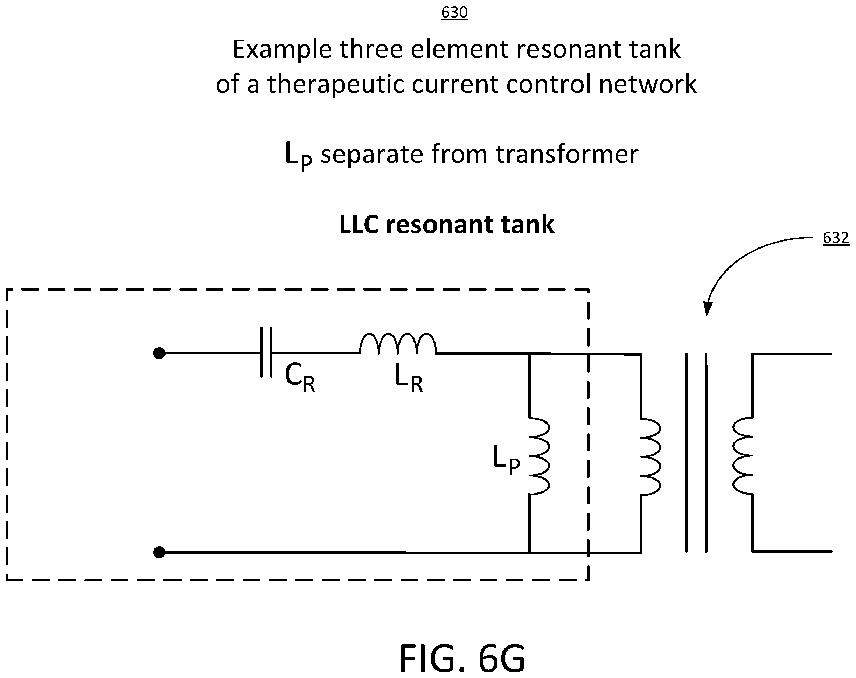

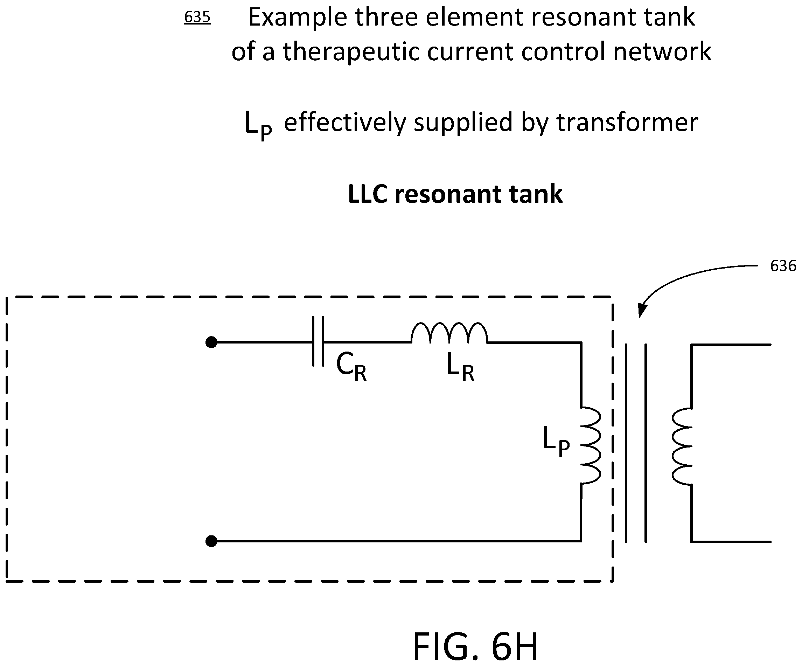

FIG. 4B is a schematic diagram 350 illustrating an additional example resonant tank based therapeutic current control network 351 of a therapeutic current control system. A circuit includes an energy storage capacitor 352 electrically coupled with a full bridge driver based current control switch network 353 of the therapeutic current control network 351. The therapeutic current control network 351 also includes a resonant tank 354, a synchronous rectifier or active rectifier 355, a low pass filter 356 and a polarity control element 357. As depicted, the resonant tank is an LLC resonant tank. The Resonant tank 354 incorporates parasitic inductance of transformer 365 as one of the resonant tank's inductive components. Further example implementations of an LLC resonant tank are provided with reference to FIGS. 6G and 6H herein. More specifically, FIG. 6H provides of an example in which the resonant tank 635 incorporates parasitic inductance of a transformer 636 as one of the resonant tank's inductive components, similar to the resonant tank 354 as depicted in FIG. 4B. However, it is noted that, in other embodiments of systems disclosed herein, an implementation of a resonant tank, or other resonant electrical circuit, can be used that does not utilize or rely on parasitic capacitance as part of the resonant tank, such as depicted, for example, in the implementation of the LLC resonant tank 630 of FIG. 6G herein. The therapeutic current control network 351 is electrically coupled with a patient 358. Signals 359 corresponding with sensed current and voltage output to the patient 358 are received by a controller.

FIG. 4C is a schematic diagram of an example controller 400 of a therapeutic current control system. It is noted that various embodiments of controllers are contemplated, which may be configured differently and operate in different ways. For example, in some embodiments, data, parameters or signaling providing a specification of a waveform may be input to a memory of the controller or elsewhere and provided to the controller, such as prior to delivery of the electrotherapeutic waveform. As another example, in other embodiments, a specification of a waveform may effectively be input or fed over time, such as substantially in real time during delivery of the waveform. Furthermore, data, parameters or signaling associated with a current provided to a patient may also be provided or facilitated in different ways. For example, in some embodiments, a controller may receive a varying signal corresponding with sensed current being provided to the patient. However, in other embodiments, sensed current may not be utilized, and, instead, one or more other varying signals may be utilized in specifying, implicitly or explicitly, a waveform being delivered to a patient, for instance, measuring the sensed voltage across the output circuit nodes to the patient (e.g. V sense 310).

Continuously or repeatedly, for example, the controller 400 may effectively compare the specified waveform with the waveform being delivered. Continuously or repeatedly, based at least in part on the comparison, the controller adjusts operation of, at least, a portion of switches of a therapeutic current control network as and when needed to adjust the waveform being delivered to the patient to match the specified waveform.

In particular, for example, in some embodiments, the controller 400 may utilize a control loop or otherwise operate to create, calculate or utilize a signal or parameter indicative of a quantitative difference between a current provided to a patient and a desired current to be provided to the patient, such as may be associated with a specified waveform. The controller may then operate to minimize this difference, thus minimizing, over time of delivery, any difference between a current provided to the patient and a current desired to be provided to the patient, such as by control, at least, of at least a portion of switches of the system. More particularly, in some embodiments, the controller may create, calculate or obtain (or repeatedly or continuously create, calculate or obtain) an e(t) (error signal as a function of time), or e(t) based, signal or parameter, where:

$$(\text{Equation 1}):\ e(t) = C_M - C_R$$

Where, in equation 1:

$C_M$ is measured current to the patient (or an electrical parameter indicative of, or associated with, current to the patient); and
$C_R$ is current associated with a specified current or waveform.

E(t) may therefore quantitatively indicate a (or any) difference, for example, at a given time/time period or for a particular individual comparison, between a measured level of current to a patient and a level of current associated with a specified current or waveform. This may include indicating whether a measured level of current to the patient is higher or lower (such as may be given by the sign of e(t) as positive or negative, for example) and the amount by which it is higher or lower (such as may be given by the magnitude or absolute value of e(t), for example). The controller 400 may then operate to minimize e(t) by appropriate control of, for example, at least the switches QA - Q D, or QA - QF, to appropriately control requested current, to appropriately affect a current level provided to the patient, which results in the current level provided to the patient matching the specified current or waveform as closely as possible.

In the embodiment depicted in FIG. 4C, the controller 400 receives input 401 including the signal relating to a specified waveform, such as a signal corresponding with current level, and one or more other signals, which may include one or more signals corresponding with current level relating to the actual electrotherapeutic waveform being delivered to the patient.

Signal output from the PID portion 406 of the controller 400 is used in generation of a phase shift PWM signal 403 that is used in assuring that a carrier signal is kept within certain limits. In some embodiments, however, a frequency changing signal could be utilized. An output signal from the controller 400 controls operation of switches of a therapeutic current control network, depicted as switches QA - QF 405, to make any necessary adjustments, such as increasing or decreasing current, to cause the waveform being delivered to the patient to match the specified waveform. As depicted, QA - QD represent switches of a current control switch network such as a full bridge driver based current control switch network 353 as depicted in FIG. 4A, and switches QE and QF represent switches of an synchronous or active rectifier, such as the synchronous or active rectifier 355 as depicted in FIG. 4A.

Clock related components 402, 403 of the controller 400, including dead time element 404, are used in controlling timing relating to switching turn-on and turn-off. Portion 402 is depicted separately but actually continuous with element 403, with feature 407 being the same feature. The clock related components 402, 403 and switch turn-on and turn-off timing are used in implementing soft-switching, substantially zero voltage switching (ZVS) or substantially zero current switching (ZCS), as described in detail with reference to FIG. 9 herein.

It is also noted that all or some of the switches 405 that are operatively controlled by the controller 400 may include switch components including wide bandgap, such as silicon carbide (SiC) or gallium nitride (GaN), further reducing any switching losses and increasing overall system efficiency.

As noted above, some embodiments utilize wide bandgap materials in circuit components, such as may include switch components. Generally, a wide bandgap material can include a semiconductor material with a relatively large band gap compared to conventional semiconductors, where a band gap is essentially an energy range in a solid in which no electronic states exist. Conventional semiconductor materials typically have a band gap in the range of 1 - 1.5 ev, whereas wide bandgap materials may have bandgaps at or above 2 ev, such as between 2 - 4 ev or between 2 - 6 ev. Generally, use of wide bandgap materials can permit electronic devices and components to operate at much higher voltages, frequencies and temperatures, as well as higher power conversion efficiencies, which allows for enhanced overall component or device performance or efficiency, as well as permits use of higher power density devices. Wide bandgap, such as may include ultra-wide bandgap, materials that may be used in various embodiments can include materials such as SiC and GaN, as well as other materials such as diamond, gallium oxide ($Ga_2O_3$), aluminum gallium nitride (AlGaN) or aluminum nitride (AlN). For example, some embodiments use the wide bandgap material SiC, which has useful properties including high thermal conductivity, high electric field breakdown strength and high maximum current density.

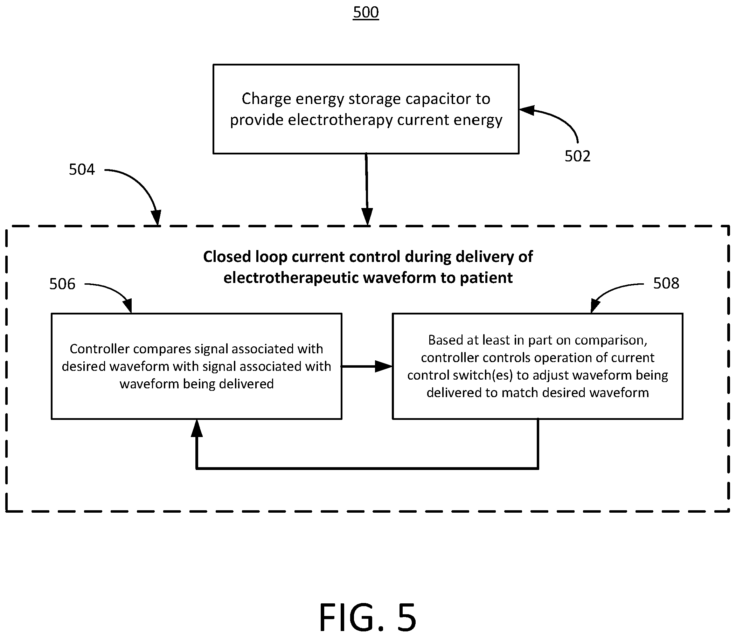

FIG. 5 is a flow diagram of an example method 500 for closed loop therapeutic current control, when optionally implemented. At step 502, an energy storage capacitor, such as a high voltage capacitor, is charged to provide energy for electrotherapy current, such as for defibrillation or pacing. Broken box 504 conceptually represents closed loop current control used during delivery of an electrotherapeutic waveform to a patient, using energy provided by the energy storage capacitor. At step 506, the controller compares one or more signals associated with a specified waveform with one or more signals associated with a waveform being delivered to the patient, such as, for example, a signal corresponding with a current output to a patient. At step 508, based at least in part on this comparison, the controller controls, at least, operation of at least one current control switch of a closed loop therapeutic current control system to adjust the electrotherapeutic waveform being delivered to the patient to match the specified waveform.

FIGS. 6A - 6V are schematic diagrams of example resonant tank configurations that can be used in embodiments of a therapeutic current control network or as a bidirectional charging control network or an element thereof. Resonant tanks may also be used elsewhere in a closed loop or non-closed loop therapeutic current control system according to embodiments described herein.

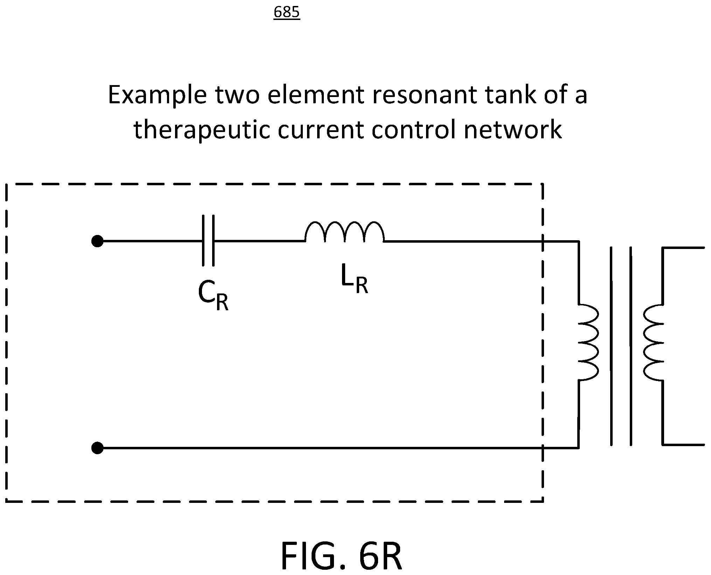

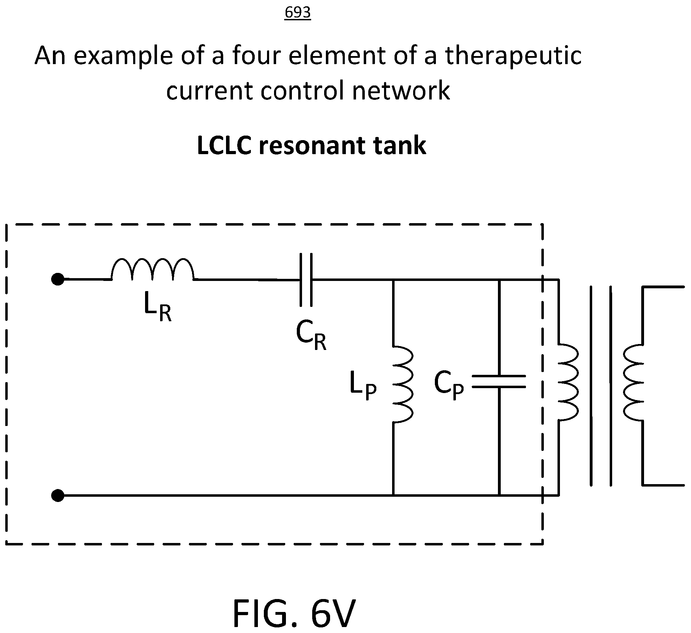

More particularly, FIGS. 6A - 6Q depict examples of three element resonant tank configurations, FIGS. 6R - 6U depict examples of two element resonant tank configurations, and FIG. 6V depicts an example of a four element resonant tank configuration, various of which can be used in various embodiments described herein. Each element is either an inductor (L) or a capacitor (C). In FIGS. 6A - 6V, components connected in series in the resonant tank are designated with the subscript "R" while components in parallel in the resonant tank are designated with the subscript "P". In general, in an electrical circuit, a resonant tank includes a configuration of one or more inductors and one or more capacitors, each of which is coupled either in series or in parallel in the resonant tank. The resonant tanks shown in FIGS. 6A-6V are not intended to be limiting, and other types of resonant tanks, or other resonant electrical circuits, may be used in various embodiments of closed therapeutic current control systems as described herein.

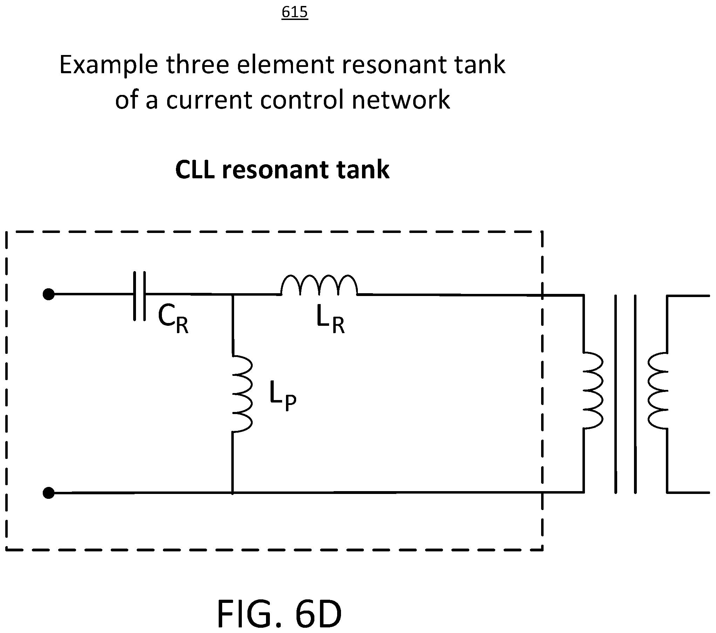

In various embodiments described herein, a variety of resonant tanks may be utilized. These include two-element, three-element and four-element resonant tanks. In various embodiments described herein, for some uses or ranges of uses, a resonant tank such as an LCC, CLL, LLC or LCLC resonant tank, for example, may be optimal or may be viewed as optimal. For example, FIG. 6C depicts an example LCC resonant tank implementation, FIG. 6D depicts an example CLL resonant tank implementation, FIGS. 6G and 6H depict example LLC resonant tank implementations, and FIG. 6V depicts an example LCLC resonant tank implementation. A particular resonant tank that is preferable or provides desired performance or efficiency in a therapeutic current control network according to an embodiment described herein may depend on a variety of factors. These factors may include, for example, physical, component related, functional or use related aspects or parameters, which may include particular practical, performance, or efficiency related requirements, priorities or preferences, for example. In some cases, an LCC resonant tank may be preferable, such as may include some applications involving a wide input or output voltage range.

In some cases, a CLL resonant tank may be preferable, such as may include some cases in which the resonant tank switching frequency is higher than the resonant frequency or frequencies.

In some cases, an LLC resonant tank may be preferable, such as may include some cases in which the LLC resonant tank may provide advantages including high efficiency, high power density, and relatively easy implementation of magnetic integration, among other potential advantages. Furthermore, in some embodiments, an LLC resonant tank based therapeutic current control network may have features or characteristics including the following. It may have a magnetics ratio, for example, in the range of between three to ten. A parallel to series inductance ratio ("K") may be selectable in such a way to have minimum circulating current while yet maintaining soft-switching. It may have an associated selectable quality factor, which may typically be between 0.1 and 1, that may directly affect the size of magnetics along with the selected resonant frequency, frequencies or frequency range. The quality factor may affect aspects such as Root-Mean-Squared (RMS) current, turn off current, range of relative frequency range, DC gain for regulation, as well as the selected K. In some embodiments, ZVS may be a function of K and quality factor. In some embodiments, selected K and quality factor may involve a balance or trade-off in certain ways. For example, a K or quality factor increase may be associated with lower primary RMS value, lower ZVS angle range, lower turn-off current, lower DC gain, higher size of magnetics and higher stress on an energy storage capacitator.

In some cases, an LCLC resonant tank may be preferable, such as may include some cases in which the LCLC resonant tank provides optimal performance by effectively homogenizing characteristics of an LLC resonant tank and an LCC resonant tank. Other resonant tanks, or other resonant electrical circuits, may be preferable in other cases.

A resonant tank may act as an electrical resonator, storing energy oscillating at the resonant tank's resonant frequency, frequencies or frequency range. In some embodiments described herein, a therapeutic current control network may include, connected in the following series: (1) an energy storage capacitor, (2) at least one current control switch, (3) a resonant tank, (4) a rectifier and (5) a filter. The topology created by elements (2)-(5) in the preceding list is commonly referred to as a resonant converter.

Parasitic capacitance and parasitic inductance refer to usually unwanted but unavoidable capacitance or inductance that exists between elements of an electronic circuit. According to some embodiments of the present disclosure, however, the parasitic inductance of a transformer positioned in the vicinity of a resonant tank may be intentionally utilized to provide capacitance or inductance for, and of, the resonant tank or other resonant electrical circuit. In such instances, the inductance provided by the transformer may effectively be part of the resonant tank, replacing the role of a resonant tank inductor.

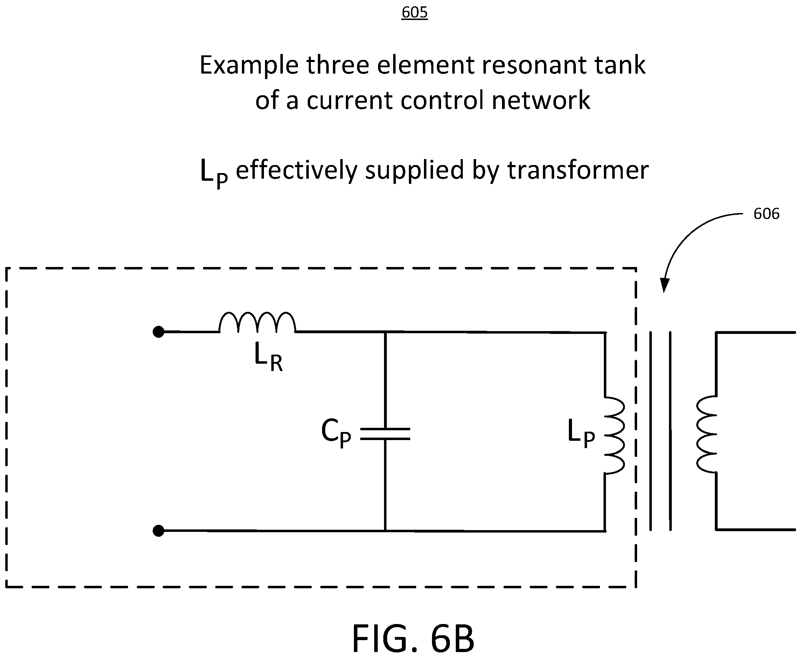

For example, FIG. 6A and 6B depict two embodiments 600, 605 of a three element resonant tank, which includes one inductor in series in the resonant tank ($L_R$) one inductor in parallel in the resonant tank ($L_P$) and one capacitor in parallel in the resonant tank ($C_P$). In the embodiment 600 depicted in FIG. 6A, the $L_P$ is separate from the depicted transformer 601. In the embodiment 605 depicted in FIG. 6B, however, the $L_P$ is effectively provided by the transformer 606. As another example, FIG. 6G and 6H depict two embodiments 630, 635 of an LLC resonant tank, which includes one inductor in series in the resonant tank ($L_R$) one inductor in parallel in the resonant tank ($L_P$) and one capacitor in series in the resonant tank ($C_R$). In the embodiment 630 depicted in FIG. 6G, the $L_P$ is separate from the depicted transformer 632. In the embodiment 635 depicted in FIG. 6H, however, the $L_P$ is effectively provided by the transformer 636. It is noted that, while not depicted in every resonant tank implementation shown in FIGS. 6A - 6V, in various embodiments, parasitic inductance can also be used to supply an $L_R$ in various resonant tanks or other resonant electrical circuits.

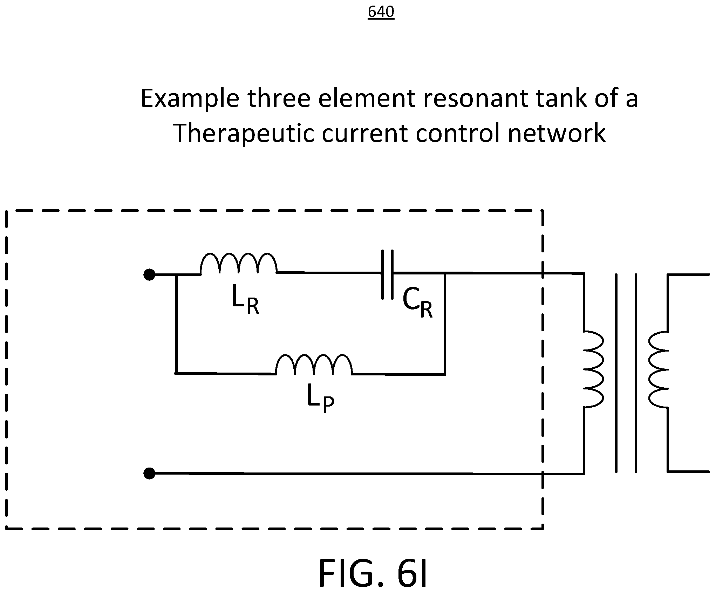

FIGS. 6C-6F, depicting embodiments, respectively, 610, 615, 620 and 625, and FIGS. 6I-6Q, depicting embodiments, respectively, 640, 645, 650, 655, 660, 665, 670, 675 and 680, provide additional examples of three element resonant tanks that may be used in some embodiments of a therapeutic current control network as described herein. FIGS. 6R - 6U, depicting embodiments 685, 690, 691 and 692, provide examples of two element resonant tanks that can be used in some embodiments of a therapeutic current control network as described herein. FIG. 6V depicts an embodiment 693 of a four element resonant tank that can be used in some embodiments of a therapeutic current control network as described herein.

FIG. 7A is a conceptual diagram 700 illustrating therapeutic current control switch configurations 702 for generating no requested current during a time period of T1 - T2. Switches Q1-Q4 of FIG. 7A may correspond, for example, with switches Q1-Q4 of the current control switch network 304 of the therapeutic current control network 303 as depicted in FIG. 3 herein. Switches Q5 and Q6 of FIG. 7A may correspond, for example, with switches Q5 and Q6 of the rectifier 306 of the therapeutic current control network 303 of FIG. 3. However, in embodiments in which passive rectification is utilized, switches Q5 - Q6 may not be present or needed. Resonant tank 701 is conceptually depicted between switches Q1-Q4 and switches Q5-Q6 and may correspond, for example, with the resonant tank 305 or other resonant electrical circuit of the therapeutic current control network 303 of FIG. 3. However, it is to be understood that the switch configurations of FIG. 7A can be used in many different embodiments of a therapeutic current control network and in many different embodiments of a closed loop or non-closed loop therapeutic current control system.

In some embodiments, a controller, such as controller 313 of FIG. 3, may control, at least, operation of switches Q1-Q6 or some subset thereof, such as Q1-Q4, to adjust an electrotherapeutic waveform being delivered to a patient to match a specified waveform. In some embodiments, each of switches Q1-Q6 may be operated to be open / turned off, so that current cannot pass through the respective switch, or closed / turned on, so that current can pass through the respective switch.

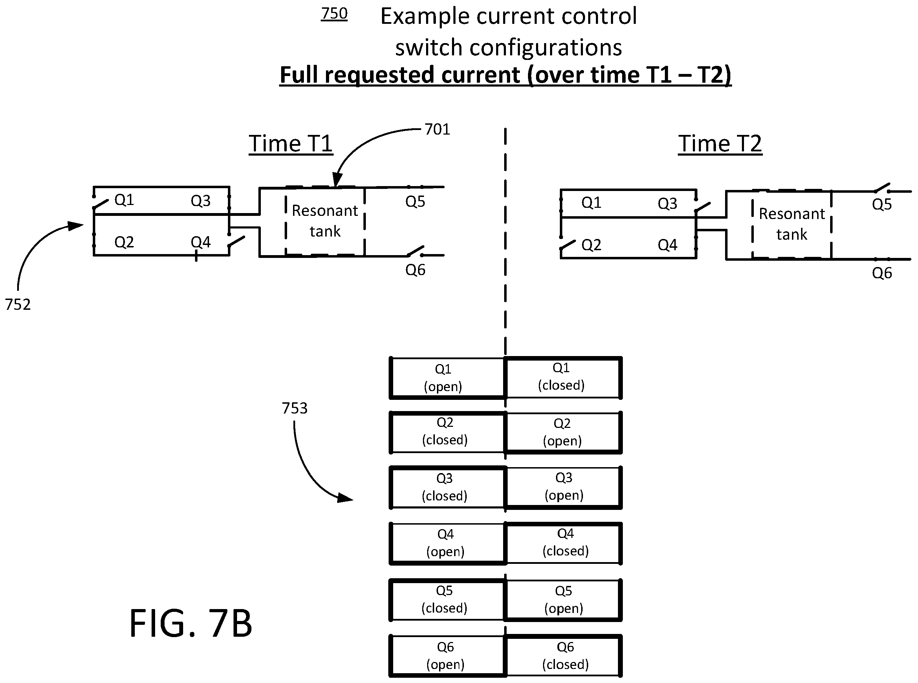

In the embodiment depicted in FIGS. 7A and 7B, by appropriate control of the status, as opened or closed, of each of switches for a time period, an amount of output current can be controlled or determined. This, in turn, can be used to adjust the manner in which the electrotherapy waveform is delivered to the patient, continuously or repeatedly during delivery.

More particularly, by controlling the status, as opened or closed, of some or all of switches Q1-Q6, no requested current can be provided, full (available) requested current can be provided, or partial requested current (a requested portion of the available current), or a particular desired amount or fraction of the available current, can be provided. It is to be understood that the switch operation depicted with regard to FIGS. 7A and 7B is only exemplary, and different operation of the switches may be possible to achieve particular adjustments. It is noted that the requested current does not necessarily equal or correspond with, or exactly equal or correspond with, the amount of current delivered to a patient at a given time. However, a controller may control requested current in order to appropriately affect current delivered to the patient, as described in detail in with reference to FIG. C, for example.

Specifically, in this particular embodiment, for no current, with respect to switches Q1-Q4, during a particular time period, the following pairs of switches are never turned on at the same time: Q1 and Q4, Q2 and Q3, Q1 and Q2, and Q3 and Q4.

FIG. 7A depicts status 702 of switches Q1-Q6 that may be used for time periods T1 and T2 to provide no current. In particular, during time period T1, switches Q2 and Q4 are open / turned off, and switches Q1, Q3, Q5 and Q6 are closed / turned on. During time period T2, switches Q1 and Q3 are open / turned off, and switches Q2, Q4, Q5 and Q6 are closed / turned on. The associated output signal 703 for each of the switches during time periods T1 and T2 is also depicted, in simplified form.

For full requested current, in this particular embodiment, the following pairs of switches are never turned on at the same time: Q1 and Q2, and Q3 and Q4. However, Q4 is turned on for all of the time that Q1 is turned on, and Q2 is turned on for all of the time that Q3 is turned on.

FIG. 7B depicts status 752 of switches Q1-Q6 that may be used for time periods T1 and T2 to provide full requested current. In particular, during time period T1, switches Q1, Q4 and Q6 are open / turned off, and switches Q2, Q3 and Q5 are closed / turned on. During time period T2, switches Q2, Q3 and Q5 are open / turned off, and switches Q1, Q4 and Q6 are closed / turned on. The associated output signal 753 for each of the switches during time periods T1 and T2 is also depicted, in simplified form.

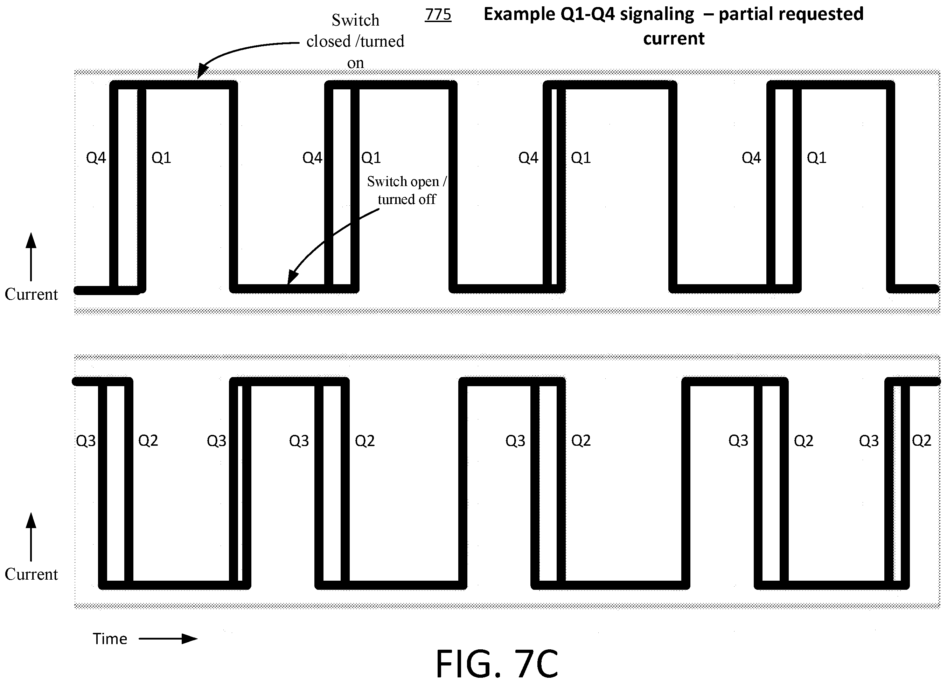

FIG. 7C provides a graphical illustration 775 of signaling associated with providing partial requested current. For partial requested current, the following pairs of switches are never turned on at the same time: Q1 and Q2, and Q3 and Q4. However, Q1 is turned on for part, but not all, of the time that Q4 is turned on, and Q3 is turned on for part, but not all, of the time that Q2 is turned on. The amount of current provided may be adjusted based at least in part on the amount of time that Q1 and Q4 are turned on together, and the amount of time that Q3 and Q2 are turned on together.

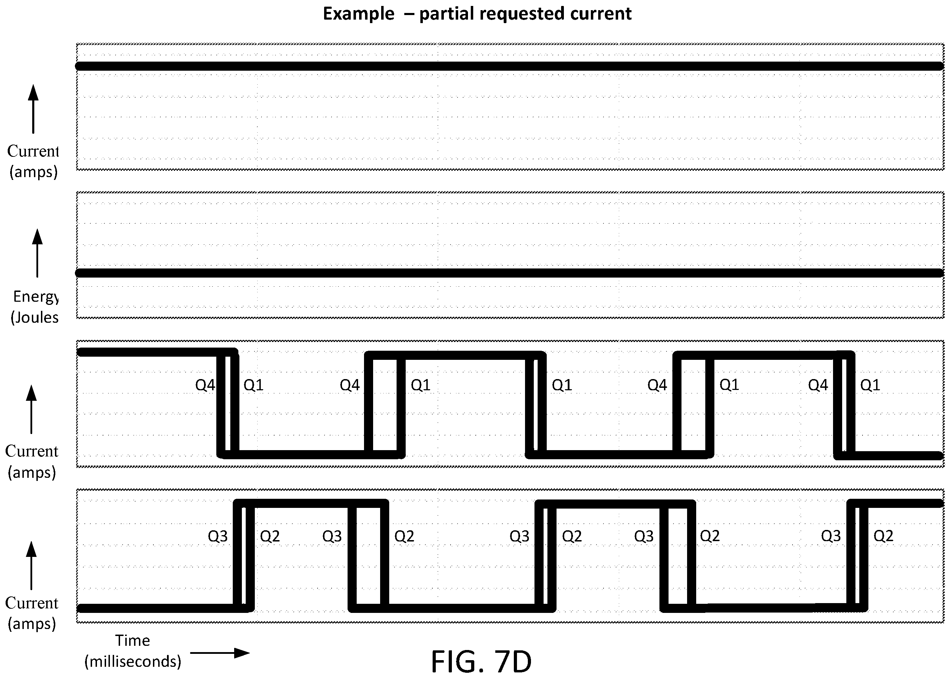

FIGS. 7D-F provide further graphical illustrations including Q1-Q4 signaling associated with generating partial requested current, no requested current, and full requested current, respectively.

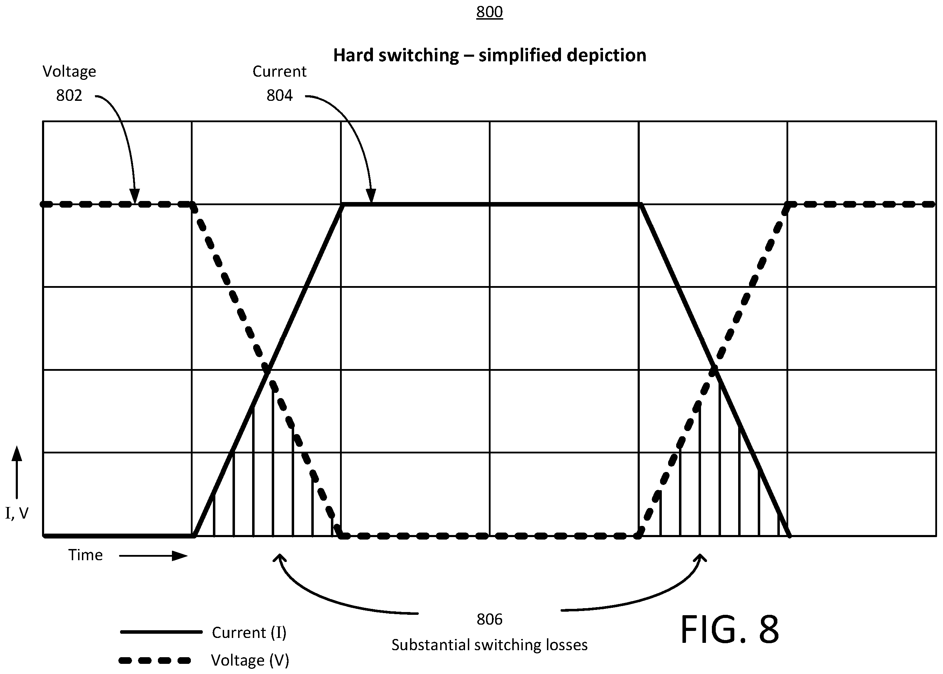

FIG. 8 is a simplified graphical illustration 800 of aspects of hard switching, while FIG. 9 is a simplified graphical illustration 900 of aspects of soft switching. Switching losses can occur at the intersection periods of non-zero portions of current and voltage waveforms; that is, during time periods when non-zero current coexists with non-zero voltage. Switching losses are associated with wasted energy and reduce the energy efficiency of the associated system. What is often referred to as "zero current switching" (ZCS) and "zero voltage switching" (ZVS) utilize soft switching. For example, substantially ZCS can occur when transistor turn-off transition occurs at substantially zero current, and substantially ZVS can occur when transistor turn-on transition occurs at zero voltage. Some embodiments described herein utilize soft switching, substantially ZCS or substantially ZVS to increase system efficiency and reduce wasted energy.

FIG. 8 provides a simplified graphical illustration 800 of hard switching. The timing of the current waveform 804 relative to the timing of the voltage waveform 802 is such that substantial waveform intersection periods occur, leading to substantial switching losses, as conceptually represented by the shaded areas 806.

By contrast, FIG. 9 provides a simplified graphical illustration 900 of soft switching, which is implemented in certain embodiments of the present disclosure. The timing of the current waveform 904 relative to the timing of the voltage waveform 902 is determined, controlled or arranged such that minimal to substantially zero intersection periods occur, as conceptually represented by the intersection areas 906 occurring at or near substantially zero voltage and substantially zero current. Soft switching can include, for example, staggering timing of initiation of voltage switching relative to timing of initiation of associated current switching so as to facilitate minimization of switching losses.

FIG. 10A depicts two flow diagrams illustrating different capacitor charging related methods 1000, 1014, such as may be used to charge an energy storage capacitor that may supply current for generation of an electrotherapeutic waveform to be delivered to a patient. In method 1000, a battery 1002 is used to charge an energy storage capacitor 1004. Excess or unused energy stored at the energy storage capacitor 1004 may be disposed of or dumped at one or more resistors 1006. Such excess energy can include, for example, excess energy following generation and delivery of an electrotherapeutic waveform to a patient. It can also include, for example, unused energy such as in a case in which the energy storage capacitor is charged for generation and delivery of an electrotherapeutic waveform to a patient but subsequently it is decided or determined not to go forward with the generation and delivery of the electrotherapeutic waveform to the patient. Such a decision or determination could be made, for instance, due to certain changed circumstances or changes in the patient's condition that would cause generation and delivery of the electrotherapeutic waveform to the patient to be currently not indicated, suboptimal, inadvisable or too risky. By contrast, in method 1014, a bidirectional charging control network 1010 facilitates not only charging of the energy storage capacitor 1012 by the battery 1008, not also enables return of excess or unused energy from the energy storage capacitor 1012 to the battery 1008 for storage at, and to charge, the battery 1008. The bidirectional charging control network 1010 may be implemented, for example, as the bidirectional charging control network 216 as depicted in FIG. 2C herein. Some embodiments of therapeutic current control systems utilize method 1014, increasing system efficiency, reducing heat production, and increasing battery life or the number of battery uses available before recharge of the battery 1008.

FIGS. 10B - 10D illustrate an example of a bidirectional charging control network 1020 that can be use in some embodiments described herein. The example described with reference to in FIGS. 10B - 10D could be used, for example, as the bidirectional charging control network 1010 as shown in FIG. 10A herein, or as (or as part of) the bidirectional charging control network 216 as shown in FIG. 2C herein.

It is noted that many types of circuit configurations and topologies can be used as, or as part of, a bidirectional charging control network, according to various embodiments. The example shown in FIGS. 10B-D includes a resonant tank, such as one of the resonant tanks shown in FIGS. 6A -6V herein. Example resonant tanks that could be used in embodiments of a bidirectional charging control network may include, for example, an LCC, CLL, LLC or LCLC resonant tank. The examples shown in FIGS. 10B - 10D may use DSP or FPGA components, but other components may be utilized in various embodiments. In some embodiments, a bidirectional charging control network is used that does not include a resonant tank but may use, for example, a flyback converter or other power converter, but a bidirectional charging control network that uses a resonant tank may be more or most efficient.

FIG. 10B is a schematic diagram of an example bidirectional charging control network 1020, including a primary side 1002 and a secondary side 1004, each of which includes a network including switches, such as switch 1024, inductors, such as inductor 1027, and capacitors, such as capacitor 1026. The primary side includes a battery 1022 and the secondary side includes an energy storage capacitor 1028.

In operation, a mode selection signal can be used in selecting the direction of energy flow, including forward so that energy flows from the battery 1022 to the energy storage capacitor 1028 to be stored at the energy storage capacitor 1028, or reverse so that energy flows from the energy storage capacitor 1028 to the battery 1022 to be stored at the battery 1022. As described with reference to FIG. 2C, for example, in some embodiments, the forward flow direction may be used to charge the energy storage capacitor 1028 using the battery 1022, such as prior to use in providing current for generation and delivery of an electrotherapeutic waveform to a patient. The reverse flow direction may be used to return excess or unused energy from the energy storage capacitor 1028 to the battery 1022 to be stored at the battery, such as following generation and delivery of an electrotherapeutic waveform to a patient.

Engagement of various combinations of elements of the bidirectional charging control network 1020 over time can be used in various ways to select, adjust, manipulate or optimize its operation for a particular use. For instance, selection of the status of each switch as turned on / closed or turned off / open can lead to engagement of one or more particular inductors and one or more capacitors in series or parallel in the resonant tank, thus effectively creating a resonant tank accordingly.

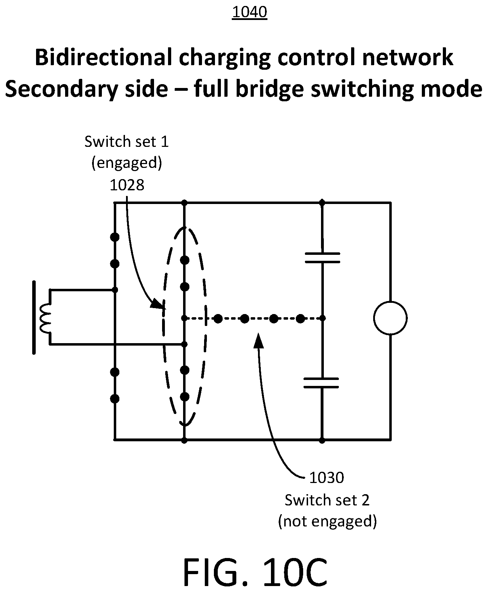

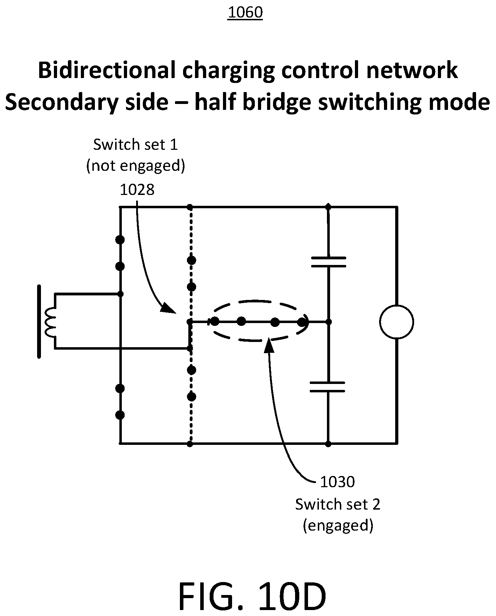

As illustrated in FIGS. 10C and 10D, the bidirectional charging control network 1020 can be operated in a full bridge mode or a half bridge mode, respectively. In FIG. 10B, switch set 1 1028 and switch set 2 1030 are shown. As shown in FIG. 10C, in full bridge mode 1040, switch set 1 1028 is engaged, while switch set 2 1030 is not engaged. Conversely, as shown in FIG. 10D, in half bridge mode, switch set 1 1028 is not engaged, while switch set 2 1030 is engaged. Operation in full bridge mode or half bridge mode, and time periods or ratios of operation in each mode, can be used in manipulating operating parameters, such as voltage provided, and in controlling or regulating energy transfer between the primary side 1002 and the secondary side 1004, as depicted in FIG. 10B, and thus between the battery 1022 and the energy storage capacitor 1028.

FIGS. 11A-K provide graphical illustrations of the ability of a system according to embodiments described herein in generating waveforms to match specified waveforms at particular energy levels. Some embodiments described herein provide an ability to provide a wide variety of arbitrary waveforms with great accuracy and precision, and over a wide dynamic range, including for a wide range of energy outputs.

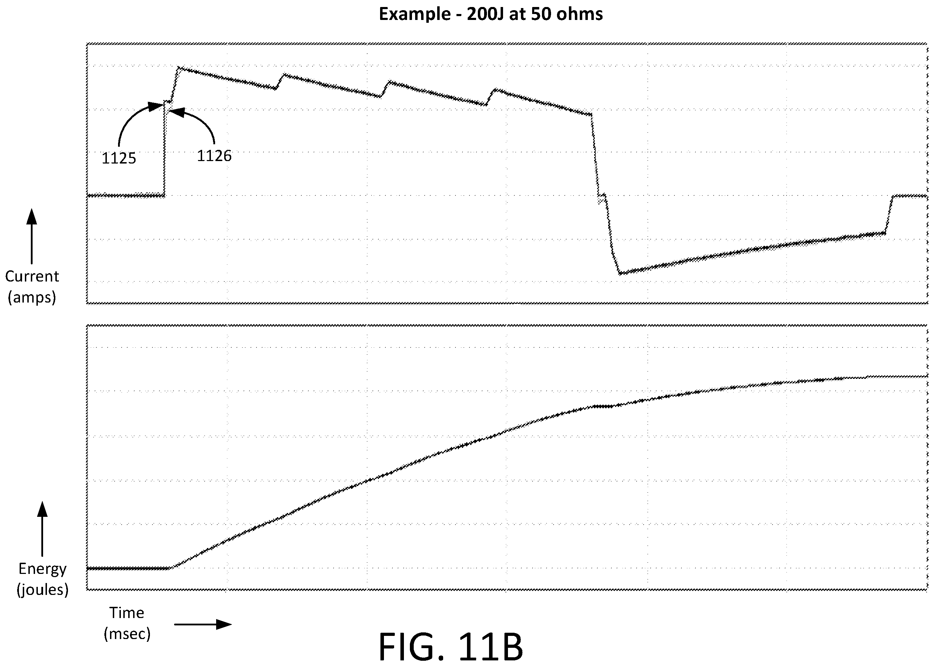

FIGS. 11A and 11B provide simplified and unsimplified depictions, respectively, of matching of a waveform at an energy of 200 joules and a resistance of 50 ohms. In FIG. 11A, curve 1101 represents the specified waveform, and curve 1102 represents the generated waveform. In FIG 11B, curve 1125 represents the specified waveform, and curve 1126 represents the generated waveform. Specifically, matching a biphasic rectilinear waveform is shown.

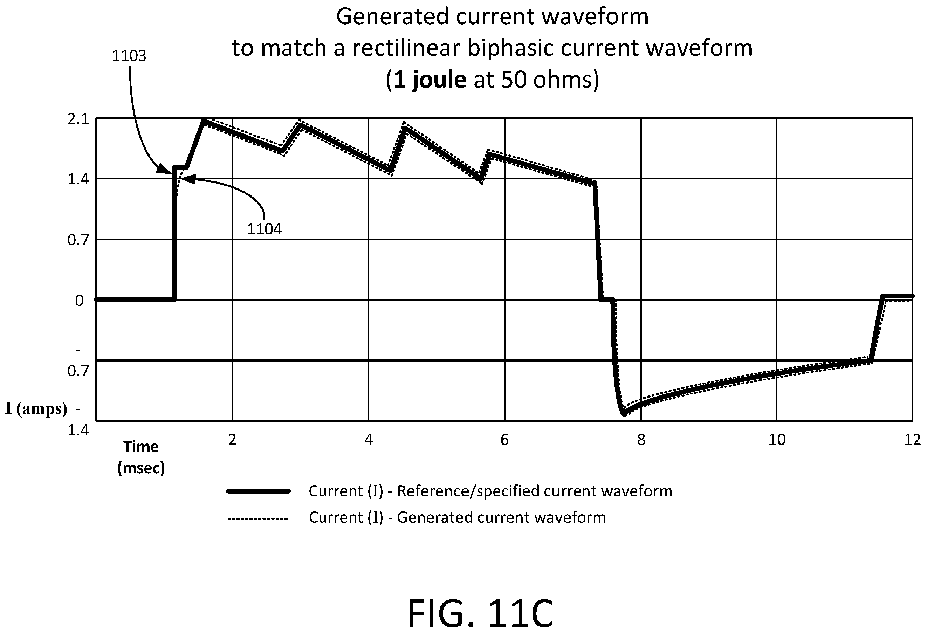

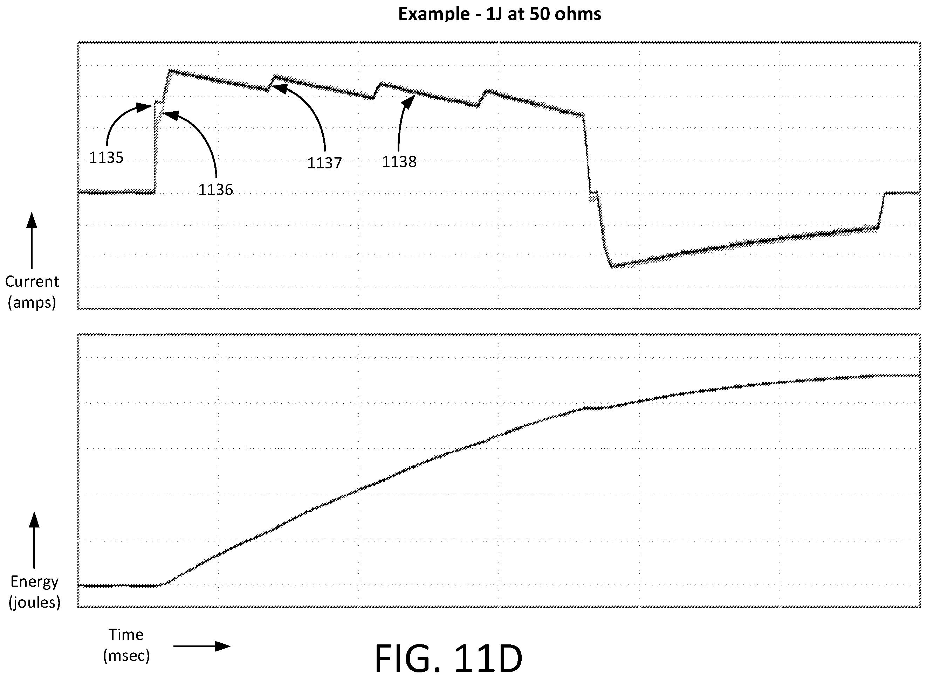

FIGS. 11C and 11D provide simplified and unsimplified depictions, respectively, of matching of a waveform at an energy of a much different energy level than that of FIG. 11B - specifically, 1 joule - and a resistance of 50 ohms. In FIG. 11C, curve 1103 represents specified waveform, and curve 1104 represents the generated waveform. In FIG 11D, curve 1135 represents the specified waveform, and curve 1136 represents the generated waveform. Specifically, matching of a biphasic rectilinear waveform is shown. It is notable that the upper sections of the reference waveform curve 1103 includes ripples, or relatively sharp jumps, such as generally depicted by example feature 1137 may be followed by declines that are more gradual, such as generally depicted by example feature 1138.

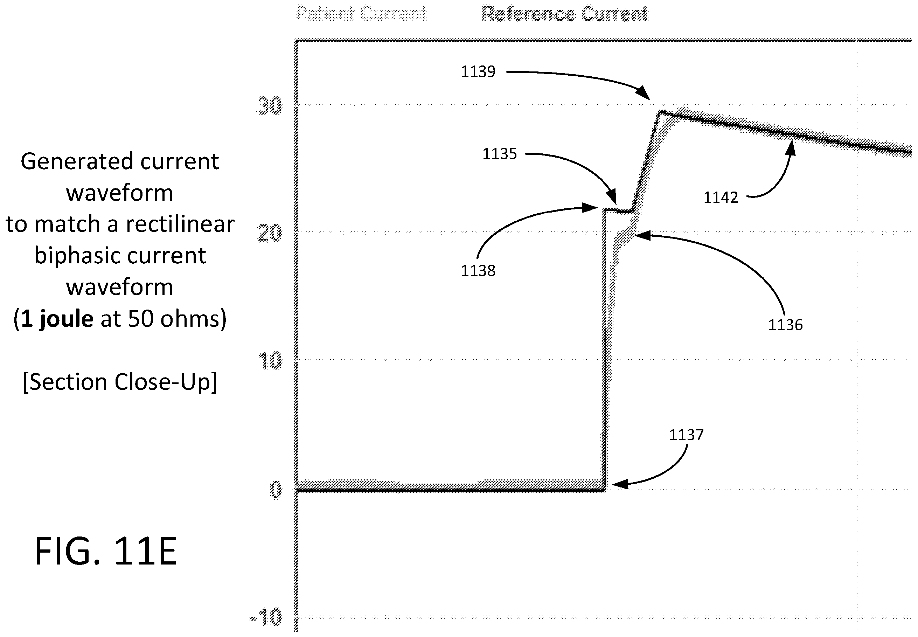

FIG. 11E provides a close-up view of a portion of the depicted waveforms 1135, 1136 of FIG. 11D. In particular, the generated waveform 1136 is closely matched with the reference waveform, including at sharp variations or ripples such as generally depicted by example features 1137, 1138 and 1139 as well as smoother portions such as generally depicted by example feature 1142.

Taken together, FIGS. 11A-E provide an exemplary illustration of the wide range of energy levels for which embodiments described herein can accurately generate a waveform to match specified waveforms of, or associated with any of, various forms, shapes, or electrical parameters. In particular, FIGS. 11A-E show matching of different biphasic rectilinear waveforms at much different energy levels (200 joules and 1 joule), with substantial accuracy. It is noted that the degree of accuracy depicted in any of FIGS. 11A - 11L is not intended to represent a limit of accuracy of embodiments disclosed herein, but provides examples of accuracy and range of some embodiments of the present disclosure. Any of a great variety of waveforms can be matched using embodiments disclosed herein, and, in at least that regard, embodiments disclosed herein provide matching of specified waveforms with substantial control and accuracy.

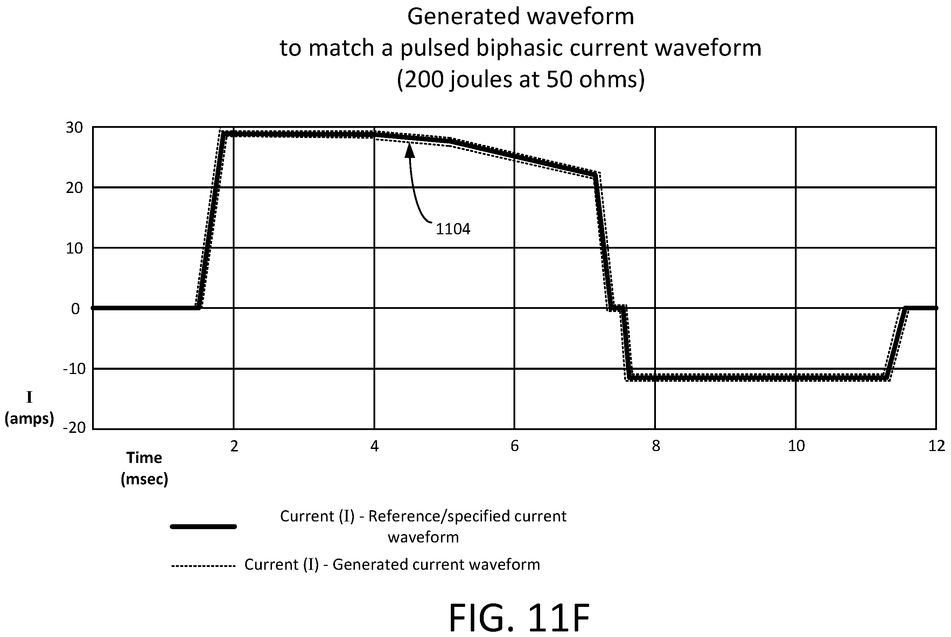

FIG. 11F shows a simplified depiction of matching of a pulsed biphasic waveform at 200 joules and 50 ohms, with the generated waveform represented as broken curve 1104. Unlike the waveforms of FIGS. 11A - 11C, the upper section of the waveform depicted in FIG. 11F is generally smooth, with a slightly downward slope midway through the positive phase of the biphasic waveform.

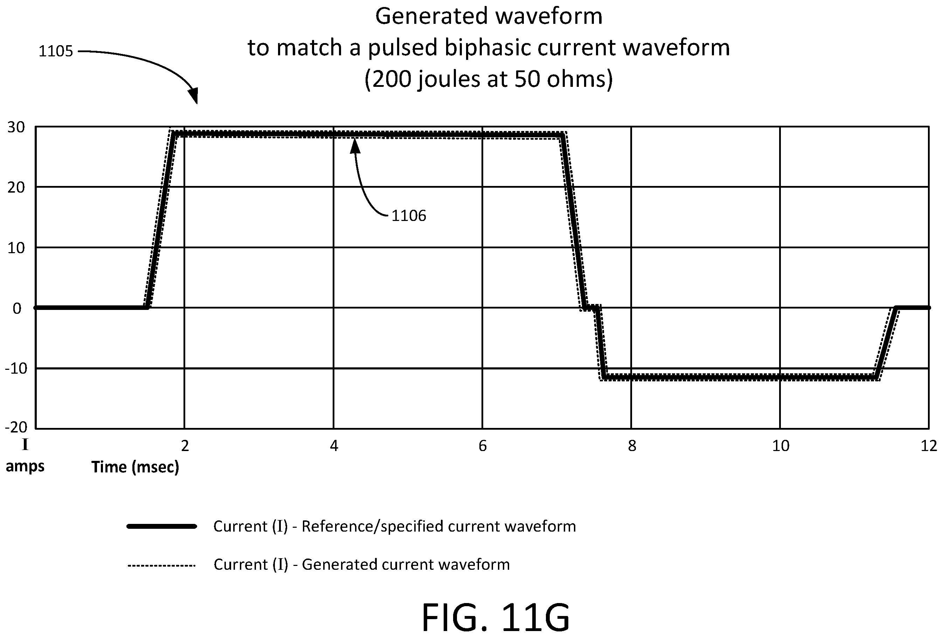

FIG. 11G shows a simplified depiction of matching of another pulsed biphasic waveform at 200 joules and 50 ohms, with the generated waveform represented as curve 1105. The waveform 200 depicted in FIG. 11G is similar to the waveform depicted in FIGS. 11A - 11C, but has a straight line uppermost portion 1106. In some cases, it may be desirable, optimal or least risky to deliver a constant amount of current over at least a portion of a time of delivery of a waveform to a patient. For example, this may allow delivery of a therapeutically optimal level of current, as opposed to a lower level of current that may be less therapeutically effective, over a period of time without substantial spikes of higher current that may present an unacceptable level of risk to the patient, and yet without dips of substantially lower current that may be suboptimal or less effective from a therapeutic standpoint. As such, in some cases, it may be desirable or critical to deliver not substantially less than an optimal level of current to a patient during a period of delivery, but also not substantially more. Some embodiments of the present disclosure provide closed loop or non-closed loop systems for generation and delivery of, if desired, a substantially constant level of current to a patient, as illustrated by portion 1106 of the waveform 1105 depicted in FIG. 11G, for example.

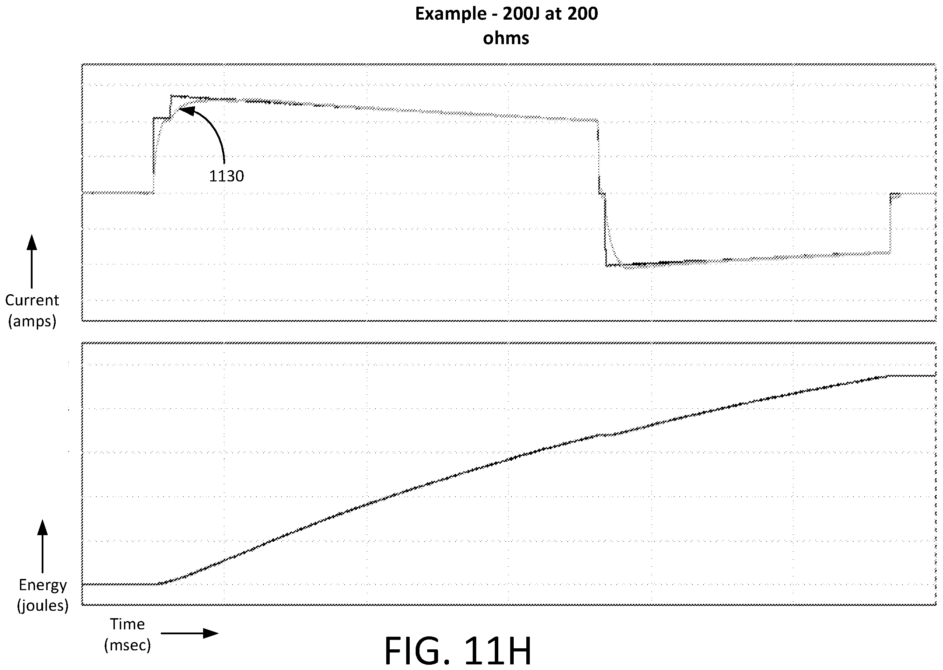

FIGS. 11H-K provide additional examples of embodiments of matching a generated waveform with any of a variety of specified waveforms, at various energy and resistance levels. Additionally, in each of FIGS. 11H-K, an associated total energy transferred or delivered from the waveform over time is shown in the bottom graph. The total energy increases over time as current is delivered to the patient over time.

FIG. 12 is a block diagram of an example quasi-resonant therapeutic current control system 1200, according to some embodiments, that can be used, for example, in defibrillators, pacemakers or other electrotherapy devices, which may include portable devices. The system 1200 includes a power supply 1204 electrically coupled with a therapeutic current control network 1202. The power supply 1204 may be or include an energy storage device such as a high voltage capacitor, which may be charged, such as by a battery or other power source, prior to delivery of electrotherapy current to a patient. Various types of capacitors can be used in various embodiments, as described, for example, with reference to FIG. 2A herein.

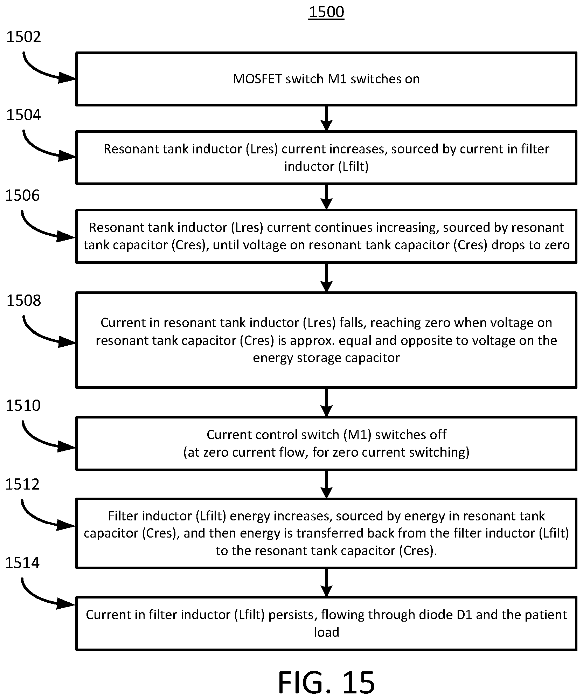

The therapeutic current control network 1202 may include at least one polarity control switch 1206, a filter 1210, a resonant tank 1212 and at least one current control switch 1214. Various components of an example corresponding therapeutic current control network are described in detail with reference to FIG. 15 herein.

Generally, by way of example and without limitation, components of the therapeutic current control network 1202 may be configured or function as follows. The at least one polarity control switch 1206 may include, for example, a full bridge used in control of direction of current flow, including through the patient load 1208. It is noted that, in the embodiment depicted, the patient load 1208 is not part of the therapeutic current control network 1202, but is electrically coupled therewith. The filter 1210 may be configured for use including electrical signal filtering. The resonant tank 1212 may include one or more inductors and one or more capacitors that act as an electrical resonator, storing energy oscillating at the resonant tank's resonant frequency or frequency range. Non-limiting examples of resonant tank configurations that may be used in a therapeutic current control network are provided in FIGS. 6A - 6V. As described with regard to FIG. 2B, for example, in various embodiments, a resonant tank may be beneficial to effectively store energy in a manner that minimizes or otherwise reduces the overall loss or dissipation of energy that may occur in other energy storage configurations. This may be advantageous in the context of defibrillation where a substantial amount of energy is stored and released over a short period of time. As described below, the controller 1220 may control operation of the at least one current control switch 1214, and may control other components of the therapeutic current control network 1202, or other components of the system 1200, to control or adjust a level of current being delivered to the patient load 1208 / patient, such as to correspond with a specified waveform 1218.

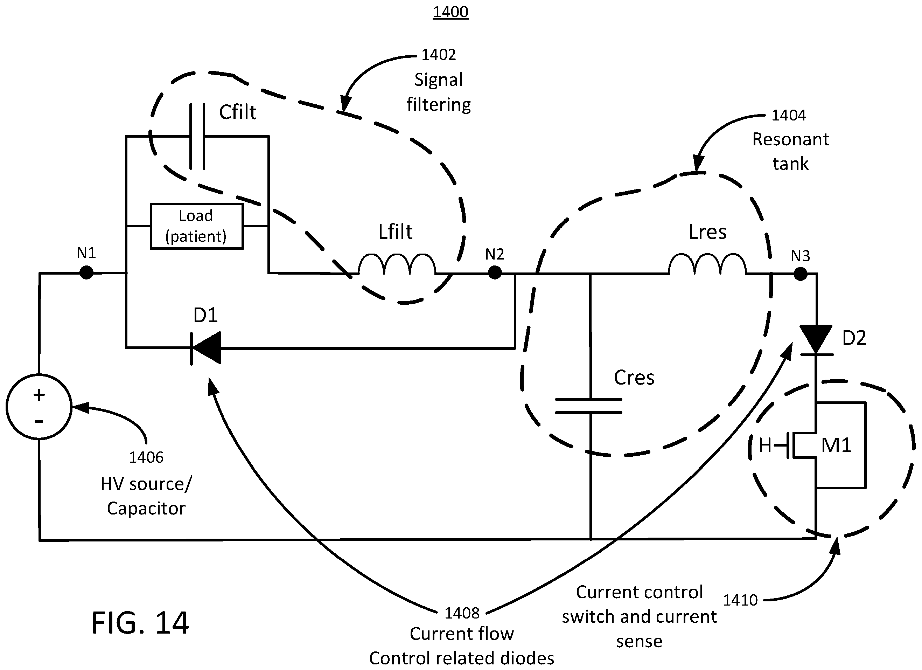

The system 1200 may also include an electrical parameter sensor 1216 configured to sense at least one electrical parameter from which a current flow to the patient / patient load 1208 can be determined or estimated. In some embodiments, the sensor 1216 may sense an electrical parameter to or through the patient. In some embodiments, however, as described with reference to FIGS. 14-15, for example, the sensor 1216 may not directly sense an electrical parameter at or through the patient, but may sense an electrical parameter elsewhere, such as at another node within a circuit of the therapeutic current control network 1202, and generate signal(s) 1224 based at least in part on which current to the patient can be determined or estimated. For example, in some embodiments, the average current through the at least one current control switch 1214 may be measured and used, potentially among other data or measured parameters, in determining or estimating the current through the patient.

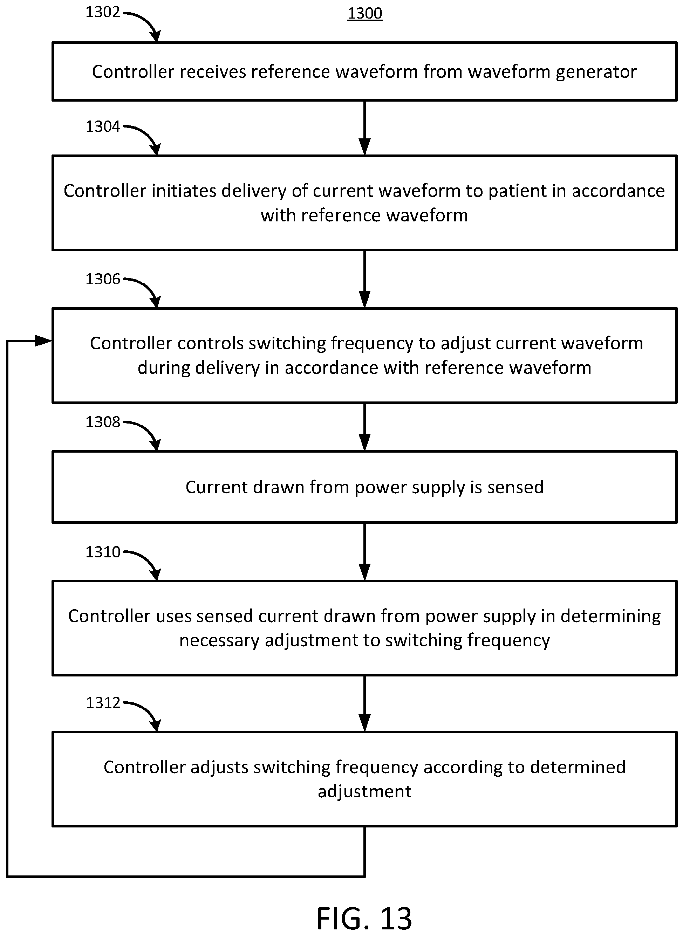

FIG. 13 is a flow diagram of a method 1300 that may be implemented using a quasi-resonant therapeutic current control system, such as the system 1200 depicted in FIG. 12. At step 1302, a controller, such as controller 1220 as depicted in FIG. 12, receives information specifying a reference waveform, such as by way of input from a waveform generator, although in some embodiments the information may be pre-stored in a memory of the controller, for example.

At step 1304, the controller initiates delivery of a current waveform to a patient in accordance with the reference waveform, such as to match, substantially match, approximately match, or correspond with the reference waveform.

At step 1306, the controller controls a switching frequency of at least one current control switch, such as the at least one current control switch 1214 as depicted in FIG. 12, to adjust the current waveform during delivery in accordance with the reference waveform.

Steps 1308 - 1312 may be used in various closed loop current control embodiments. In various non-closed loop current control embodiments, however, some or all of steps 1308 - 1312 may not be included or may be modified. For example, in some embodiments with non-closed loop current control, step 1306 may be repeated or continuously repeated throughout deliver of the current waveform to the patient. However, in some embodiments using non-closed loop current control, the adjustments may be determined and implemented based on matching the current waveform to the reference waveform, without determining what adjustments need to be made during delivery of the waveform, for example, in an open loop fashion. Current drawn from the power supply may or may not be sensed.

At step 1308, current drawn from a power supply, such as an energy storage capacitor, is sensed, such as using a sensor such as the sensor 1214 as depicted in FIG. 12.

At step 1310, after receiving a signal corresponding to the sensed current drawn from the power supply, the controller, potentially using one or more algorithms, uses the sensed current, and potentially other information or measured parameters, in determining a necessary, appropriate or optimal adjustment to the switching frequency of the at least one current control switch, such as to adjust the current waveform being delivered to the patient to correspond, or to better, more accurately or more precisely correspond, to the reference waveform.

At step 1312, the controller adjusts the switching frequency of the at least one current control switch according to the determined adjustment. It is noted that, in some embodiments, steps 1310 and 1312 can be implemented as a single step or action.

Following step 1312, the method 1300 returns to step 1306, and steps 1306-1312 are repeated until delivery of the current waveform is complete, or as necessary for complete delivery.

FIG. 14 is an exemplary simplified schematic diagram of a quasi-resonant therapeutic current control system 1400. As depicted, the system 1400 is implemented as a circuit including (potentially among other components not depicted): a high voltage source, such as a high voltage capacitor 1406, or energy storage capacitor; a filter 1402 including a capacitor (Cfilt) and an inductor (Lfilt); a resonant tank 1404 including a capacitor (Cres) and an inductor (Lres); diodes D1 and D2, which may be used in connection with current flow control; and a current sensing and current control switch portion 1410, including a current control switch (M1) that may be implemented, for example, using a high voltage MOSFET switch.

In the configuration depicted in the circuit, the energy storage capacitor 1406 and a first portion of the circuit including a patient load and are connected via a first node (N1). The first portion of the circuit and a second portion of the circuit including the resonant tank 1404 are connected via a second node (N2). The second portion of the circuit and a third portion of the circuit including the current control switch (M1) are connected via a third node. The current control switch (M1) is connected to ground, where ground is represented by the return path to the energy storage capacitor 1406.

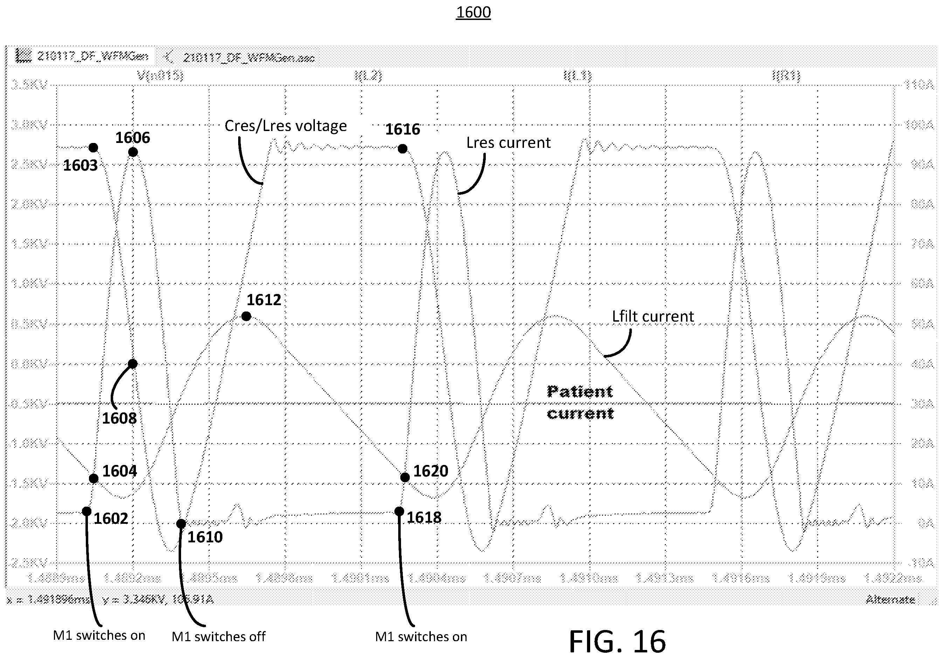

FIG. 15 a flow diagram of a method 1500 that can implemented using a quasi-resonant therapeutic current control system such as system 1400 of FIG. 14 and that can be associated with the timing diagram of FIG. 16. In some embodiments, the steps of the method 1500 may generally or approximately represent some aspects of operation of the system 1400 of FIG. 14. The electrical components referred to in the method 1500 are those depicted in the system 1400 of FIG. 14.

At step 1502, during operation, such as during delivery of an electrotherapeutic waveform to a patient, the current control switch (M1) as depicted in FIG. 14, switches on.

At step 1504, the current through the resonant tank inductor (Lres) increases, initially sourced from the current in the filter inductor (Lfilt).

At step 1506, once the current in the resonant tank inductor (Lres) exceeds the current in the filter inductor (Lfilt), the current in the resonant tank inductor (Lres) continues increasing, sourced by the resonant tank capacitor (Cres) until the voltage on the resonant tank capacitor (Cres) falls to zero. At this point, the energy in the resonant tank (Cres) has been transferred in part to the resonant tank inductor (Lres), and in part to the filter inductor (Lfilt).

At step 1508, the current in the resonant tank inductor (Lres) falls, reaching zero when voltage on the resonant tank capacitor (Cres) is approximately equal and opposite to the voltage on an energy storage capacitor of the system, such as, for example, the energy storage capacitor 1406 as depicted in FIG. 14. At this point, energy in the resonant tank inductor (Lres) has transferred in part back to the resonant tank capacitor (Cres) and in part to the filter inductor (Lfilt).

At step 1510, the current control switch (M1) switches off at zero or substantially zero current flow through the current control switch (M1), allowing zero current switching (ZCS) or substantially ZCS. Diode D2 prevents current flow in the body diode of the current control switch (M1).

At step 1512, the energy of the filter inductor (Lfilt) increases, sourced initially by energy in the resonant tank capacitor (Cres), and then energy is transferred back from the filter inductor (Lfilt) to the resonant tank capacitor (Cres).

At step 1514, once the energy transfer from the filter inductor (Lfilt) to the resonant tank capacitor (Cres) is complete, the current in the filter inductor (Lfilt) persists, flowing through D1 and the patient load.

FIG. 16 is an exemplary timing diagram 1600 relating to operation of a quasi-resonant therapeutic current control system, in accordance with embodiments of the present disclosure, which is associated with the method 1500 of FIG. 15.

On the plot showing current in the resonant tank inductor (Lres), point 1602 corresponds to the time at which the current control switch (M1) switches on, which corresponds to step 1502 of FIG. 15. On the plot showing voltage on the resonant tank inductor, point 1603 also corresponds to the time when the current control switch (M1) switches on.

After point 1602, it can be seen that the current in the resonant tank inductor (Lres) begins increasing, sourced by the current in the filter inductor (Lfilt), which can be seen simultaneously decreasing, which corresponds to step 1504 of FIG. 15. Also, after point 1603, it can be seen that the voltage on node N2 as depicted in FIG. 14 (voltage on resonant tank capacitor (Cres)/voltage on resonant tank inductor (Lres)) begins decreasing.

Point 1604 corresponds to the time at which the current in the filter inductor (Lfilt) reverses direction and begins to increase. After this point, the current in the resonant tank inductor (Lres) continues to increase, sourced by the resonant tank capacitor (Cres), until it reaches a peak, as represented by point 1606, when the voltage on the resonant tank capacitor (Cres) drops to zero, as represented by point 1608, corresponding with step 1506 of FIG. 15.

After point 1606, it can be seen that the current in the resonant tank inductor (Lres) begins decreasing, corresponding with step 1508 of FIG. 15.

Point 1610 represents the point at which the current in the resonant tank inductor (Lres), and the current through the current control switch (M1) falls to zero, at which time the current control switch (M1) switches off, providing for zero current switching (ZCS). This corresponds with step 1510 of FIG. 15.

Following the current control switch (M1) switching off, the current in the filter inductor (Lfilt) can be seen continuing to increase, reaching a peak at point 1612, with this period corresponding with step 1512 of FIG. 15.

After point 1612, the current in the filter inductor (Lfilt) persists until it reverses direction, flowing through diode D1 and the patient load and therefore the patient.

Point 1618 is analogous with point 1602, and point 1616 is analogous with point 1603, representing the next time that the current control switch (M1) switches on again. Furthermore, point 1620 is analogous with point 1604, and corresponds to the time at which the current in the filter inductor (Lfilt) reverses direction and begins to increase.

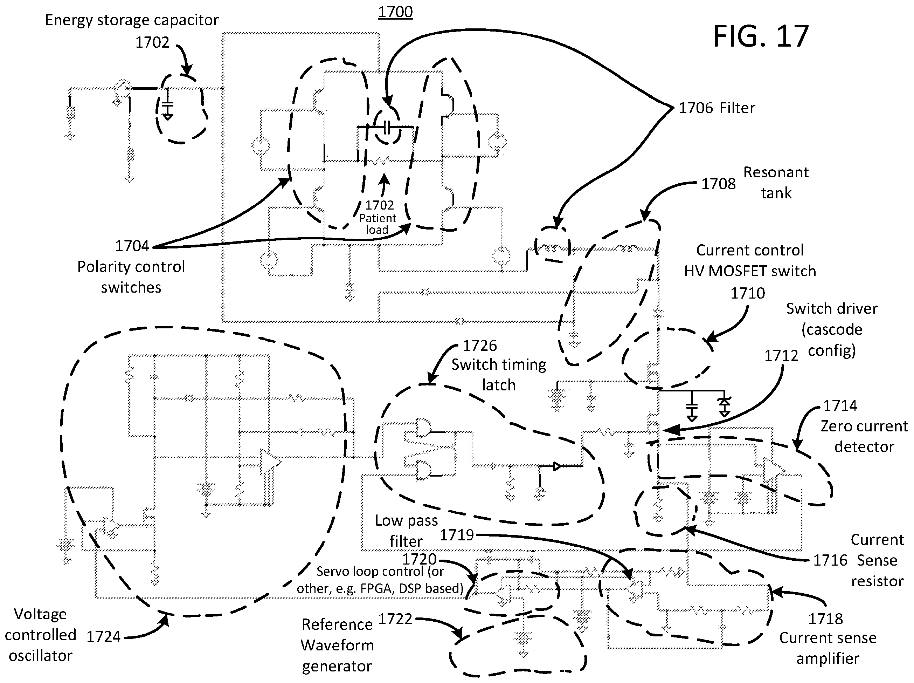

FIG. 17 is an exemplary schematic diagram illustrating components of a quasi-resonant therapeutic current control system, implemented with a circuit 1700 (with potentially other components not depicted). The circuit 1700 illustrates an example of the system 1200 as depicted in FIG. 12 as well as an example of the system 1400 as depicted in FIG. 14.

The circuit 1700 includes: an energy storage capacitor 1702, such as a high voltage capacitor (which can be an example of the power supply 1204 as depicted in FIG. 12 or the energy storage capacitor 1406 as depicted in FIG. 14); polarity control switches 1704, implemented as a full bridge (which can be an example of the polarity control switch(es) 1206 of FIG. 12); a patient load 1702 (which can be an example of the patient load 1208 as depicted in FIG. 12 or the patient load as depicted in FIG. 14); a filter 1706 including a filter capacitor and a filter inductor (which can be an example of the filter 1210 as depicted in FIG. 12 or the filter 1402 as depicted in FIG. 14); a resonant tank 1708 including a resonant tank capacitor and a resonant tank inductor (which can be an example of the resonant tank 1212 as depicted in FIG. 12 or the resonant tank 1404 as depicted in FIG. 14); a current control high voltage MOSFET switch 1710 with a switch driver 1712 using a cascode configuration (which can be an example of the current control switch(es) 1214 as depicted in FIG. 12 or the M1 switch component of feature 1410 as depicted in FIG. 14); a zero current detector 1714, current sense resistor 1716 and current sense amplifier 1718 including a low pass filter 1719 (which can be examples of or include components of the electrical parameter sensor 1214 as depicted in FIG. 12 or the current sense component of feature 1410 of FIG. 14); a servo control loop 1720 (which, in other embodiments, may be implemented differently, such as with FPGA based control or a DSP microcontroller) and a voltage controlled oscillator (VCO) 1724 (which can be components of an example of the controller 1220 as depicted in FIG. 12); and a reference waveform generator 1722 (which can be an example of the specified waveform generator 1218 as depicted in FIG. 12).

Although, in the embodiment depicted, a servo loop control 1720 including an operational amplifier (op amp) is included, in other embodiments, implementations and applications, another form or forms of control may be used an may be more optimal and/or flexible, such as, for example, other embodiments may use FPGA based control or a DSP microcontroller. In the embodiment depicted, the servo loop control 1720 op amp is used in controlling the VCO. Output from the servo loop control 1720 is input to the VCO 1724, and the VCO 1724 generates fixed duration pulses used in control of the switching frequency of the current control MOSFET switch 1710, which is used in adjusting current level through the patient, where increased switching frequency causes increased current to the patient, and vice versa.

In some embodiments, diodes may be used in the circuit 1700 in preventing unwanted residual current, such as through the filter inductor of the filter 1706 when the current control MOSFET switch 1710 turns off, for example. Furthermore, in some embodiments, a capacitor, such as the filter capacitor of the filter 1706, may function as a bypass capacitor, reducing ripple in current through the patient load 1702. Also, in some embodiments, high speed comparators may be used, such as in the zero current detector 1714 and the VCO 1724.

As depicted, the switch driver 1712 using a cascode configuration. In some embodiments, however, other configurations may be used, such as, for example, a straight gate drive. However, a cascode configuration may provide advantages such as high voltage protection/isolation, such as of low voltage transistors. For example, a cascode configuration may provide for increased stability as large voltage fluctuations may occur in the resonant topology.

In various embodiments, the reference waveform generator 1722 may generate a reference waveform using various techniques and components, such as may include use of a digital to analog converter (DAC), for example.

In some embodiments, components such as Zener diodes may be used in protecting the circuit 1700 against an overvoltage condition. Furthermore, in some embodiments, some components may be customized for high voltage operation, or to perform better or be more reliable under high voltage conditions. For example, in some embodiments, some components, such as the filter 1706 inductor and the resonant tank 1708 inductor, are be required to withstand high voltage and each may require a substantial layer of insulation on the conductors. This may, in some embodiments, include customization of these components, such as to add such insulation accordingly.

In some embodiments, the circuit 1700 includes, for example, soft switching including a zero voltage switching (ZVS) converter that may be set at a high switching speed, which may be selected considering optimization of features including size and weight, such as, for example, 1 MHz (or, e.g., 0.7-1.3MHz), which may, given the high frequency and voltage, make use of high bandgap materials, such as silicon carbide (SiC) or Gallium nitride (GaN) for components associated with components such as, for example, switches, diodes and others. Furthermore, the zero current detector 1714 may be used in detecting, detecting more accurately or precisely, or confirming a zero current condition in order to more facilitate more accurately or precisely accomplishing soft switching such as ZVS.

The current control high voltage MOSFET switch may be or include, for example, one or more Silicon (Si) MOSFETs or Silicon Carbide (SiC) MOSFETs, such as, for example, a 3.3kV MOSFET driven in a cascode configuration.

The polarity control switches 1704 may be implemented, for example, as a monolithic IGBT bridge, or in other ways, although as depicted includes full bridge (e.g., H-bridge) using four MOSFETs and four bipolar PNP transistors to switch between positive and negative phases of the resolution bandwidth filter (RBF). The associated gate drive could be or include, for example, independent voltage sources or isolated gate drivers, such as used for motor control. While not all diodes are shown in FIG. 17, in some embodiments, various components such as diodes may be implemented using wide bandgap materials, such as silicon carbide (SiC) or Gallium nitride (GaN), including, for example, one or more 3.3kV rated SiC or GaN diodes.

The reference waveform generator 1722 may be or include, for example, a voltage source programmed to generate a reference waveform for the servo loop control 1720 that controls the mean source current in the current control MOSFET switch 1710.

In some embodiments, the current sense resistor 1716 is used in obtaining a signal or signals that can be used by the controller (potentially among other information or measured parameters) in determining any necessary adjustment to the current control switch 1710 to cause the electrotherapeutic waveform being delivered to the patient to correspond, or to more accurately or precisely correspond, to the reference waveform provided by the reference waveform generator 1722. The current sense resister 1716 is a component of a current sensing portion of the circuit that outputs the average current being drawn from the power supply. Switching frequency of the current control MOSFET switch 1710 is adjusted in order to adjust the waveform being delivered to the patient, such as by causing an incremental increase or decrease in the amount of current delivered to the patient relative to amount that would be delivered with no switching frequency adjustment.

Details of an example controller that may be used in some embodiments are provided with reference to FIG. 4A.

In the example circuit 1700, as described generally with reference to FIG. 4C, an error function e(t), for example, may be used. For example, in some embodiments, an error term may be generated from input including output from the current sense resistor 1716 and the low pass filter 1719, such as in comparing, at particular times during delivery of the electrotherapeutic waveform to the patient, a signal representative of the measured current (in the depicted embodiment, the average current drawn from the power supply) with a signal representative of the reference waveform current. Based at least in part on this comparison (or some other comparison, examples of which are described below), an error term may be generated and used as input to, or in driving and affecting the output of, the voltage controlled oscillator (VCO) 1724. The output of the VCO may, in turn, be used in affecting or controlling the switching frequency of the current control MOSFET switch 1710. For example, the output of the VCO may be used as input to, or in driving, the switch timing latch, which may include a D flip flop (DFF) that is used for the gate drive to the current control MOSFET switch 1710. Furthermore, in some embodiments, the latch or DFF may be reset when current in the current control MOSFET switch 1710 falls to zero, which generates a reset pulse causing the reset, and this may facilitate ensuring zero current switching (ZCS) or substantially ZCS.

In the present example circuit 1700, however, the current control MOSFET switch 1710 and the current sense components, including the current sense resistor 1716, are placed at a different node than the patient load, and, particularly, connected to ground. In defibrillation applications, for example, the patient load 1702 does not need to be referred to ground, so no patient load connection to ground is required. This placement of the current control switch 1710 and the current sense 1716 can create advantages, such as, for example, that the drive requirement is much easier to meet and isolation of the associated gate drive is not required. Nonetheless, in other embodiments, however, the current control switch 1710 (or switches) and the current sense may be placed to measure current through the patient load 1702 directly. This may have advantages in that current sense through the patient may be measured directly and precisely, but may require high voltage isolation.

Since current to the patient is not directly measured, operation of the controller, in determining a necessary adjustment of the switching frequency of the current control MOSFET switch, may need to account for the difference between the measured current and the current to the patient. This may be necessary since it is ultimately the current through the patient that must be determined or estimated and adjusted as necessary. There are a number of different ways in which this can be accomplished.

In some embodiments, current to the patient / patient load 1702 is determined or estimated based at least in part on the measured current, potentially also using other data, such as measured electrical parameters within the circuit 1700, data about circuit configuration or components, data about actual or anticipated conditions present during delivery of the electrotherapeutic waveform to the patient, or other information. For example, in some embodiments, current to the patient may be determined or estimated based on measured current through the current control MOSFET switch 1710 in combination with other measured or estimated parameters such as one or more capacitor voltages, one or more measured or estimated switching losses, or other data. In some embodiments, one or more algorithms may be used in this regard. As such, a correspondence or mapping may be made between the combination of parameters including the measured current and the determined or estimated current through the patient load. This mapping may be utilized by the controller in determining necessary adjustments to the current control MOSFET switch 1710 such that the current waveform being delivered to the patient matches the reference waveform.

In various embodiments, this mapping may be done prior to delivery of the electrotherapeutic waveform to the patient, potentially by using one or more algorithms, and for a range of parameters and current levels, or may be done by the controller during delivery, such as in real time, potentially using one or more algorithms stored in a memory of the controller. Furthermore, the mapping may be done analytically, such as by using circuit analysis, or may be done empirically, such as by actual testing using operation of the circuit, or may done by some combination of the two. In some embodiments, mapping data may be stored in the memory of the controller, or may be input to the controller.

In various embodiments, the controller may operate in various ways in incorporating the mapping. For example, the controller may compare a signal representative of the measured current with a signal representative of the reference waveform current, generating an error signal relating to the difference between the measured current and the reference waveform current. However, in the embodiment described with reference to FIG. 4C, the controller may determine the necessary adjustment based on minimizing an error function e(t), which represents, over time, the difference between the current to the patient and the reference waveform current. As such, in some embodiments, the mapping may be used to determine a derived error signal representing the difference between the determined or estimated current to the patient and reference waveform current, and the error function e(t) may then be used. Of course, there are many other ways in which the controller could operate. In some embodiments, however, these may have in common that the current to the patient must be determined or estimated using one or more parameters including the measured current (not to the patient), and the adjustment to the current control MOSFET switch 1710 must be based on adjusting the current waveform being delivered to the patient to correspond to the reference waveform.

In some embodiments, typically commercially available electrical components may not be designed, or ideally designed, to handle the high voltages and associated stresses associated with embodiments of the disclosure, including the circuit 1700, such as in defibrillation applications, for example. As such, in some embodiments, adjustment, modification, or additions are made to some components, or multiple components may be used, for example, in series to achieve, or better achieve, an additive effect using available components. For example, some commercially available components, such as inductors, may have a dielectric insulation capable of withstanding up to 2.8 kV or 5.6kV, which may not be sufficient or ideal for some applications according to the present disclosure. In some embodiments, for example, measures are taken to improve such specifications or tolerances, such as by applying some form of encapsulation or conformal coat immersion to commercially available components, which can effectively lead to creation of custom components better suited to the very high voltage applications. Extensive experimentation and testing may be performed to ensure the effectiveness, reliability and safety of such customizations.

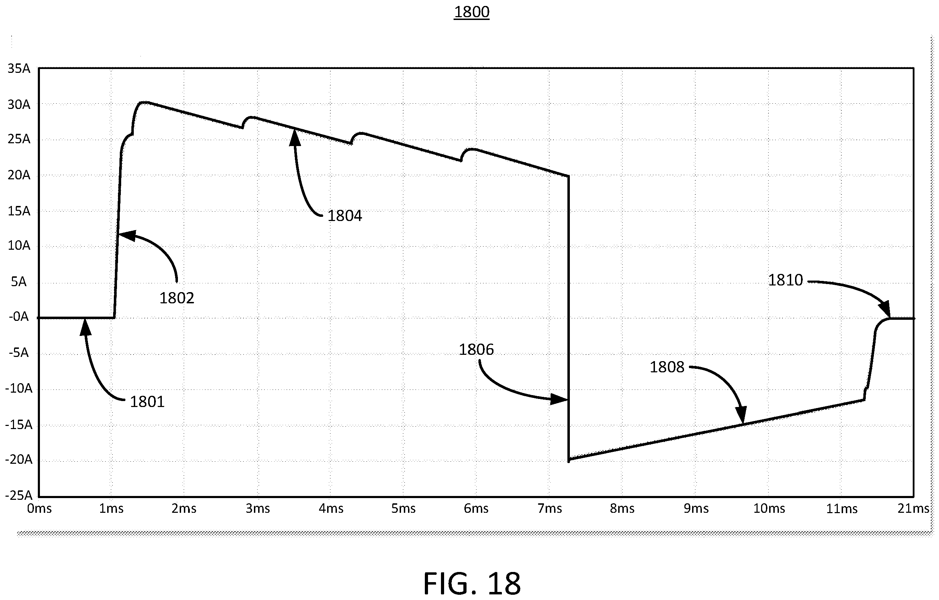

FIG. 18 is a graphical illustration of a biphasic rectilinear waveform 1800 that can be produced by a quasi-resonant therapeutic current control system, such as may be implemented as circuit 1700 of FIG. 17. The depicted waveform 1800 represents an actual waveform produced the circuit 1700 depicted in FIG. 17. In some embodiments, quasi-resonant circuits may be used in delivering specified, arbitrary current waveforms to patients, of a wide variety of shapes and forms. The waveform 1800 represents one particular such waveform, which may be advantageous to use in some applications, including defibrillation applications. To produce the depicted waveform 1800, a matching reference current waveform is generated, and associated signals are input to a controller of the circuit 1700. The controller then repeatedly or continuously adjusts a switching frequency of the high voltage MOSFET switch 1710 of the circuit 1700 to adjust the generated electrotherapeutic current waveform during delivery to the patient to match the reference waveform, as described, for example, with reference to FIGS. 12-17.

The biphasic rectilinear waveform 1800 includes, from zero current 1801, a sharp current level spike 1802, followed by a roughly steady but slightly declining and somewhat varying/bumpy current level period 1804, followed by a sharp drop 1808 to a negative current level, representing a change in polarity, followed by a period 1808 during which the current level first gradually, and then more sharply, moves back to zero current 1810.

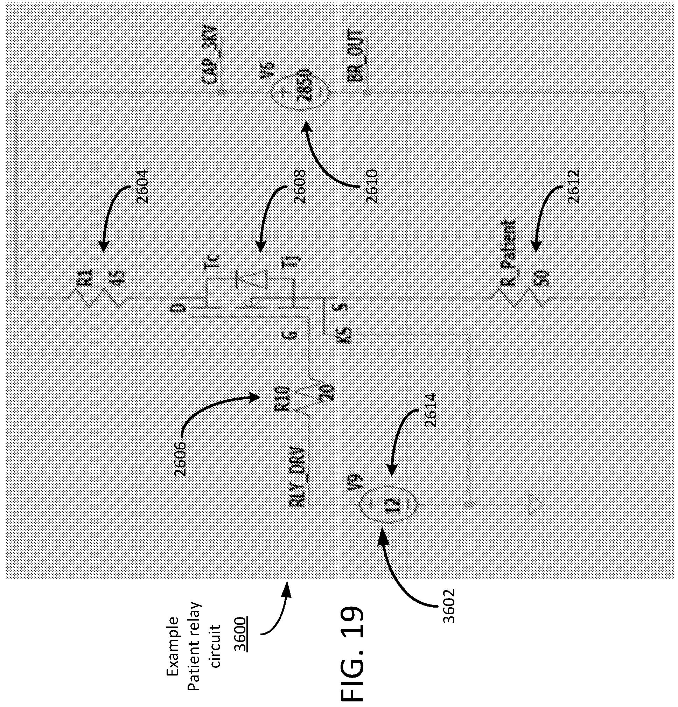

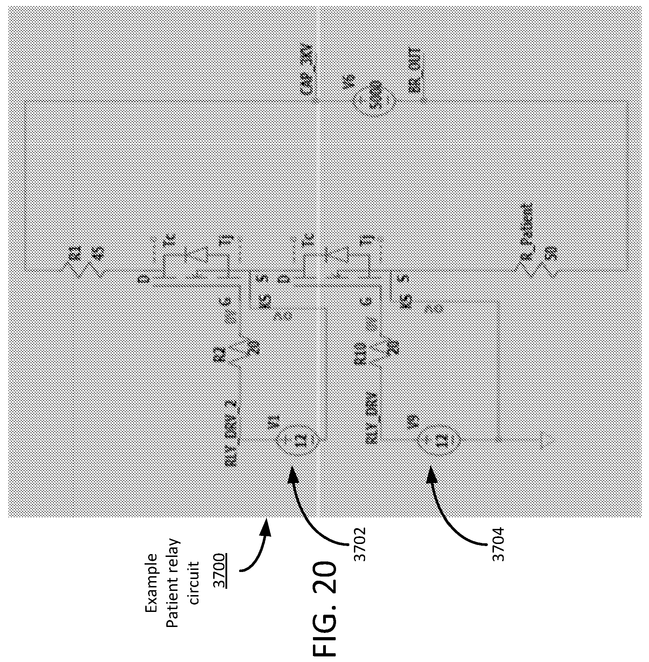

FIG. 19 is an exemplary schematic diagram of a patient relay circuit 3600 with a single driver circuit 3602, while FIG. 20 is an exemplary schematic diagram of a patient relay 3700 with two driver circuits 3702, 3704 connected in series, which can be used in or with any of various therapeutic current control networks, in accordance with embodiments of the present disclosure, such as, for example, embodiments of the networks 275, 200, 1200 shown in FIGS. 2A, 2B, and 12. In some embodiments, wide bandgap materials, such as silicon carbide (SiC) or gallium nitride (GaN) are used in components of the patient relays, such as may include SiC MOSFETs. For example, in some embodiments, use of SiC MOSFETs can increase energy efficiency and performance, including minimizing leakage current. This can include, for example, keeping total leakage current (such as may be calculated as the sum of the maximum leakage currents of all of the SiC MOSFETs) under a maximum, such as, for example, under 50 microamps (or, e.g. more or less, as required, such as 30-70 microamps).

In some embodiments, such as the embodiment 3700 depicted in FIG. 20, two or more driver circuits, such as driver circuits each associated with a SiC MOSFET, may be included and connected in series, such as to achieve the necessary voltage, such as in the event that individual SiC MOSFETs are not rated at a sufficient voltage. For example, in some embodiments, each SiC MOSFET may be rated at 3.3kV, and two (or more) may be used in driver circuits in series. In some embodiments in which two or more driver circuits are used, the patient relay circuit may include one or more balancing circuit portions that may include one or more resistors and/or one or more capacitors.

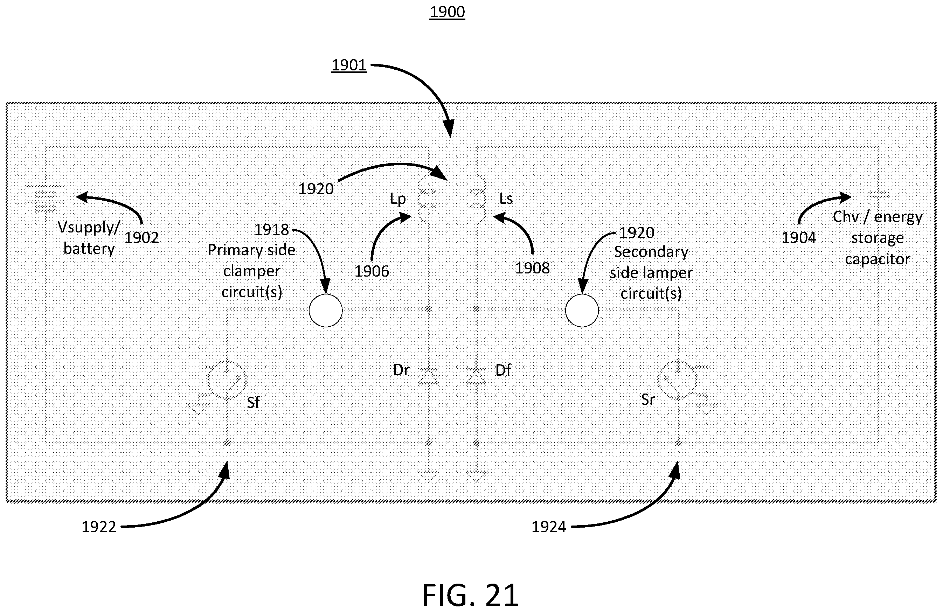

FIG. 21 is an exemplary schematic diagram of a bidirectional charging network 1900, including a simplified circuit 1901, in accordance with embodiments of the present disclosure. The overall circuit 1901 generally includes some aspects that may be associated with a flyback converter configuration. The circuit 1901 is connected with a voltage supply source, such as a battery 1902 and an energy storage capacitor 1904, such as a high voltage energy storage capacitor, which energy storage capacitor 1904 may be electrically coupled with, and provide energy to, a therapeutic current control network for delivery of an electrotherapeutic waveform to a patient, for example. Various characteristics of bidirectional charging networks, generally, and some other specific examples, according to various embodiments, are described, for example, with reference to previous figures, including FIGS. 2C and 10A-D.

The overall circuit 1901, which may include other non-depicted components, includes a first circuit or primary side 1922, connected with the battery 1902 and a second circuit or secondary side 1924 connected with the energy storage capacitor 1904. The circuit 1901 further includes a central transformer 1920, including inductors Lp and Ls, connected with the primary side 1922 and the secondary side 1924. The transformer 1918 is for use, such as during particular time periods, in storing energy from the battery 1902 and in transferring at least a portion of the stored energy to the energy storage capacitor 1904, as well as storing second energy from the energy storage capacitor 1904 and in transferring at least a portion of the stored second energy to the battery 1902. The circuit 1901 also includes switches Sf and Sr and diodes Dr and Df, as described below.

The circuit 1901 further includes a primary side clamp circuit 1918 and a secondary side clamp circuit 1920. In various embodiments, circuits may include one or more active or passive clamp circuits on either or both of the primary side 1922 and the secondary side 1924, and may, in some embodiments, include one or more snubber circuits. Generally, clamp circuits can be used, for example, to fix signal peaks. In some embodiments of a bidirectional charging control circuit with flyback aspects, active and/or passive clamp circuits can be used on both the primary side 1922 and the secondary side 1924 to advantage and to improve performance. For example, clamp circuits can facilitate greater or more precise control, or greater ease of control, of circuit operation. Also, clamp circuits generally can facilitate saving of energy or greater energy efficiency. Active clamps can facilitate re-use of energy and enable or facilitate valley switching, for increased energy efficiency, by slowing oscillations, although passive clamp circuits do not preclude valley switching. Furthermore, clamp circuits can cap or reduce overly high voltages or voltage fluctuations or peaks, which can stress or damage electrical components, particularly in high voltage applications.

Given the high voltage on the secondary side 1924, however, active clamp circuits on the secondary side may be larger, heavier and more expensive, while active clamp circuits on the primary side may be smaller, lighter and less expensive. As such, depending on a particular optimized balance of factors, an active clamp circuit may be preferred on the primary side 1922, while a passive clamp circuit may be preferred on the secondary side 1924. However, in some embodiments, an active clamp on the secondary side can increase energy efficiency significantly, such as by approximately 10-20%, for example, and so, in some embodiments, an active clamp on the secondary side may be preferred in spite of potential disadvantages or costs. Additionally, control of operation of the primary side 1922 is generally easier than of the secondary side 1924, given the lower voltage on the primary side 1922. Since an active clamp circuit requires control, which is generally easier on the primary side 1922 and more difficult on the secondary side 1924, this presents a reason to potentially prefer an active clamp circuit on the primary side 1922 and a passive clamp circuit on the secondary side 1924. On the other hand, since a passive clamp also has potential advantages, as described, in some embodiments, a passive clamp may be used on the primary side 1922.

Conceptually, in some embodiments, a bidirectional charging control circuit with flyback aspects, such as in accordance with simplified circuit 1901, may be viewed as essentially including a transformer (such as transformer 1920) with a control switch portion (such as switch Sf and diode Dr) and flyback switch portion (such as switch Sr and diode Df), which can be viewed as mirrored across the transformer 1920 (from primary side 1922 to secondary side 1924). Further simplification is possible with the recognition that the flyback diode (such as Dr) is essentially a switch; therefore, the diode function can be integrated into control switches, allowing reduction in this area to a single switch on either side of the transformer 1920 (such as switches Sf and Sr). In such a simplified conceptual model, the control of the direction and magnitude of energy moved across the transformer 1920 is controlled by controlling the timing of operation of the two switches (such as switches Sf and Sr). In the depicted simplified circuit 1901, diodes Dr and Df are used, rather than integrating the switch functionality, to simplify the circuit design and controls. However, the concept of using the control switch (such as Sf) to perform the function of the diode (such as Dr) can be leveraged to significantly improve overall practical circuit performance.

Generally, bidirectional charging control networks using flyback related configurations, such as circuit 1901, may provide certain advantages in applications in various embodiments. For example, they may utilize relatively few components. Furthermore, they may allow a high voltage boost ratio with a low transformer turns ratio, which can allow reduced transformer parasitics. Additionally, they may also provide a relatively high power density relative to other configurations, which can increase portability. However, they may require particular or precise configurations to allow sufficient control. Also, they may require components that can withstand a high degree of stress including high voltage stress, where such with high voltage stress exceeds input and output voltages. Furthermore, in some embodiments, flyback configuration aspects require storing significant energy in a magnetic field in the transformer core, which can result in potentially greater core losses than may be experienced in other configurations if those configurations do not store energy in a magnetic field in the transformer core, for example. Generally, bidirectional charging control networks using a flyback related configuration, such as circuit 1901, may, in some embodiments, provide or allow advantages such as high power density and relatively few components, for example.

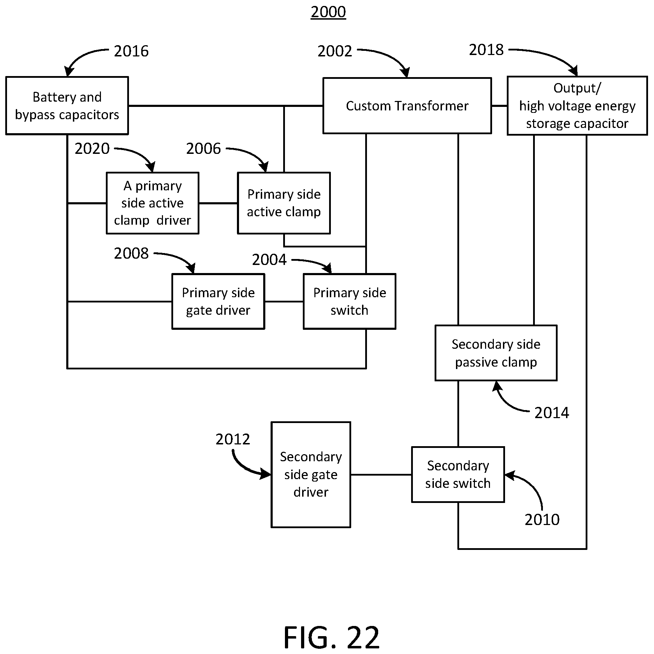

FIG. 22 is a block diagram of a bidirectional charging network, implemented as a circuit 2000, which may be an example implementation incorporating aspects of the simplified circuit 1901 as depicted in FIG. 21. The simplified circuit 2000 includes (potentially among other things not depicted): a custom transformer 2002 (which may be an example of the transformer 1920 as depicted in FIG. 21), a primary side switch 2004 (which may be an example of switch Sf as depicted in FIG. 21), a primary side active clamp (clamp circuit) 2006 (which may be an example of the primary side clamp circuit 1918 as depicted in FIG. 21), a primary side gate driver 2008, a secondary side switch 2010 (which may be an example of switch Sr as depicted in FIG. 21), a secondary side gate driver 2012, a secondary side passive clamp 2014 (which may be an example of the secondary side clamp circuit 1920 as depicted in FIG. 21), a battery (which may be an example of the battery 1902 as depicted in FIG. 21) and bypass capacitors 2016, an output/high voltage energy storage capacitor 2018 (which may be an example of the energy storage capacitor 1904 as depicted in FIG. 21), and a primary side active clamp driver 2020.

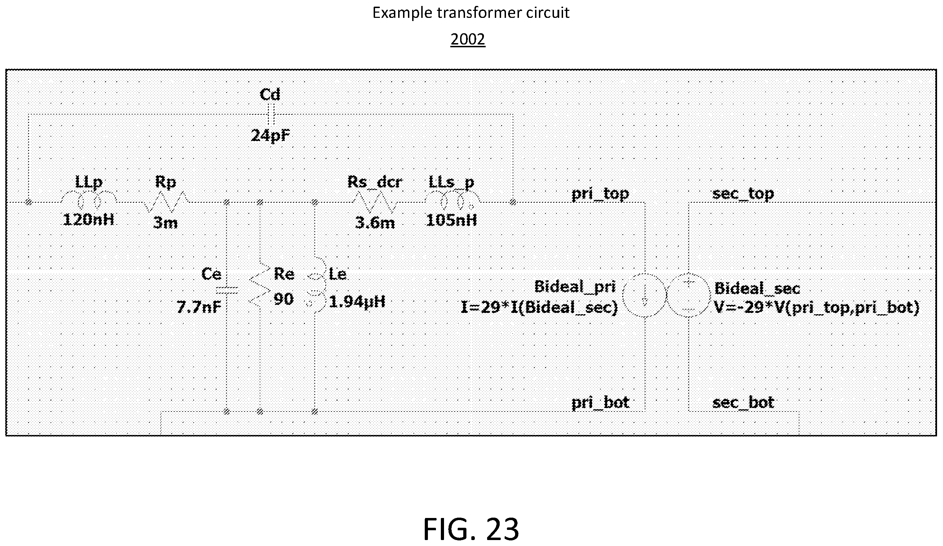

FIGS. 23-29 are schematic diagrams of example circuits implementing components of the circuit 2000 as depicted in FIG. 22.

FIG. 23 is a schematic diagram of a custom transformer circuit 2002 (as depicted in FIG. 22). Generally, the custom transformer circuit 2002 is designed for high voltage isolation and has a very high turns ratio.

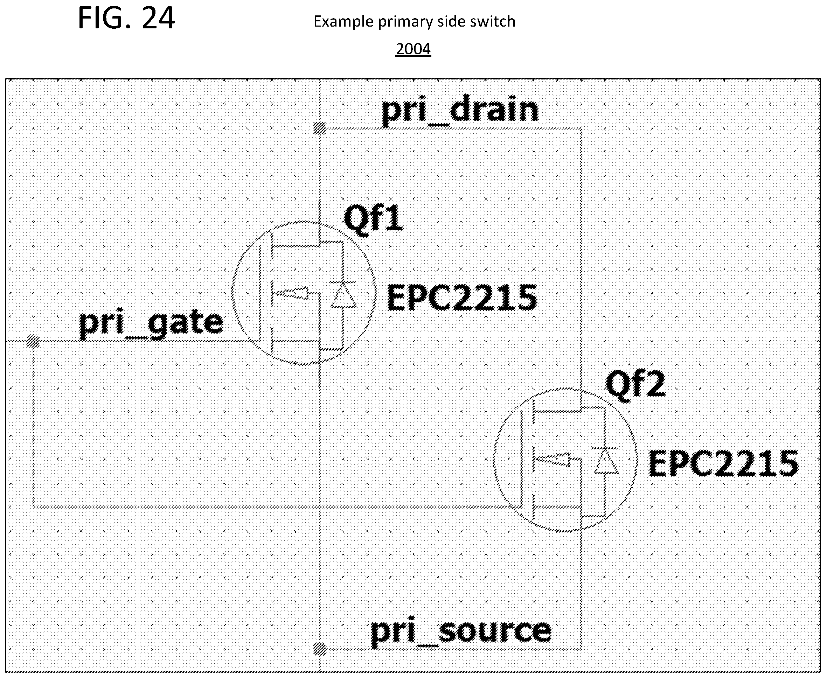

FIG. 24 is a schematic diagram of a primary side switch 2004. The primary side switch 2004 is configured or selected for minimized switching losses and minimal physical volume.

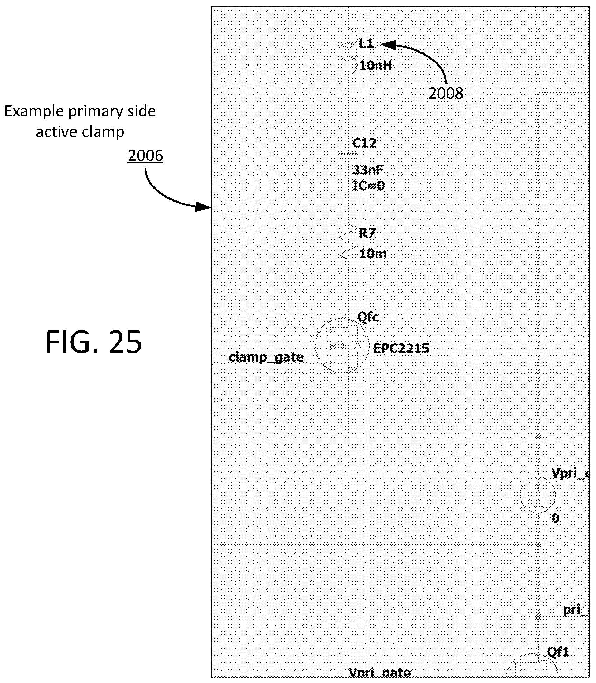

FIG. 25 is a schematic diagram of a primary side active clamp 2006. Advantages of an active clamp, particularly on the primary side, given its lower voltage relative to the secondary side, are described with reference to FIG. 21. The clamp 2006 uses a switched RC damper. Generally, tuning of the resistor (R7) and the capacitor (C12) is performed in connection with the physical device configuration, the resistance is minimized to minimize energy losses. In some embodiments, R7 may be omitted, such as, for example, if the ESR of the capacitor C12 provides sufficient damping. In FIG. 25, inductor 2008 may, for example, represent either the parasitic inductance of the clamp 2006 or an intentional inductance to slow the clamp switching transients to improve EMC performance.

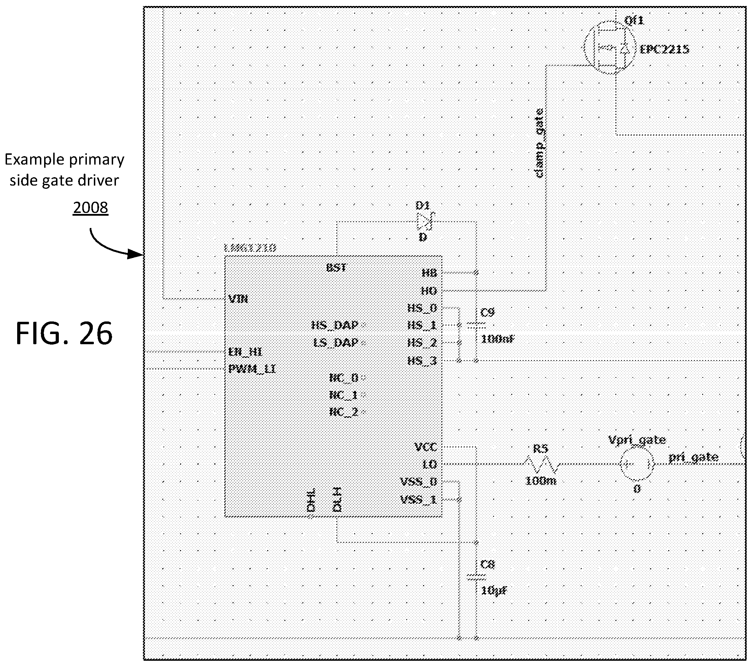

FIG. 26 is a schematic diagram of a primary side gate driver 2008. The gate driver 2008 is configured or selected to keep control of the primary side switch 2004 and clamp switch as simple as possible, and is specifically designed for driving GANFETs (Gallium nitride FETs).

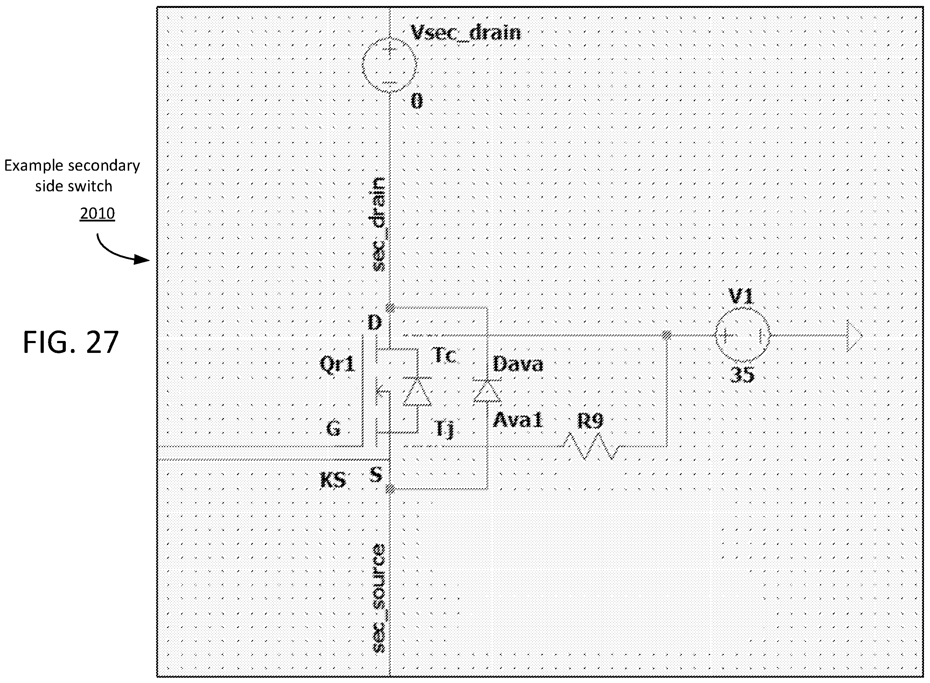

FIG. 27 is a schematic diagram of a secondary side switch 2010. The switch 2010 is configured or selected to allow for ultra-fast switching speed and to minimize energy losses.

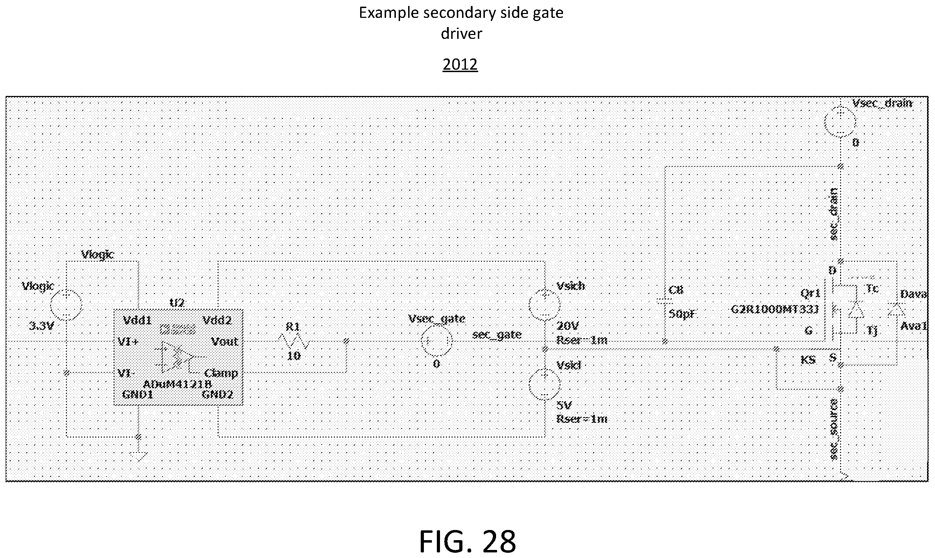

FIG. 28 is a schematic diagram of a secondary side gate driver 2012, implemented as an isolated gate driver. In some embodiments, the gate driver may require an isolated DC/DC converter. In various embodiments, the gate driver 2012 may use a separate converter or may be combined with a controller.

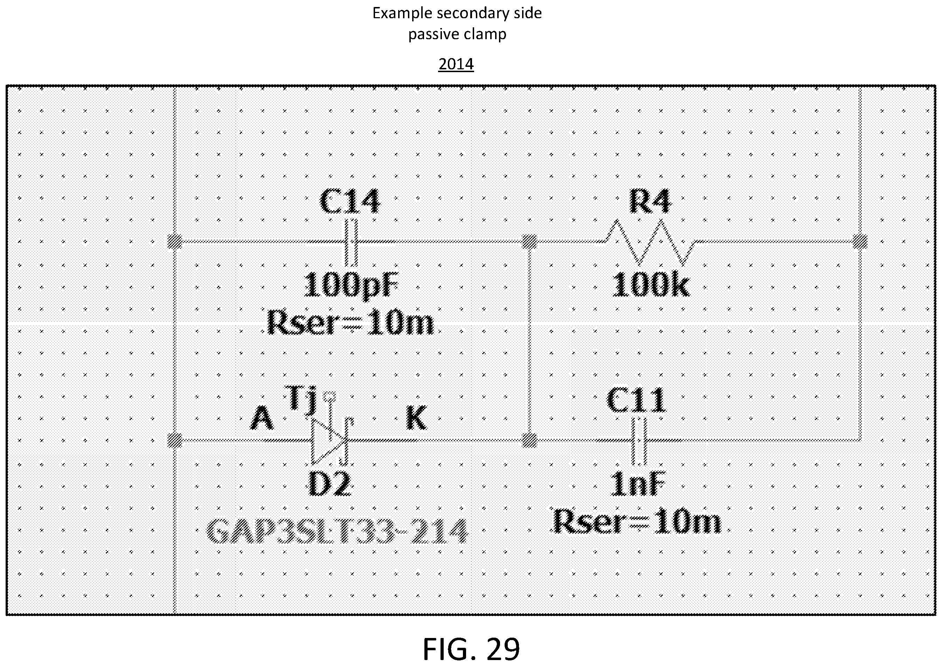

FIG. 29 is an exemplary schematic diagram of a secondary side passive clamp 2014. In some embodiments, the clamp 2014 may use one or more Schottky diodes for faster response time. The clamp 2014 may need to be tuned to the physical circuit configuration.

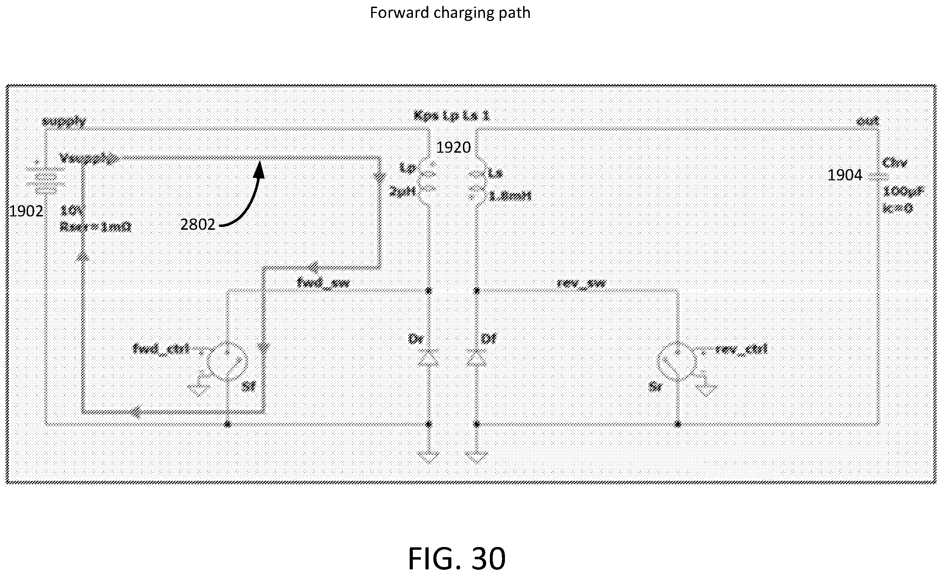

FIGS. 30-33 are exemplary schematic diagrams of charging and current flow paths that can be implemented with the simplified bidirectional charging control circuit 1901 as depicted in FIG. 21 or the circuit 2000 as depicted in FIG. 22.

With the bidirectional charging control circuit 1901 (or a circuit implementing it, such as the circuit 2000 as depicted in FIG. 22), in forward charging, energy is transferred from the battery to 1902 to the energy storage capacitor 1904, whereas, in a reverse charging stage, energy is transferred from the energy storage capacitor 1904 to the battery 1902. Forward charging includes two stages - a charging stage in which energy is transferred from the battery 1902 to the transformer 1920 for storage in the transformer 1920, followed by a discharging stage in which energy is transferred from the transformer 1920 to the energy storage capacitor 1904 for storage in the energy storage capacitor. In this way, the forward charging and discharging stages transfer energy from the battery 1902 to the energy storage capacitor 1904, for storage by the energy storage capacitor 1904. This may be used, for example, to charge the energy storage capacitor 1904 so that it has energy to provide to a therapeutic current control network for delivering an electrotherapeutic waveform to a patient.

However, as the circuit 1901 is bidirectional, there is also a generally analogous reverse mode. Reverse charging also includes two stages - a charging stage in which energy is transferred from the energy storage capacitor 1904 to the transformer 1920 for storage in the transformer 1920, followed by a discharging stage in which energy is transferred from the transformer 1920 to the battery 1902 for storage in the battery 1902. In this way, the reverse charging and discharging stages transfer energy from the energy storage capacitor 1904 to the battery 1902, for storage by the battery 1902. This may be used, for example, in returning unused energy from the energy storage capacitor 1904 back to the battery 1902.

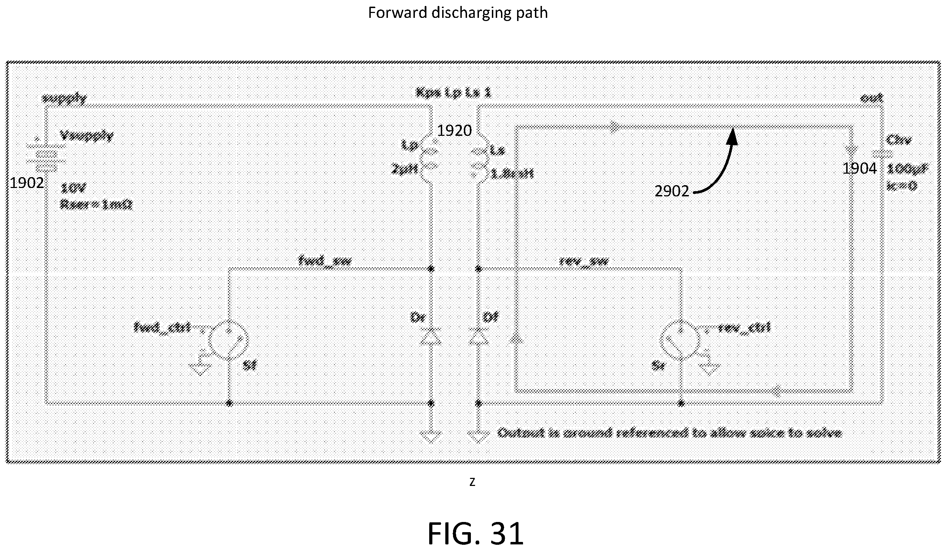

FIG. 30 shows a forward charging path 2802, with current generally flowing in a clockwise direction, allowing energy to transfer from the battery 1902 to the transformer 1920 for storage in the transformer 1920. FIG. 31 shows a forward discharging path 2902, with generally clockwise current flow, allowing energy transfer from the transformer 1920 to the energy storage capacitor 1906 for storage by the energy storage capacitor 1904.

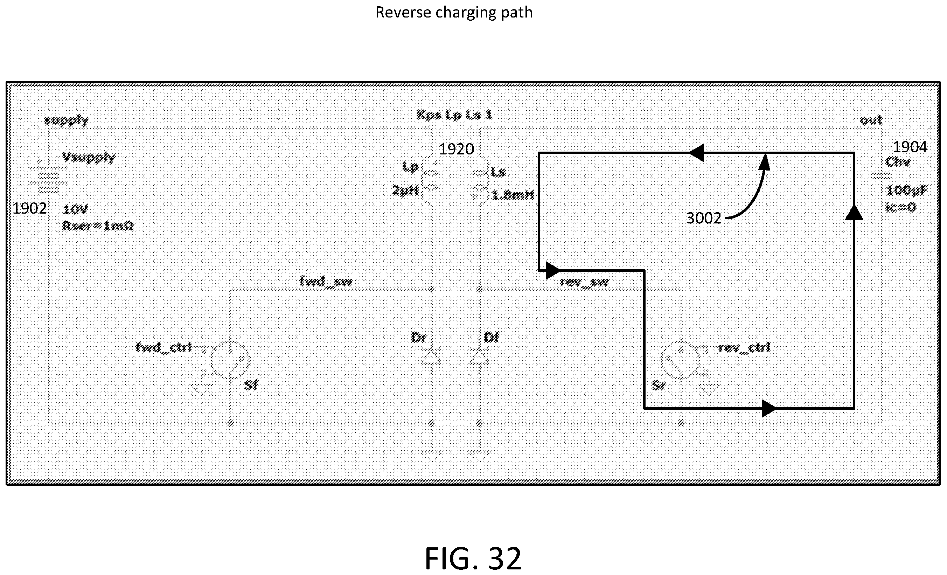

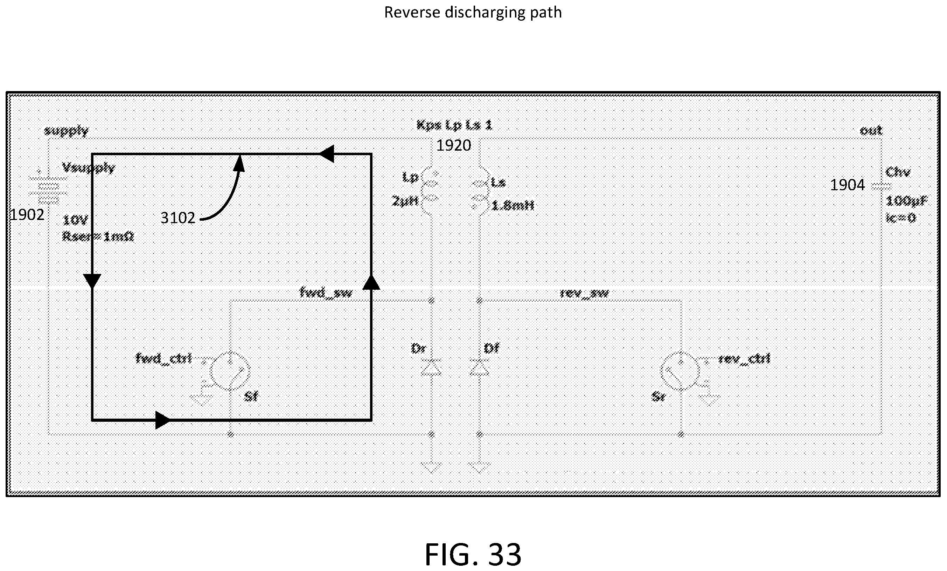

FIG. 32 shows a reverse charging path 3002, with generally counterclockwise current flow, allowing energy to transfer from the energy storage capacitor 1904 to the central transformer 1920 for storage in the transformer 1920. FIG. 33 shows a reverse discharging path, with generally counterclockwise current flow, allowing energy to transfer from the transformer 1920 the battery 1902 for storage by the battery.

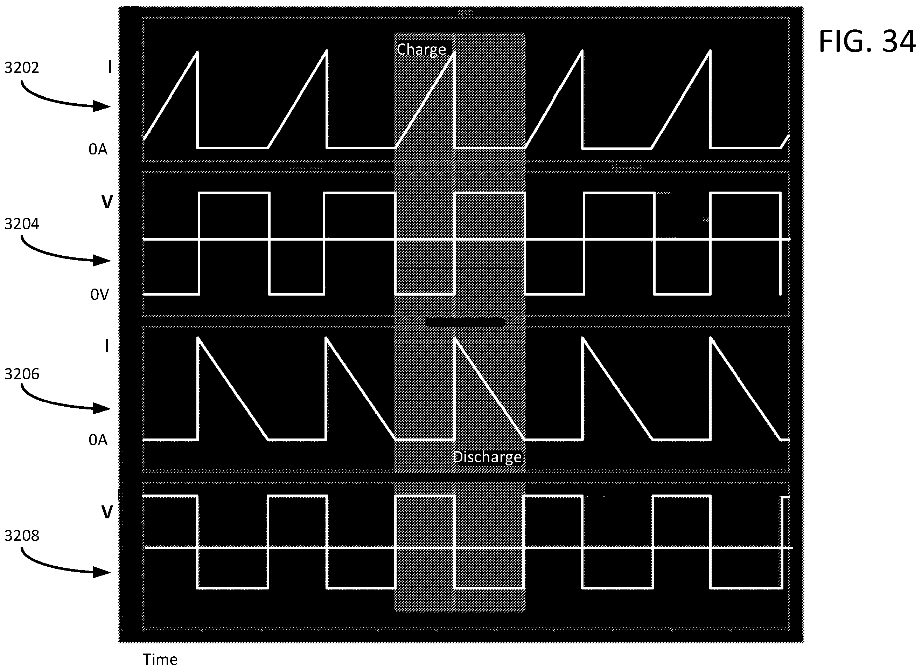

FIG. 34 is a graphical illustration of idealized charging waveforms in forward and reverse charging (with different scaling), which can be associated with operation of the simplified bidirectional charging control circuit 1901 as depicted in FIG. 21. As depicted, the waveforms are associated with Boundary Conduction Mode (BCM) control. Particularly, plot 3202 shows the current as measured at switch Sf, which represents the current in the primary side 1922 winding, in forward charging, or the current as measured at switch Sr, which represents the current in the secondary side 1924 winding, in reverse charging. Plot 3206 shows the current as measured at switch Df, which represents the current in the secondary side 1924 winding, in forward charging, or the current as measured at Dr, which represents the current in the primary side 1922 winding, in reverse charging. As such, in the embodiment depicted, the current through Df and Dr always represents the current through the primary side 1922 winding and the current through Sf and Dr always represents the current through the secondary side 1924 winding.

In voltage waveform plots 3204 and 3208 show, respectively, the square waves show the voltage at the bottom of the primary and secondary winding, respectively, relative to the negative terminal of the battery 1902 and capacitor 1904, as depicted in FIG. 21, respectively. In each of the square waves, voltage drops to zero - the bottom horizontal line of each square wave - when current is switched on and flowing through, but increases to the higher voltage - the top horizontal line of each square wave - when current is switched off and not flowing through. In plot 3204, the central horizontal line represents battery 1902 voltage, and, in plot 3208, the central horizontal line represents energy storage capacitor 1904 voltage.

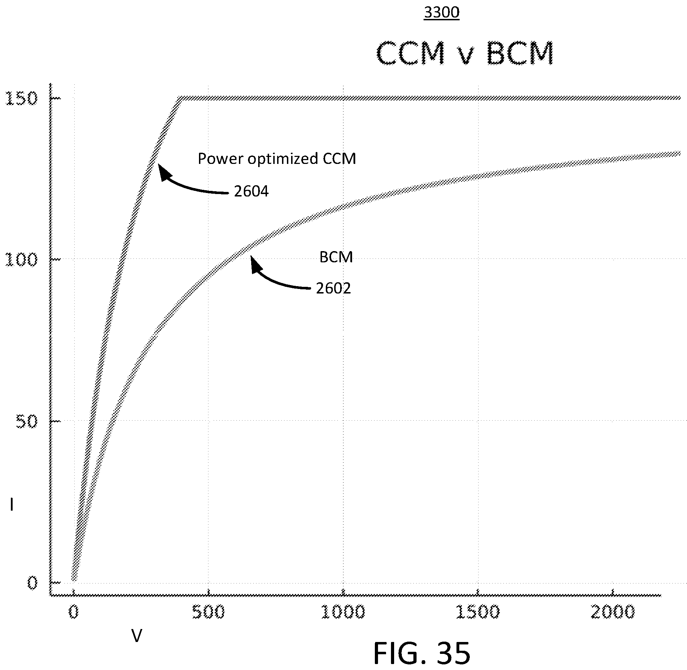

FIG. 35 is a graphical illustration of a power optimized continuous conduction mode (CCM) waveform and a boundary conduction mode (BCM) waveform, in accordance with embodiments of the present disclosure. Plot 2602 relates to BCM while plot 2604 relates to CCM.

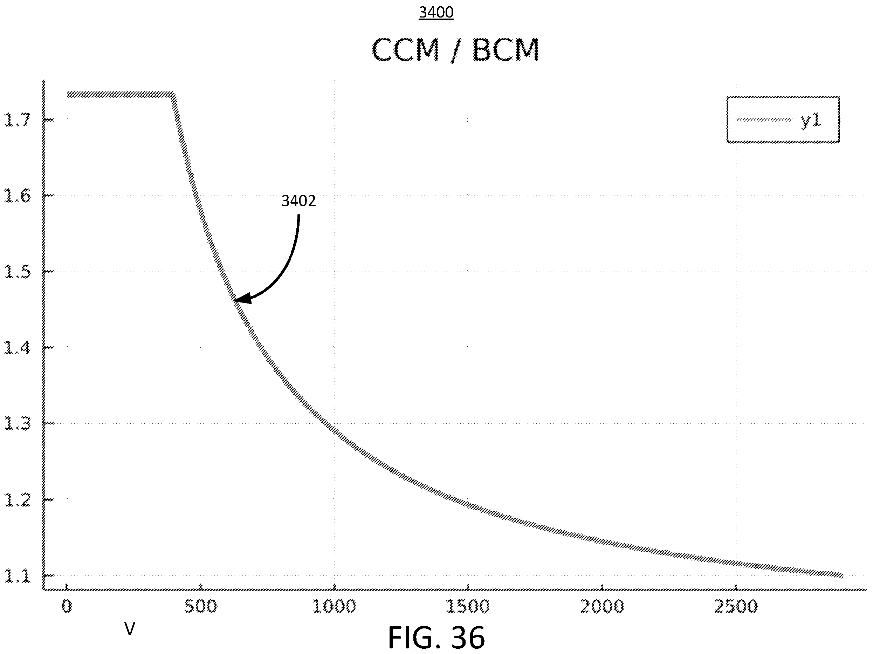

FIG. 36 is a graphical illustration where plot 3402 shows power optimized CCM energy transfer divided by BCM energy transfer, according to some embodiments, over a range of voltages, shown on the horizontal axis, at the output /energy storage capacitor. As such, the vertical axis value corresponds with how much faster energy is transferred with power-optimized CCM relative to BCM.

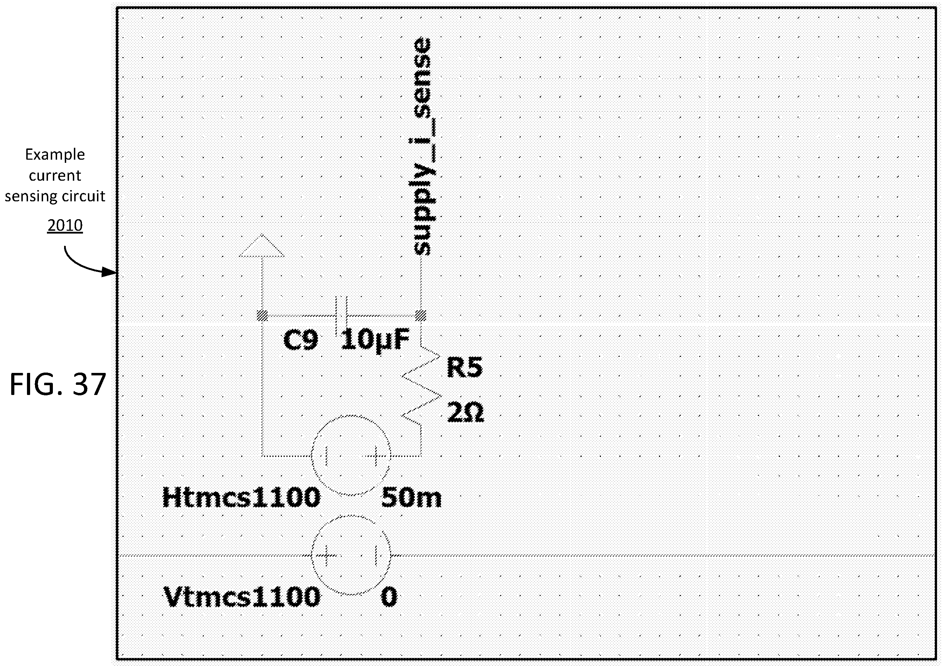

FIG. 37 is an exemplary schematic diagram of an average input current sense circuit 2010 for use with CCM control in accordance with embodiments of the present disclosure. Not all CCM control is based at least in part on the average battery current. For example, many are based on measurements of the winding current. Some embodiments of the present disclosure provide, for example, CCM control that compensate for instability of the winding voltages and currents at least in part by using the average battery current, such as in conjunction with the known source (e.g., battery) and load (e.g., capacitor), along with an available converter design, such as to ensure maximum energy transfer.

In some embodiments, BCM is used with valley switching, which can increase energy efficiency. In general, average current may be proportional to average energy transfer, whether with CCM vs BCM. A potential advantage of BCM mode, however is that it can, under some circumstances. On the other hand, the CCM may have a higher average current with the same peak current, so it can have a higher energy transfer rate, although potentially at a cost in terms of higher losses / lower efficiency.

As shown by the plots 2602, 2604, power optimized CCM can provide faster energy transfer than BCM at low output voltages, such as, for example, 75% faster energy transfer at low voltages. However, power optimized CCM may also lead to higher switch and transformer losses, relative to BCM, and may make sufficiently stable control difficult or impossible, such as at voltages above a certain threshold, and also may make control especially difficult in reverse mode. In some embodiments, discontinuous conduction mode (DCM) may be used as a fallback in the condition that BCM becomes insufficiently controllable. In some embodiments, the controller may in modes that use CCM, such as power optimized CCM, BCM with valley switching, BCM without valley switching, and DCM, for example, potentially among other things. In some embodiments, the controller may switch between particular such modes, under different circumstances, with factors that may include voltage or voltage range, to provide optimal performance and/or efficiency. In some embodiments, the controller may use one or more algorithms in this regard.

Given the relative advantages and disadvantages of CCM and BCM, particularly at different voltage ranges, some embodiments use a combination of CCM and BCM for optimal operation. For example, CCM may be used during certain time periods when it is optimal or determined to likely be optimal, such as time periods associated with lower voltage ranges (and potentially other conditions), while BCM may be used during certain time periods when it is optimal, such as time periods associated with lower voltage ranges. In some embodiments, a controller may use one or more algorithms to determine whether, at a particular time or during a particular time period, CCM or BCM is optimal or determined to be likely to be optimal. The controller may cause use of CCM or BCM accordingly, and may cause alternation between the two, depending on conditions that may include an associated operational voltage range, for instance.

In bidirectional charging, since energy may be transferred to the battery (such as battery 1902 as depicted in FIG. 21), it may be problematic if the battery is or may become overcharged, such as during operation of the bidirectional charging control circuit 1901. Battery overcharging or battery overcharge, as the terms are used herein, can include, for example, charging a battery, or charge of a battery, beyond a particular predetermined limit, which limit may be defined in view of one or more factors, which factors may include, for example, in connection with the battery and/or its operation, factors relating to rating or voltage rating of the battery, safety, reliability, not damaging the battery, efficient operation, optimal operation or optimal performance, potentially among other things. A battery charging limit or battery charge limit, as the terms are used herein, can include a limit of battery charging or battery charge beyond which the battery is overcharged.

In some embodiments, in use of bidirectional charging control networks and circuits, various configurations, operations, strategies, techniques or methods may be used in order avoid or minimize risk of battery overcharging. For example, in some embodiments, battery charging, or determined, estimated or anticipated battery charging, is limited to a certain percentage of a battery charging limit (such as, for example, at or over 80% and less than 100% of the batter charging limit), so that, for example, unanticipated battery charge beyond a particular anticipated level does not, or is less likely to, unintentionally or accidentally increase battery charge to exceed the battery charging limit. Furthermore, in some embodiments, when energy is transferred from the energy storage capacitor (such as energy storage capacitor 1904 as depicted in FIG. 21) to the battery (such as battery 1902 of FIG. 21), via the transformer 1920, the rate, or the determined, estimated or anticipated rate, at which energy is transferred to the battery 1902 for storage by the battery 1902 may be limited or throttled to avoid overcharging or minimize risk of overcharging, such as may, in some embodiments, include use of one or more algorithms. For example, in some embodiments, the rate, or the determined, estimated or anticipated rate, of energy consumption in circuit operation (which may, for example, energy loss during operation), and the consequent drain on the battery 1902 charge, may be taken into account in determining the limitation or throttling level relating to transfer of energy to the battery 1902, such that overcharging does not occur or is less likely to occur. This can include, for example, ensuring that the rate, or the determined, estimated or anticipated rate, of energy transfer to, or charging of, the battery 1902 does not exceed the rate, or the determined, estimated or anticipated rate, of energy transfer out of, or discharging of, the battery 1902, thus preventing or minimizing the risk of unintended increase of charge of the battery or overcharging the battery.

In some embodiments, control of the bidirectional charging control network or circuit, such as circuit 1901, may include use of valley switching, which can save energy or lead to greater energy efficiency by reducing energy losses, for example. In some embodiments, BCM may be used, or used during some time periods, along with valley switching, and one or more active clamps, such as the active clamp 2006 on the primary side 1922 as depicted in FIG. 25 (or one or more other active clamps on the primary side 1922 or secondary side 1924 as depicted in FIG. 21). However, in some embodiments, valley switching may be used without active clamps.

In some embodiments, with bidirectional charging control networks, such as circuit 1901, wide bandgap materials, such as silicon carbide (SiC) and gallium nitride (GaN) may be used in various electrical components, providing advantages in operation and efficiency, including allowing faster switching with less energy losses, thus allowing smaller components, such as magnetics, thereby reducing weight and size, and increasing potential portability. In some embodiments, such as using CCM control, battery and capacitor charge times of, for example, approximately 4 seconds may be achieved (or, e.g., in seconds, 0.3 - 10, such as 0.3-0.5, 0.5-0.7, 0.7-1.0, 1.0-2.0, 2.0-3.0, 3.0-4.0 or 4.0-7.0, or 7.0-10). However, in some embodiments or implementations, faster charge times may require trade-offs, such as, for example, a substantially larger converter. As such, in some embodiments, selection of charging speed and components may be taken into account and optimized based on factors that may include the anticipated application or applications, the need for portability or degree of portability, and potentially other factors.

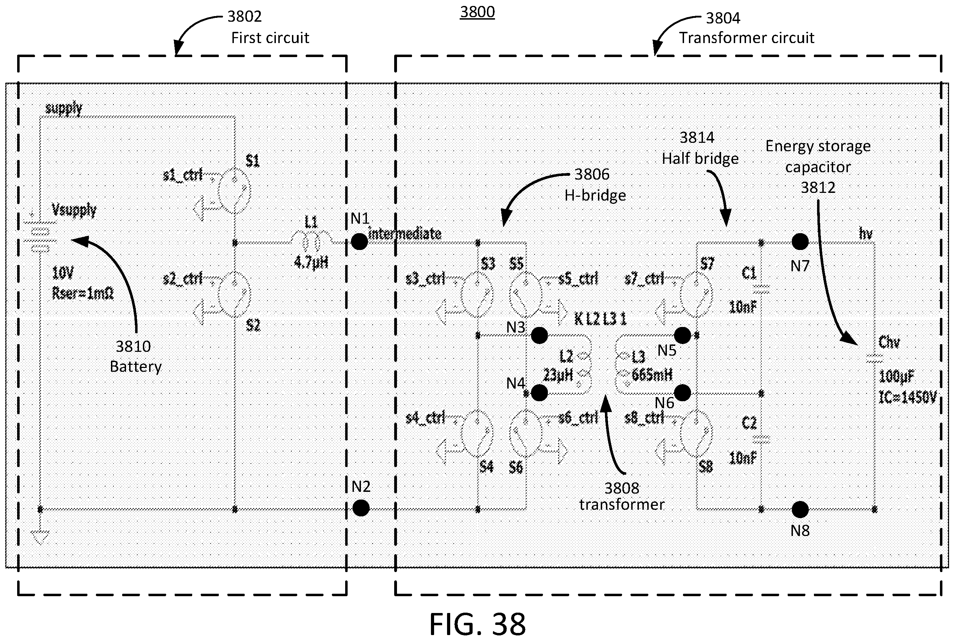

FIG. 38 is an exemplary schematic diagram of a bidirectional charging control network 3800, including a first circuit 3802 connected with a transformer circuit 3804, in accordance with embodiments of the present disclosure. As depicted, the bidirectional charging control network 3800 may be referred to as a form of a current fed DC-DC converter.

As depicted, in the bidirectional charging control network 3800 the first circuit 3802 is connected with a battery 3810 and the transformer circuit 3804 is connected with an energy storage capacitor 3812 for providing energy for electrotherapy current for delivery to a patient. The first circuit 3802 is configured to operate in a forward mode in a buck capacity and in a reverse mode in a boost capacity. More particularly, the first circuit 3802 is configured as a synchronous converter to operate in the forward mode as a synchronous buck converter, and to operate in the reverse mode as a synchronous boost converter. In the forward mode, energy flows from the battery 3810 to the energy storage capacitor 3812 for storage by the energy storage capacitor 3812, and in the reverse mode, energy flows from the energy storage capacitor 3812 to the battery 3810 for storage by the battery 3810.

As depicted, in the first circuit 3802 includes two switches, S1 and S2, configured as a half bridge, and one inductor, L1. The first circuit 3802 is connected via intermediate nodes N1 and N2 to the transformer circuit 3804.

The transformer circuit includes an H-bridge 3806 including switches S3-S6, connected via nodes N3 and N4 to a transformer 3808. The transformer 3808 is connected via nodes N5 and N6 to a portion of the circuit 3800 including a half bridge 3814 including switches S7 and S8 as well as capacitors C1 and C2, which portion is connected via nodes N7 and N8 to the energy storage capacitor 3812.

In some embodiments, the transformer circuit 3804 uses high frequency input chopping to achieve nearly perfect DC transformer behavior. The use of the H-bridge 3806 (full bridge) can help maximize use of the transformer copper, helping minimize overall size and weight. Also, in some embodiments and circuit 3800 configurations, the transformer 3808 only requires one primary side winding, and dead time, where no current is passing through the transformer 3808, is eliminated, which characteristics can help minimize an amount of copper required.

Furthermore, the use of the half bridge 3814 can help minimize the necessary number of high voltage active components, also helping minimize size, weight and/or cost. For example, for higher voltage operation, larger, more expensive high voltage switches may be requires, as well as isolated gate drivers may be required to turn switches on and off. Each of the isolated gate drivers may, in turn, require its own power supply with transformers for isolation, which may considerably increase size, weight and/or expense of circuit 3800 components as a whole.

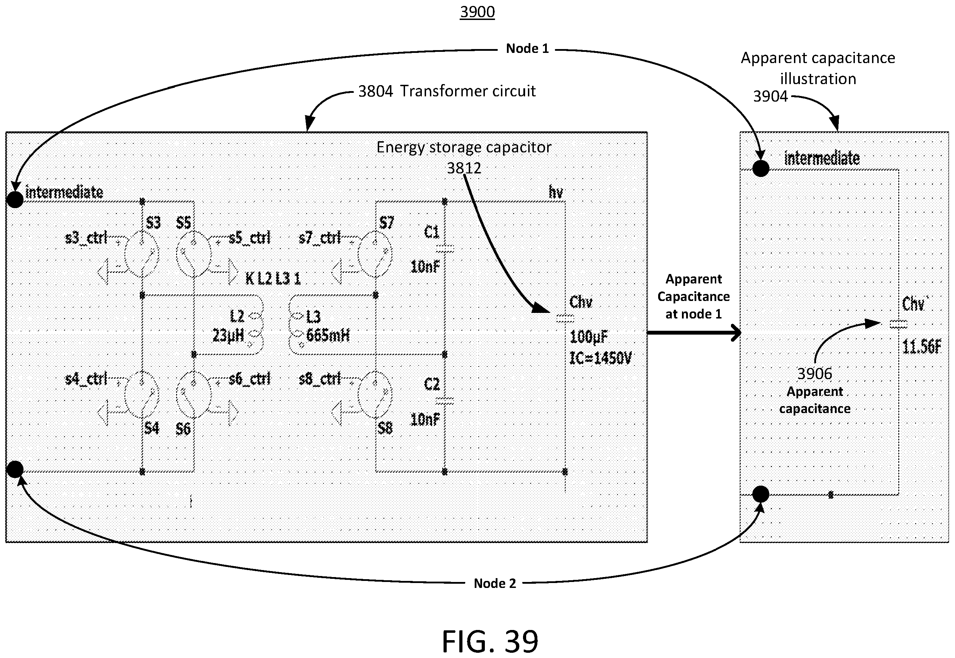

FIG. 39 is an exemplary schematic diagram 3900 of the example transformer circuit 3804 as depicted in FIG. 38, including an illustration 3904 of an apparent capacitance 3904, in accordance with embodiments of the present disclosure. Referring to FIGS. 38 and 39, at intermediate nodes N1 and N2, and therefore relative to the operation of the first circuit 3802, the transformer circuit 3804 has as simplified equivalent, or apparent, capacitance that is much greater than the capacitance of the energy storage capacitor, while also causing the apparent capacitance to appear as a high capacitance, but low voltage, capacitor. Specifically, in the depicted example, the energy storage capacitor 3812 has a capacitance of 100 microfarads, whereas the apparent capacitance is much greater, at 11.56 farads. For example, in some embodiments, the impedance transformation is a factor of (2 * Nsec/Npri) squared, where Nsec is the secondary turns count and Npri is the primary turns count of the transformer 3808. For example, the turns ratio may be 170 (or, e.g., 150-190), so the capacitance may be (2*170) squared) * 100uF = 100uF = 11.56 F. In some embodiments, lower apparent voltage across the higher apparent capacitance 3906 can result in advantages including that operation of the circuit 3800 in a buck capacity may be made more simple and efficient.

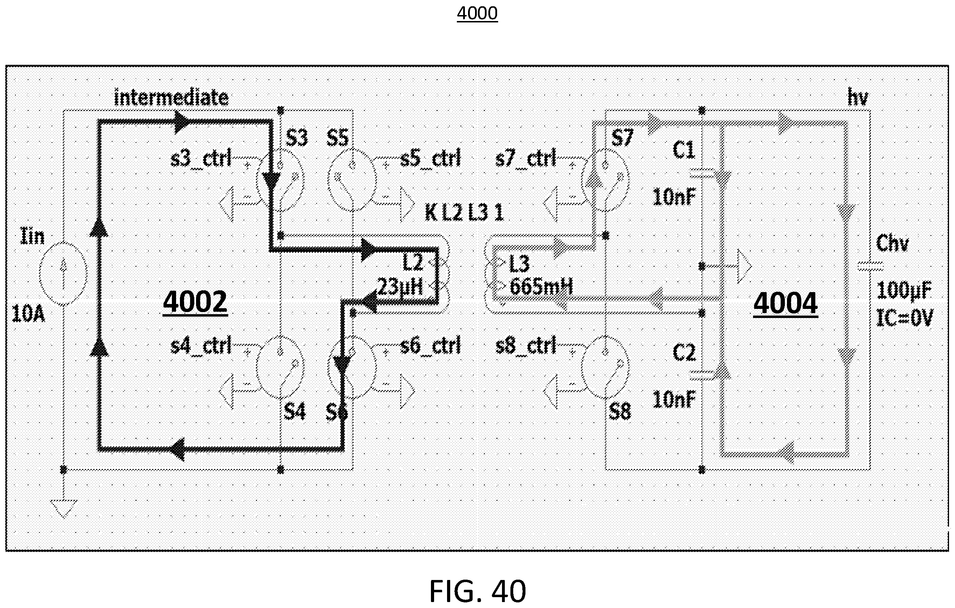

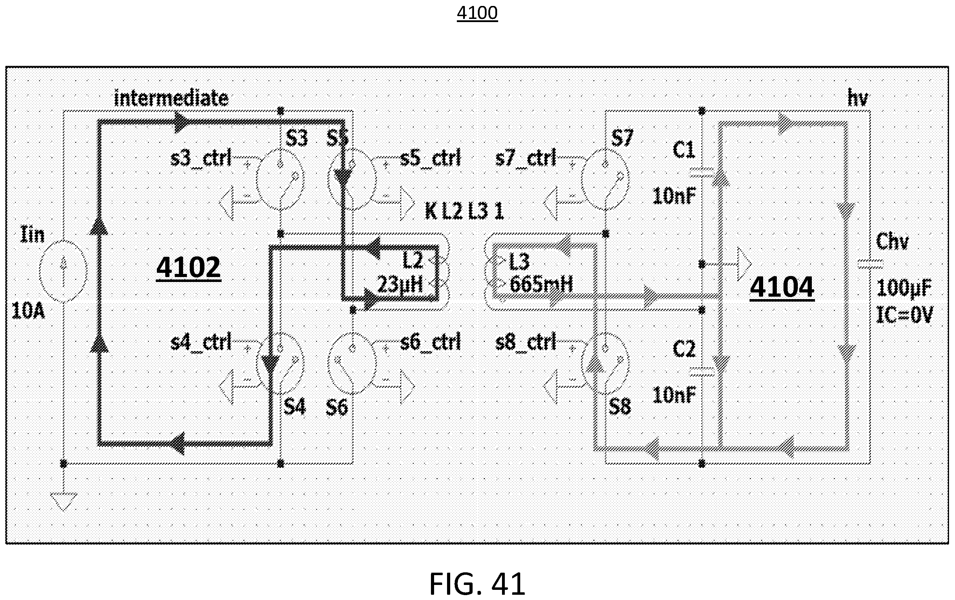

FIG. 40 is an exemplary schematic diagram of the bidirectional charging control network of FIG. 38, including an illustration of a first forward mode current flow path 4000, including subpaths 4002 and 4004, in accordance with embodiments of the present disclosure. FIG. 41 illustrates a second forward mode current flow path 4100, including subpaths 4102 and 4104. The forward mode paths 4000, 4100 result in a simple square wave drive and have a 50% duty cycle, which allows relatively simple control and operation. In particular, for example, a 50% duty cycle results in zero or substantially zero average current through the transformer 3808, which eliminates or greatly reduces field buildup, which is important for optimal transformer operation.

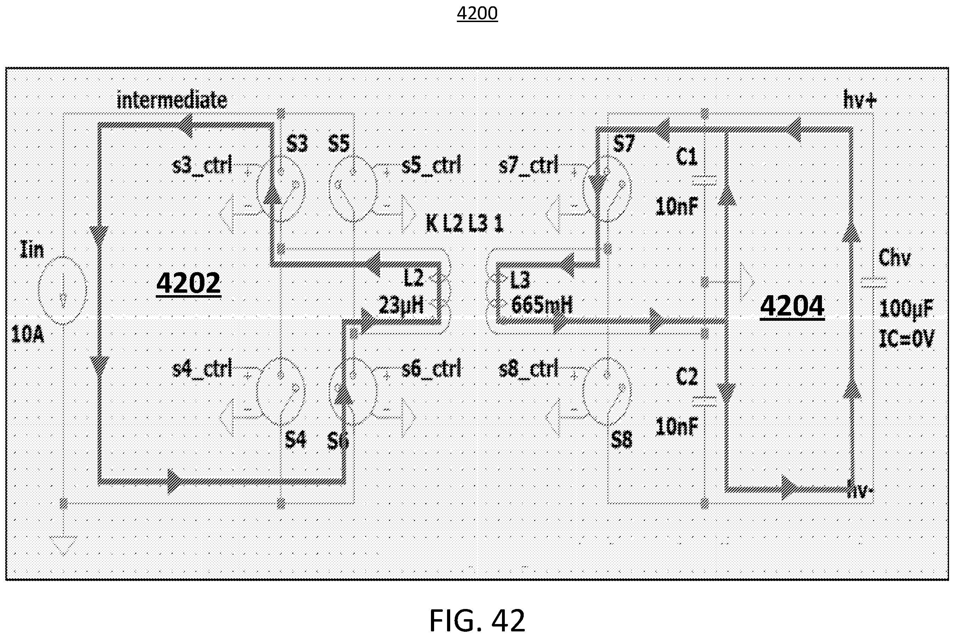

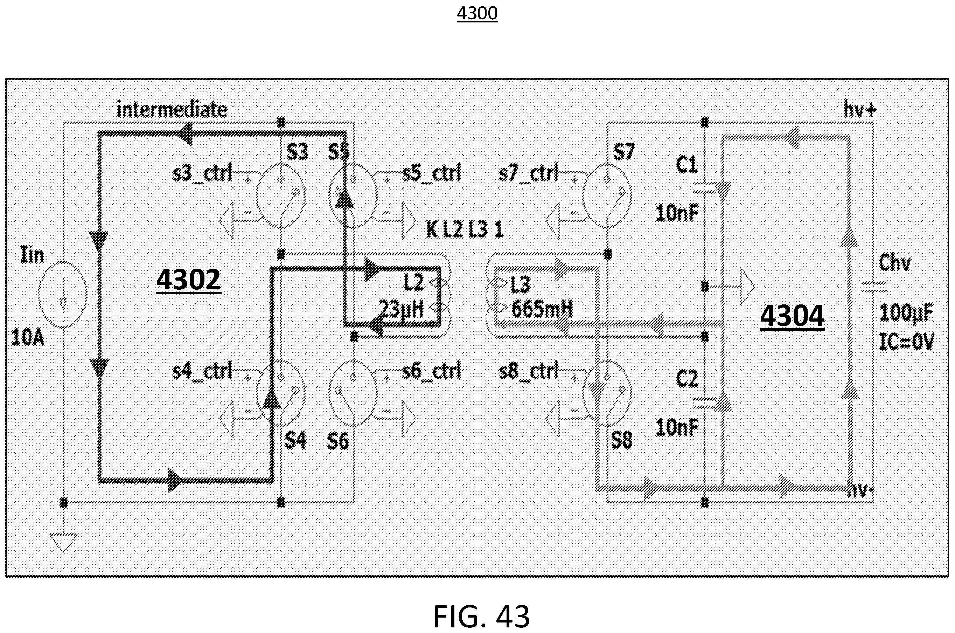

FIG. 42 is an exemplary schematic diagram of the bidirectional charging control network of FIG. 38, including an illustration of a first reverse mode current flow path 4200, including subpaths 4202 and 4204, in accordance with embodiments of the present disclosure. FIG. 43 illustrates of a second reverse mode current flow path 4300, including subpaths 4302 and 4304. Similar to the forward paths 4000, 4100, the reverse paths 4200, 4300 result in a simple square wave drive and have a 50% duty cycle, which allows relatively simple control and operation.

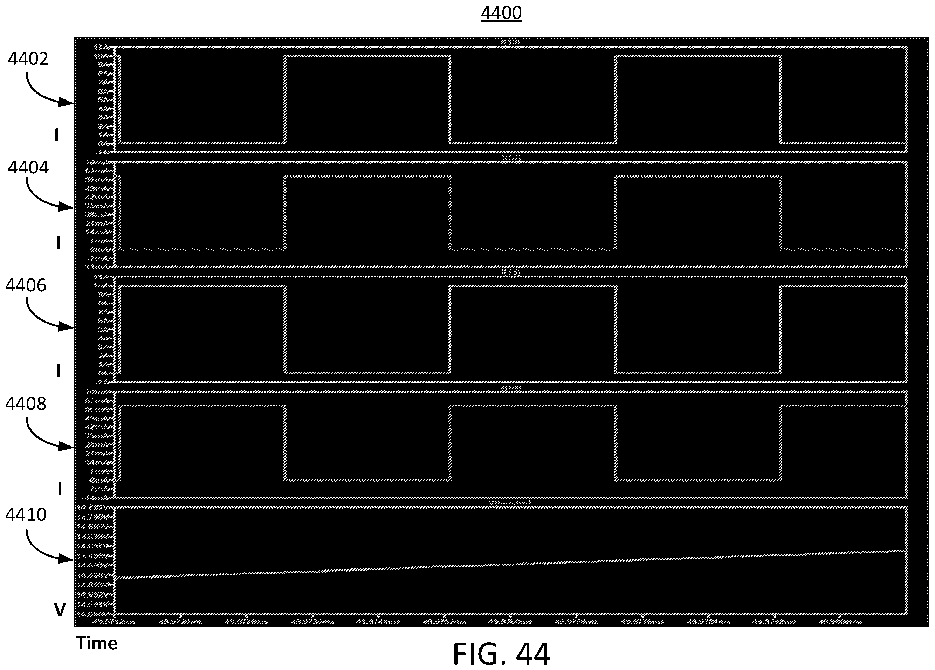

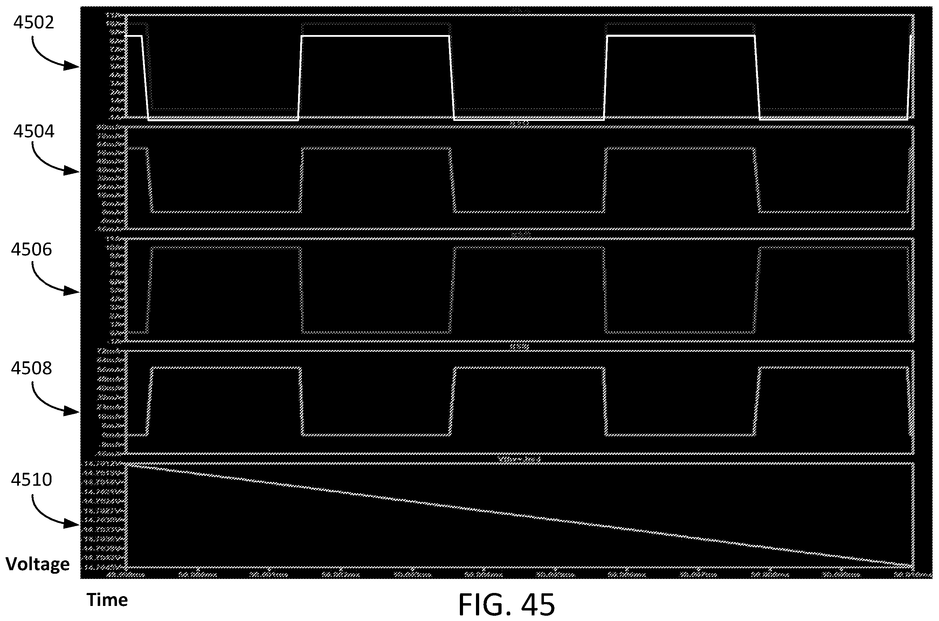

FIG. 44 includes graphical illustrations of current and voltage waveforms 4402-4410 associated with FIGS. 40-41, in accordance with embodiments of the present disclosure. FIG. 45 includes graphical illustrations of voltage waveforms 4502-4210 associated with FIGS. 42-43. More particularly, plots 4402 and 4404 show substantially square waves resulting from the first and second forward mode current flow paths 4000 and 4100, respectively. Plots 4406 and 4408 show substantially square waves resulting from the first and second reverse mode current flow paths 4200 and 4300, respectively. In FIG. 44, plot 4410, relating to operation in the forward mode, shows the steadily rising voltage on the energy storage capacitor, as energy flows from the battery 3810 to the energy storage capacitor 3812. Conversely, in FIG. 45, plot 4510, relating to operation in the reverse mode, shows the steadily falling voltage on the energy storage capacitor, as energy flows from the energy storage capacitor 3812 to the battery 3810.

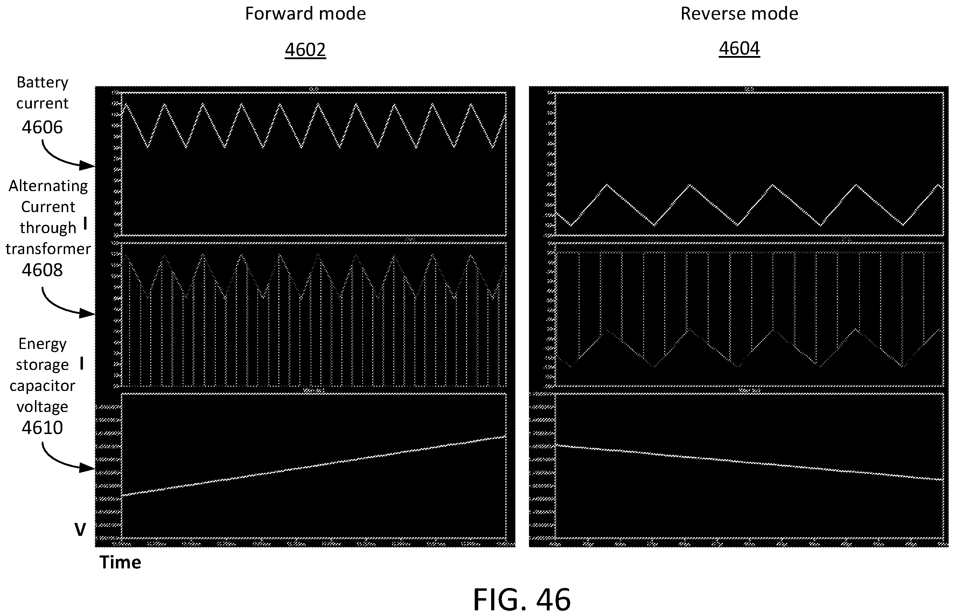

FIG. 46 includes graphical illustrations of voltage waveforms associated with the bidirectional charging control network of FIG. 38, in accordance with embodiments of the present disclosure. Particularly, plot 4606 shows the rising and falling current through the battery 3810 in forward mode 4602 and reverse mode 4604. Plot 4608 shows the alternating current through the transformer 3808 in forward mode 4602 and reverse mode 4604. Plot 4610 shows the energy storage capacitor 3812 voltage, steadily rising in forward mode 4602, as energy flow from the battery 3810 to the energy storage capacitor 3812, and steadily falling in the reverse mode 4604, as energy flow from the energy storage capacitor 3812 to the battery 3810.

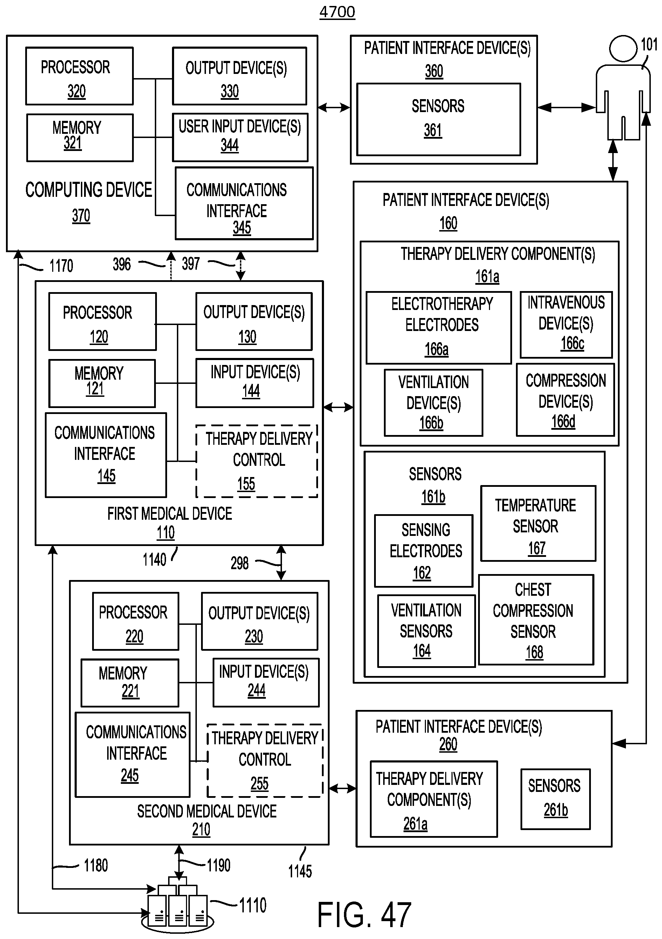

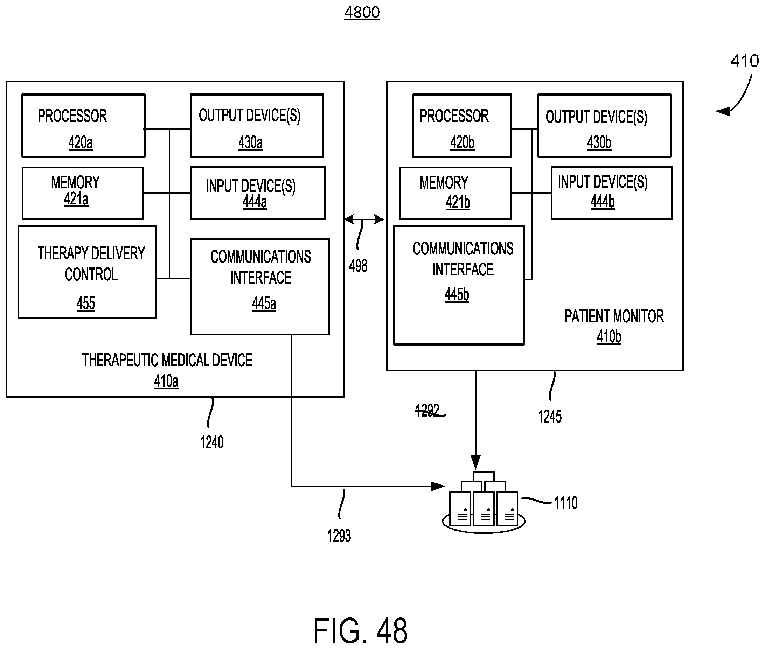

FIG. 47 is a schematic diagram illustrating components of a system 4700 of various devices including therapy delivery control elements that may incorporate aspects of embodiments of a therapeutic current control system. FIG. 48 is a schematic diagram illustrating components of a system, including a patient monitor and a therapeutic medical device including therapy deliver control elements that may incorporate aspects of embodiments of a therapeutic current control system. In FIGS. 47 and 48, medical devices can include such devices as defibrillators or pacemakers, which can incorporate embodiments described herein, such as a therapeutic current control system or a therapeutic current control network. Medical devices as shown in FIGS. 47 and 48 can further include portable, wearable or handheld varieties.

Referring to FIG. 47, examples of components of various devices discussed with regard to various figures herein, for example, are shown schematically. These devices may include medical device 110, one or more additional medical device(s) 210, one or more computing device(s) 310, and one or more server(s) 1110. In an implementation, at least one of the medical devices 110 and 210 may be a therapeutic medical device configured to deliver medical therapy to the patient and may not be limited to patient monitoring and/or diagnostic care. The computing device 370 may be adapted to function as a medical device. In an implementation, the computing device 370 may not be a therapeutic medical device configured to deliver therapy to the patient. In such an implementation, the computing device 370 may be limited to patient monitoring and/or diagnostic care.

One or more of the devices 110, 210, 310, and 1110 may be communicatively coupled via communicative couplings 298, 396, 397, 1170, 1180, and/or 1190. These communicative couplings may be each be a wired and/or a wireless communications link. The wired communications links may include a wired electrically coupling, an optical coupling via an optical cable, etc. The wireless communications link may include coupling via a radio frequency or other transmission media and/or via a network such as a local area network, an ad hoc network, a mesh network, a cellular and/or other communications network, a computer network, etc. The communications links as described herein may utilize protocols such as, for example, 802.11, ZigBee®, Bluetooth®, etc. The communications links may include near field communications which may be implemented via a communications RFID tag. The communications links may include one or more networks (e.g., the networks 101 and 111) such as a local area network, a cellular network, a satellite network, and/or a computer network (e.g., an Internet Protocol (IP) network). In various implementations, the communicative couplings described herein may provide secure and/or authenticated communications channels. In an implementation, the devices described herein may encrypt and/or decrypt the data transmitted and/or received via the communicative couplings.

In FIG. 47, the components 120, 121, 130, 144, 145, and 155 are communicatively coupled (directly and/or indirectly) to each other for bi-directional communication. Similarly, the components 220, 221, 230, 244, 245, and 255 are communicatively coupled (directly and/or indirectly) to each other for bi-directional communication and the components 320, 321, 330, 344, and 345 are communicatively coupled (directly and/or indirectly) to each other for bi-directional communication.

Although shown as separate entities in FIG. 47, the components 120, 121, 145, and/or 155 may be combined into one or more discrete components 145 and/or 155 may be part of the processor 120. The processor 120 and the memory 121 may include and/or be coupled to associated circuitry in order to perform the functions described herein. Although shown as separate entities in FIG. 47, the components 220, 221, 245, and/or 255 may be combined into one or more discrete components 245 and/or 255 may be part of the processor 220. The processor 220 and the memory 221 may include and/or be coupled to associated circuitry in order to perform the functions described herein. Although shown as separate entities in FIG. 47, the components 320, 321, and 345 may be combined into one or more discrete components and component 345 may be part of the processor 320. The processor 320 and the memory 321 may include and/or be coupled to associated circuitry in order to perform the functions described herein.

The medical devices 110 and/or 210 may include the therapy delivery control module 155 or 255. For example, the therapy delivery control module 155 and/or 255 may be an electrotherapy delivery circuit that includes one or more capacitors configured to store electrical energy for a pacing pulse or a defibrillating pulse. The electrotherapy delivery circuit may further include resistors, additional capacitors, relays and/or switches, electrical bridges such as an H-bridge (e.g., including a plurality IGBTs), voltage measuring components, and/or current measuring components. As another example, the therapy delivery control module 155 and/or 255 may be a compression device electro-mechanical controller configured to control a mechanical compression device. As a further example, the therapy delivery control module 155 and/or 255 may be an electro-mechanical controller configured to control drug delivery, temperature management, ventilation, and/or other type of therapy delivery.

The medical device 110 (e.g., a first medical device) may incorporate and/or be configured to couple to one or more patient interface device(s) 160. The patient interface device(s) 160 may include one or more therapy delivery component(s) 161a and one or more sensor(s) 161b. Similarly, the medical device 210 (e.g., a second medical device) may incorporated and/or be configured to couple to one or more patient interface device(s) 260. The patient interface device(s) 260 may include one or more therapy delivery components 261a and one or more sensors 261b. The computing device 370 may be adapted for medical use and may incorporate and/or be configured to couple to one or more patient interface device(s) 360. The patient interface device(s) 360 may include one or more sensors 361. The therapy delivery component(s) 261a may be substantially as described herein with regard to the therapy delivery component(s) 161a. Similarly, the sensor(s) 261b and 361 may be substantially as described herein with regard to the sensor(s) 161b.

The medical device 210 may receive patient data in a manner substantially similar to that described above for the medical device 110. The device 210 may receive the patient data based on signals received from the therapy delivery component(s) 261a and the sensor(s) 261b. The sensor(s) 261b may be substantially as described herein with regard to the sensor(s) 161b.

The sensor(s) 161b, 261b, and 361 may include sensing electrodes (e.g., the sensing electrodes 162), ventilation and/or respiration sensors (e.g., the ventilation and/or respiration sensors 164), temperature sensors (e.g., the temperature sensor 167), chest compression sensors (e.g., the chest compression sensor 168), etc. For example, the sensing electrodes may include cardiac sensing electrodes. The cardiac sensing electrodes may be conductive and/or capacitive electrodes configured to measure changes in a patient's electrophysiology to measure the patient's ECG information. The sensing electrodes may further measure the transthoracic impedance and/or a heart rate of the patient. The ventilation and/or respiration sensors may include spirometry sensors, flow sensors, pressure sensors, oxygen and/or carbon dioxide sensors such as, for example, one or more of pulse oximetry sensors, oxygenation sensors (e.g., muscle oxygenation/pH), O2 gas sensors and capnography sensors, impedance sensors, and combinations thereof. The temperature sensors may include an infrared thermometer, a contact thermometer, a remote thermometer, a liquid crystal thermometer, a thermocouple, a thermistor, etc. and may measure patient temperature internally and/or externally. The chest compression sensor may include one or more motion sensors including, for example, one or more accelerometers, one or more force sensors, one or more magnetic sensors, one or more velocity sensors, one or more displacement sensors, etc. The chest compression sensor may provide one or more signals indicative of the chest motion to the medical device 110 and/or 210 via a wired and/or wireless connection. The chest compression sensor may be, for example, but not limited to, a compression puck, a smart-phone, a hand-held device, a wearable device, etc. The chest compression sensor may be configured to detect chest motion imparted by a rescuer and/or an automated chest compression device (e.g., a belt system, a piston system, etc.). The chest compression sensor may provide signals indicative of chest compression data including displacement data, velocity data, release velocity data, acceleration data, force data, compression rate data, dwell time data, hold time data, blood flow data, blood pressure data, etc. In an implementation, the defibrillation and/or pacing electrodes may include or be configured to couple to the chest compression sensor.

**In** various implementations, the sensor(s) 161b, 261b and/or 361 may include one or more sensor devices configured to provide sensor data that includes, for example, but not limited to electrocardiogram (ECG), blood pressure, heart rate, respiration rate, heart sounds, lung sounds, respiration sounds, end tidal $CO_2$, saturation of muscle oxygen ($SMO_2$), oxygen saturation (e.g., $SpO_2$ and/or $PaO_2$), cerebral blood flow, point of care laboratory measurements (e.g., lactate, glucose, etc.), temperature, electroencephalogram (EEG) signals, brain oxygen level, tissue pH, tissue fluid levels, images and/or videos via ultrasound, laryngoscopy, and/or other medical imaging techniques, near-infrared reflectance spectroscopy, pneumography, cardiography, and/or patient movement. Images and/or videos may be two-dimensional or three-dimensional.

The one or more therapy delivery components 161a and 261a may include electrotherapy electrodes (e.g., the electrotherapy electrodes 166a), ventilation device(s) (e.g., the ventilation devices 166b), intravenous device(s) (e.g., the intravenous devices 166c), compression device(s) (e.g., the compression devices 166d), etc. For example, the electrotherapy electrodes may include defibrillation electrodes, pacing electrodes, and combinations thereof. The ventilation devices may include a tube, a mask, an abdominal and/or chest compressor (e.g., a belt, a cuirass, etc.), etc. and combinations thereof. The intravenous devices may include drug delivery devices, fluid delivery devices, and combinations thereof. The compression devices may include mechanical compression devices such as abdominal compressors, chest compressors, belts, pistons, and combinations thereof. **In** various implementation, the therapy delivery component(s) 161a and/or 261a may be configured to provide sensor data and/or be coupled to and/or incorporate sensors. For example, the electrotherapy electrodes may provide sensor data such as transthoracic impedance, ECG, heart rate, etc. Further the electrotherapy electrodes may include and or be coupled to a chest compression sensor. As another example, the ventilation devices may be coupled to and/or incorporate flow sensors, gas species sensors (e.g., oxygen sensor, carbon dioxide sensor, etc.), etc. As a further example, the intravenous devices may be coupled to and/or incorporate temperature sensors, flow sensors, blood pressure sensors, etc. As yet another example, the compression devices may be coupled to and/or incorporate chest compression sensors, patient position sensors, etc. The therapy delivery control modules 155 and 255 may be configured to couple to and control the therapy delivery component(s) 161a and 261a, respectively.

The one or more sensor(s) 161b, 261b, and 361 and/or the therapy delivery component(s) 161a and 261a may provide sensor data. The patient data provided at the operational interface and/or playback interface may include the sensor data. For example, the medical device 110 (e.g., the first medical device) may process signals received from the sensor(s) 161b and/or the therapy delivery component(s) 161a to determine the sensor data. Similarly, the medical device 210 may process signals received from the sensor(s) 261b and/or the therapy delivery component(s) 261a to determine the sensor data and the computing device 370 may process signals received from the sensor(s) 361 to determine the sensor data.

Referring to FIG. 48, examples 4800 of components of the modular therapeutic medical device/patient monitor are shown. The modular therapeutic medical device/patient monitor may include a therapeutic medical device and patient monitor configured to communicatively couple to one another. For example the therapeutic medical device may be a defibrillator and the modular therapeutic medical device/patient monitor may include a defibrillator and patient monitor configured to communicatively couple to one another Although shown as separate entities in FIG. 48, the components 420a, 421a, 445a, and/or 455 may be combined into one or more discrete components 445a and/or 455 may be part of the processor 420a. Similarly, although shown as separate entities in FIG. 12, the components 420b, 421b, and/or 445b may be combined into one or more discrete components and component 445b may be part of the processor 420b. The processor 420a and the memory 421a may include and/or be coupled to associated circuitry in order to perform the functions described herein. Similarly, the processor 420b and the memory 421b may include and/or be coupled to associated circuitry in order to perform the functions described herein. The components 420a, 421a, 430a, 444a, 445a, and 455 are communicatively coupled (directly and/or indirectly) to each other for bi-directional communication. Similarly, the components 420b, 421b, 430b, 444b, and 445b are communicatively coupled (directly and/or indirectly) to each other for bi-directional communication

The therapy delivery control module 455 may be an electrotherapy delivery circuit substantially as described with regard to the therapy delivery control modules 155 and 255. As another example, the therapy delivery control module 455 may be a compression device electro-mechanical controller configured to control a mechanical compression device. As a further example, the therapy delivery control module 455 may be an electro-mechanical controller configured to control drug delivery, temperature management, ventilation, and/or other type of therapy delivery.

Referring to FIGS. 38 and 39, the medical device 110 may include a processor 120, a memory 121, one or more output devices 130, one or more user input devices 144, and a communications interface 145. The medical device 210 may include a processor 220, a memory 221, one or more output devices 230, one or more user input devices 244, and a communications interface 245. The computing device 370 may include a processor 320, a memory 321, one or more output devices 330, one or more user input devices 344, and a communications interface 345. The therapeutic medical device 410a may include a processor 420a, a memory 421a, one or more output devices 430a, one or more input devices 444a, and a communications interface 445a. The patient monitor 410b may include a processor 420b, a memory 421b, one or more output devices 430b, one or more input devices 444b, and a communications interface 445b.

The processors 120, 220, 320, 420a, and 420b are physical processors (i.e., an integrated circuit configured to execute operations on the devices 110, 210, 310, 410a, and 410b, respectively, as specified by software and/or firmware stored in a computer storage medium). The processors 120, 220, 320, 420a, and 420b are operably coupled, respectively, to the memory 121, the memory 221, the memory 321, the memory 421a, and the memory 421b. The processors 120, 220, 320, 420a, and 420b may be intelligent hardware devices (for example, but not limited to, a central processing unit (CPU), a graphics processing unit (GPU), one or more microprocessors, a controller or microcontroller, an application specific integrated circuit (ASIC), a digital signal processor (DSP), etc.) designed to perform the functions described herein and operable to carry out instructions on the devices 110, 210, 310, 410a, and 410b, respectively. Each of the processors 120, 220, 320, 420a, and 420b may be one or more processors and may be implemented as a combination of hardware devices (e.g., a combination of DSP and a microprocessor, a plurality of microprocessors, one or more microprocessors in conjunction with a DSP core, or another such configuration). Each of the processors 120, 220, 320, 420a, and 420b may include multiple separate physical entities that may be distributed in the devices 110, 210, 310, 410a, and 410b respectively. Each of the processors 120, 220, 320, 420a, and 420b is configured to execute processor-readable, processor-executable software code containing one or more instructions or code for controlling the processors 120, 220, 320, 420a, and 420b to perform the functions as described herein. The processors 120, 220, 320, 420a, and/or 420b may utilize various architectures including but not limited to a complex instruction set computer (CISC) processor, a reduced instruction set computer (RISC) processor, or a minimal instruction set computer (MISC). In various implementations, the processors 120, 220, 320, 420a and/or 420b may be a single-threaded or a multi-threaded processor. The processors 120, 220, 320, 420a, and/or 420b may be, for example, an Intel® Itanium® or Itanium 2® processor(s), AMD® Opteron®, Athlon MP® processor(s), a Motorola ® line of processor, or an ARM, Intel Pentium Mobile, Intel Core i5 Mobile, AMD A6 Series, AMD Phenom II Quad Core Mobile, or like devices.

The memories 121, 221, 321, 421a, and 421b refer generally to a computer storage medium, including but not limited to RAM, ROM, FLASH, disc drives, fuse devices, and portable storage media, such as Universal Serial Bus (USB) flash drives, etc. Each of the memories 121, 221, 321, 421a, and 421b may include, for example, random access memory (RAM), or another dynamic storage device(s) and may include read only memory (ROM) or another static storage device(s) such as programmable read only memory (PROM) chips for storing static information such as instructions for a coupled processor (e.g., one of the processors 120, 220, 320, 420a, and 420b). The memories 121, 221, 321, 421a, and 421b may include USB flash drives that may store operating systems and other applications. The USB flash drives may include input/output components, such as a wireless transmitter and/or USB connector that can be inserted into a USB port of another computing device. The memories 121, 221, 321, 421a, and/or 421b may be long term, short term, or other memory associated with the respective device 110, 210, 310, 410a, and 410b and are not to be limited to a particular type of memory or number of memories, or type of media upon which memory is stored. The memories 121, 221, 321, 421a, and/or 421b include a non-transitory processor-readable storage medium (or media) that stores the processor-readable, processor-executable software code. The memories 121, 221, 321, 421a, and/or 421b may store information and instructions. For example, the memories 121, 221, 321, 421a, and/or 421b may include flash memory and/or another storage media may be used, including removable or dedicated memory in a mobile or portable device. As another example, hard disks such as the Adaptec® family of SCSI drives, an optical disc, an array of disks such as RAID (e.g. the Adaptec family of RAID drives), or another mass storage devices may be used. The memories 121, 221, 321, 421a, and/or 421b may include removable storage media such as, for example, external hard-drives, floppy drives, flash drives, zip drives, compact disc - read only memory (CD-ROM), compact disc - rewritable (CD-RW), or digital video disk - read only memory (DVD-ROM).

The communications interfaces 145, 245, 345, 445a, and 445b may transmit and/or receive information to and/or from one or more devices external to and communicatively coupled to the devices 110, 210, 310, 410a, and 410b, respectively. The communications interfaces 145, 245, 345, 445a, and 445b may transmit and/or receive the information via a wired and/or wireless communicative coupling (e.g., the couplings 298, 396, 397, 398a, 398b, 498, 1170, 1180, 1190, 1292, or 1293). The information may include information stored in at least one of the memories 121, 221, 321, 421a, and 421b. The information may include, for example, but not limited to, resuscitative treatment information, physiological information, patient information, rescuer and/or caregiver information, location information, rescue and/or medical treatment center information, etc. The communications interfaces 145, 245, 345, 445a, and/or 445b may enable short range and/or long range wireless communications capabilities which may include communications via near field communications, ZigBee®, Wi-Fi, Bluetooth®, satellite(s), radio waves, a computer network (e.g., the Internet), a cellular network, etc. The communications interfaces 145, 245, 345, 445a, and/or 445b may enable communications via a network such a Local Area Network (LAN), Wide Area Network (WAN), a mesh network, an ad hoc network, or another network. The communications interfaces 145, 245, 345, 445a, and/or 445b may include, for example, an RS-232 port for use with a modem based dialup connection, a copper or fiber 10/100/1000 Ethernet port, or a Bluetooth® or WiFi interface.

In an implementation, the communications interfaces 145, 245, 345, 445a, and/or 445b may enable communication between one or more of the devices 110, 210, 310, 410a, and 410b with one or more servers 1110. For example, the one or more servers 1110 may be remote servers and may include a cloud server and/or a central facility server. In an implementation, the one or more servers 1110 may be associated with a medical provider (e.g., a hospital, a physician's office, a medical records office, an emergency services office, an emergency services vehicle, a dispatch center, etc.). In an implementation, the communications interface 445b may enable the patient monitor 410b to communicatively couple with multiple therapeutic medical device(s) 410a and/or with another patient monitor. In an implementation, the therapeutic medical device 410a may communicatively couple with the one or more servers 1110 via the patient monitor 410b. For example, the communications interface 445a may provide patient data and/or other information from the therapeutic medical device 410a to the patient monitor 410b via the communicative coupling 498. The patient monitor 410b may merge the received patient data and/or other information with patient data and/or other information collected by and/or generated at the patient monitor 410b to create an integrated record. The patient monitor 410b may provide the integrated record to the one or more servers 1110 via the communications interface 445b and the communicative coupling 1292. Alternatively or additionally, the therapeutic medical device 410a may provide patient data and/or other information to the one or more servers 1110 via the communications interface 445a and the communicative coupling 1293. One or more of the communications interfaces 145, 245, 345, 445a, and 445b in combination with one or more of the communicative couplings 298, 396, 397, 398a, 398b, 498, 1170, 1180, 1190, 1292, and 1293 may enable telemedicine communications and data sharing of real-time and/or historical patient data in support of the telemedicine communications.

The output device(s) 130 and user input device(s) 144 may be included in the medical device 110 and/or coupled to the medical device 110. Similarly, the output device(s) 230 and the user input device(s) 244 may be included in the medical device 210 and/or coupled to the medical device 210, the output device(s) 330 and the user input device(s) 344 may be included in the computing device 370 and/or coupled to the computing device 370, the output device(s) 430a and the user input device(s) 444a may be included in the therapeutic medical device 410a and/or coupled to the therapeutic medical device 410a, and the output device(s) 430b and the user input device(s) 444b may be included in the patient monitor 410b and/or coupled to the patient monitor 410b. For example, the output device(s) 130, 230, 330, 430a, and/or 430b may include one or more of a display (e.g., the displays 115, 215, 315, 415a, and 415b), a speaker, and a haptic device. The display may provide a graphical user interface (GUI). The display may be, for example, but not limited to, a liquid crystal display (LCD) and/or a light emitting diode (LED) display. In an implementation, the output device(s) 130, 230, 330, 430a, and/or 430b may be input/output device(s) capable of capturing user input. For example, the display may be a touchscreen. The touchscreen may be, for example, a pressure sensitive touchscreen or a capacitive touchscreen. The touchscreen may capture user input provided via touchscreen gestures and/or provided via exertions of pressure on a particular area of the screen. Examples of touchscreen gestures provided herein with regard to user input may include pushing on the touchscreen to exert pressure that exceeds a particular threshold to indicate an input to a pressure sensitive touchscreen by the user. The touchscreen and the controlling processor (e.g., 120, 220, 320, 420a, and/or 420b) may be configured recognize touchscreen gestures including, for example, but not limited to, tap, double tap, caliper gesture, drag and drop, slide, press and drag, hold and press, etc. In an implementation, the processors 120, 220, 320, 420a, and/or 420b may control a respective display to provide visual representations of data captured by and/or received at the device 110, 210, 310, 410a, and/or 410b. The visual representations may include still images and/or video images (e.g., animated images).

In an implementation, the output device(s) 130, 230, 330, 430a, and 430b and/or the input device(s) 144, 244, 344, 444a, and 444b may include wearable devices such as, for example, a heads-up display mounted onto eyeglasses, a face shield, a watch, and/or devices that may be integrated with other wearable communications devices, such as, for example, an ear bud or a Bluetooth® hands free phone adaptor. The processors 120, 220, 320, 420a, and 420b may control the output devices 130, 230, 330, 430a, and 430b respectively, to provide information for the user. The information may include feedback (e.g., visible feedback, audible feedback, haptic feedback, numerical feedback, and graphical feedback) such as CPR feedback.

The one or more user input devices 144, 244, 344, 444a, and 444b may include, for example, a keyboard, a mouse, joystick, trackball, or other pointing device, a microphone, a camera, etc. Further, the user input devices 144, 244, 344, 444a, and 444b may be a touchscreen and/or another input/output device capable of providing information for the user and capturing information from the user. The touchscreen may be a pressure sensitive touchscreen

In an implementation, the user input devices 144, 244, 344, 444a, and/or 444b are configured to capture information, such as, for example, patient medical history (e.g., medical record information including age, gender, weight, body mass index, family history of heart disease, cardiac diagnosis, co-morbidity, left ventricular ejection fraction, medications, previous medical treatments, and/or other physiological information), physical examination results, patient identification, caregiver identification, healthcare facility information, etc.

The processor, memory, communications, and input and/or output components described above are meant to exemplify some types of possibilities. In no way should the aforementioned examples limit the scope of the disclosure, as they are only exemplary embodiments of these components.

Various modifications and additions can be made to the exemplary embodiments discussed without departing from the scope of the invention as defined by the following claims.

**Claims**

1. A system (103, 113, 200, 250, 275, 300, 1200, 1400) for generating an electrotherapeutic pulse to be delivered to a patient, the system comprising:

   an energy storage capacitor (223, 302, 352, 382, 1004, 1012, 1208, 1406, 1702, 1904, 2018, 3812) for providing electrotherapeutic current to the patient;
   a therapeutic current control network (202, 286, 238, 303, 351, 381, 1202), electrically coupled to the energy storage capacitor, for controlling the electrotherapeutic current to be delivered to the patient, the therapeutic current control network comprising a resonant electrical circuit (283) and at least one current control switch (203, 224, 282, 1214, 1710), wherein energy provided by the energy storage capacitor flows through the resonant electrical circuit; and
   a controller (212, 233, 285, 313, 1220) electrically coupled to the energy storage capacitor and the therapeutic current control network, wherein the controller is configured to, in connection with the energy provided by the energy storage capacitor that flows through the resonant electrical circuit, control operation of the at least one current control switch of the therapeutic current control network in delivering an electrotherapeutic waveform to the patient to correspond with a specified waveform.

2. The system (103, 113, 200, 250, 275, 300, 1200, 1400) of claim 1, comprising at least one sensor configured to sense at least one electrical parameter from which current flow to the patient can be determined or estimated, wherein the controller (212, 233, 285, 313, 1220) is configured to:

   process a signal associated with the sensed at least one electrical parameter;
   compare the processed signal with a second signal associated with the specified waveform; and
   control operation of the at least one current control switch (203, 224, 282, 1214, 1710) of the therapeutic current control network (202, 286, 238, 303, 351, 381, 1202) in adjusting delivery of the electrotherapeutic waveform to the patient to correspond with the specified waveform.

3. The system (103, 113, 200, 250, 275, 300, 1200, 1400) of claim 1 or 2, wherein the at least one current control switch (203, 224, 282, 1214, 1710) comprises a plurality of switches, and wherein the control of the operation of the at least one current control switch of the therapeutic current control network (202, 286, 238, 303, 351, 381, 1202) in the adjusting of the delivery of the electrotherapeutic waveform to the patient to correspond with the specified waveform comprises controlling a configuration of each of the plurality of switches as open or closed.

4. The system (103, 113, 200, 250, 275, 300, 1200, 1400) of claim 1 or 2, comprising a battery (215, 301, 1002, 1008, 1022, 1902, 3810) and a bidirectional charging control network (216, 1010, 1020), wherein:

   the battery and the bidirectional charging control network are connected via a first node and a second node, and the bidirectional charging control network and the energy storage capacitor (223, 302, 352, 382, 1004, 1012, 1208, 1406, 1702, 1904, 2018, 3812) are connected via a third node and a fourth node, wherein:
   the bidirectional charging control network is for controlling energy flow from the battery to the energy storage capacitor for storage by the energy storage capacitor, and from the energy storage capacitor to the battery for storage by the battery.

5. The system (103, 113, 200, 250, 275, 300, 1200, 1400) of claim 4, wherein the bidirectional charging control network comprises a second resonant electrical circuit (283) comprising a resonant tank.

6. The system (103, 113, 200, 250, 275, 300, 1200, 1400) of claim 1 or 2, wherein the controller (212, 233, 285, 313, 1220) is configured to control operation of the at least one current control switch (203, 224, 282, 1214, 1710) of the therapeutic current control network (202, 286, 238, 303, 351, 381, 1202) to stagger a time of initiation of current switching relative to a time of initiation of voltage switching, the voltage switching being associated with the current switching, so as to reduce switching losses relative to substantially simultaneous initiation of the current switching and the associated voltage switching.

7. The system (103, 113, 200, 250, 275, 300, 1200, 1400) of claim 1 or 2, wherein the controller (212, 233, 285, 313, 1220) is configured to control operation of the at least one current control switch to provide substantially zero voltage switching (ZVS) and substantially zero current switching (ZCS).

8. The system (103, 113, 200, 250, 275, 300, 1200, 1400) of claim 1 or 2, wherein the specified waveform is a biphasic rectilinear current waveform, and the system comprises at least one polarity control switch, wherein the at least one polarity control switch is used in controlling direction of current flow in delivering the biphasic rectilinear current waveform to the patient.

9. The system (103, 113, 200, 250, 275, 300, 1200, 1400) of claim 1 or 2, wherein the resonant electrical circuit (283) is a two element resonant tank.

10. The system (103, 113, 200, 250, 275, 300, 1200, 1400) of claim 9, wherein the two element resonant tank comprises an inductor and a capacitor.

11. The system (103, 113, 200, 250, 275, 300, 1200, 1400) of claim 1 or 2, wherein the resonant electrical circuit (283) is a three element resonant tank.

12. The system (103, 113, 200, 250, 275, 300, 1200, 1400) of claim 1 or 2, wherein the resonant electrical circuit (283) is a four element resonant tank.

13. The system (103, 113, 200, 250, 275, 300, 1200, 1400) of claim 1 or 2, wherein the resonant electrical circuit (283) is at least one of: an LCC resonant tank, a CLL resonant tank, an LLC resonant tank and an LCLC resonant tank.

14. The system (103, 113, 200, 250, 275, 300, 1200, 1400) of claim 1 or 2, wherein the electrotherapeutic waveform is for defibrillation.

15. The system (103, 113, 200, 250, 275, 300, 1200, 1400) of claim 1 or 2, wherein the control of the operation of the at least one current control switch (203, 224, 282, 1214, 1710) of the therapeutic current control network (202, 286, 238, 303, 351, 381, 1202) in the adjusting of the delivery of the electrotherapeutic waveform to the patient to correspond with the specified waveform comprises controlling a switching frequency of the at least one current control switch.

**Patentansprüche**

1. System (103, 113, 200, 250, 275, 300, 1200, 1400) zum Erzeugen eines elektrotherapeutischen Impulses, der an einen Patienten abzugeben ist, das System umfassend:

einen Energiespeicherkondensator (223, 302, 352, 382, 1004, 1012, 1208, 1406, 1702, 1904, 2018, 3812) zum Bereitstellen von elektrotherapeutischem Strom für den Patienten;
ein therapeutisches Stromsteuerungsnetzwerk (202, 286, 238, 303, 351, 381, 1202), das elektrisch mit dem Energiespeicherkondensator gekoppelt ist, um den an den Patienten abzugebenden elektrotherapeutischen Strom zu steuern, wobei das therapeutische Stromsteuerungsnetzwerk eine elektrische Resonanzschaltung (283) und mindestens einen Stromsteuerungsschalter (203, 224, 282, 1214, 1710) umfasst, wobei von dem Energiespeicherkondensator bereitgestellte Energie durch die elektrische Resonanzschaltung fließt; und
eine Steuerung (212, 233, 285, 313, 1220), die elektrisch mit dem Energiespeicherkondensator und dem therapeutischen Stromsteuerungsnetzwerk gekoppelt ist, wobei die Steuerung konfiguriert ist, um in Verbindung mit der von dem Energiespeicherkondensator bereitgestellten Energie, die durch die elektrische Resonanzschaltung fließt, den Betrieb des mindestens einen Stromsteuerungsschalters des therapeutischen Stromsteuerungsnetzwerks bei der Abgabe einer elektrotherapeutischen Wellenform an den Patienten zu steuern, die einer bestimmten Wellenform entspricht.

2. System (103, 113, 200, 250, 275, 300, 1200, 1400) nach Anspruch 1, umfassend mindestens einen Sensor, der konfiguriert ist, um mindestens einen elektrischen Parameter zu erfassen, aus dem der Stromfluss zum Patienten bestimmt oder geschätzt werden kann, wobei die Steuerung (212, 233, 285, 313, 1220) konfiguriert ist zum:

Verarbeiten eines Signals, das mit dem mindestens einen erfassten elektrischen Parameter verbunden ist;
Vergleichen des verarbeiteten Signals mit einem zweiten Signal, das mit der spezifizierten Wellenform verbunden ist; und
Steuern des Betriebs des mindestens einen Stromsteuerungsschalters (203, 224, 282, 1214, 1710) des therapeutischen Stromsteuerungsnetzwerks (202, 286, 238, 303, 351, 381, 1202), um die Abgabe der elektrotherapeutischen Wellenform an den Patienten einzustellen, damit sie der vorgegebenen Wellenform entspricht.

3. System (103, 113, 200, 250, 275, 300, 1200, 1400) nach Anspruch 1 oder 2, wobei der mindestens eine Stromsteuerschalter (203, 224, 282, 1214, 1710) eine Vielzahl von Schaltern umfasst, und wobei die Steuerung des Betriebs des mindestens einen Stromsteuerschalters des therapeutischen Stromsteuerungsnetzwerks (202, 286, 238, 303, 351, 381, 1202) bei der Einstellung der Abgabe der elektrotherapeutischen Wellenform an den Patienten, um der spezifizierten Wellenform zu entsprechen, das Steuern einer Konfiguration jedes der Vielzahl von Schaltern als offen oder geschlossen umfasst.

4. System (103, 113, 200, 250, 275, 300, 1200, 1400) nach Anspruch 1 oder 2, umfassend eine Batterie (215, 301, 1002, 1008, 1022, 1902, 3810) und ein bidirektionales Ladesteuernetzwerk (216, 1010, 1020), wobei:

die Batterie und das bidirektionale Ladesteuernetzwerk über einen ersten Knoten und einen zweiten Knoten verbunden sind, und
das bidirektionale Ladesteuernetzwerk und der Energiespeicherkondensator (223, 302, 352, 382, 1004, 1012, 1208, 1406, 1702, 1904, 2018, 3812) über einen dritten Knoten und einen vierten Knoten verbunden sind, wobei:
das bidirektionale Ladesteuernetzwerk zur Steuerung des Energieflusses von der Batterie zum Energiespeicherkondensator zur Speicherung durch den Energiespeicherkondensator und vom Energiespeicherkondensator zur Batterie zur Speicherung durch die Batterie dient.

5. System (103, 113, 200, 250, 275, 300, 1200, 1400) nach Anspruch 4, wobei das bidirektionale Ladesteuernetzwerk eine zweite elektrische Resonanzschaltung (283) umfasst, die einen Resonanztank enthält.

6. System (103, 113, 200, 250, 275, 300, 1200, 1400) nach Anspruch 1 oder 2, wobei die Steuerung (212, 233, 285, 313, 1220) konfiguriert ist, um den Betrieb des mindestens einen Stromsteuerungsschalters (203, 224, 282, 1214, 1710) des therapeutischen Stromsteuerungsnetzwerks (202, 286, 238, 303, 351, 381, 1202) zu steuern, um einen Zeitpunkt der Auslösung des Stromschaltens relativ zu einem Zeitpunkt der Auslösung des Spannungsschaltens zu staffeln, wobei das Spannungsschalten mit dem Stromschalten verbunden ist, um Schaltverluste relativ zu einer im Wesentlichen gleichzeitigen Auslösung des Stromschaltens und des zugehörigen Spannungsschaltens zu reduzieren.

7. System (103, 113, 200, 250, 275, 300, 1200, 1400) nach Anspruch 1 oder 2, wobei die Steuerung (212, 233, 285, 313, 1220) konfiguriert ist, um den Betrieb des mindestens einen Stromsteuerschalters zu steuern, um im Wesentlichen spannungsloses Schalten (ZVS) und im Wesentlichen stromloses Schalten (ZCS) bereitzustellen.

8. System (103, 113, 200, 250, 275, 300, 1200, 1400) nach Anspruch 1 oder 2, wobei die spezifizierte Wellenform eine biphasische geradlinige Stromwellenform ist und das System mindestens einen Polaritätssteuerschalter umfasst, wobei der mindestens eine Polaritätssteuerschalter zum Steuern der Richtung des Stromflusses bei der Abgabe der biphasischen geradlinigen Stromwellenform an den Patienten verwendet wird.

9. System (103, 113, 200, 250, 275, 300, 1200, 1400) nach Anspruch 1 oder 2, wobei die elektrische Resonanzschaltung (283) ein Zweielementresonanztank ist.

10. System (103, 113, 200, 250, 275, 300, 1200, 1400) nach Anspruch 9, wobei der Zweielementresonanztank eine Induktivität und einen Kondensator umfasst.

11. System (103, 113, 200, 250, 275, 300, 1200, 1400) nach Anspruch 1 oder 2, wobei die elektrische Resonanzschaltung (283) ein Dreielementresonanztank ist.

12. System (103, 113, 200, 250, 275, 300, 1200, 1400) nach Anspruch 1 oder 2, wobei die elektrische Resonanzschaltung (283) ein Vierelementresonanztank ist.

**13.** System (103, 113, 200, 250, 275, 300, 1200, 1400) nach Anspruch 1 oder 2, wobei die elektrische Resonanzschaltung (283) mindestens eines von Folgendem ist: ein LCC-Resonanztank, ein CLL-Resonanztank, ein LLC-Resonanztank und ein LCLC-Resonanztank.

**14.** System (103, 113, 200, 250, 275, 300, 1200, 1400) nach Anspruch 1 oder 2, wobei die elektrotherapeutische Wellenform zur Defibrillation dient.

**15.** System (103, 113, 200, 250, 275, 300, 1200, 1400) nach Anspruch 1 oder 2, wobei die Steuerung des Betriebs des mindestens einen Stromsteuerungsschalters (203, 224, 282, 1214, 1710) des therapeutischen Stromsteuerungsnetzwerks (202, 286, 238, 303, 351, 381, 1202) bei der Einstellung der Abgabe der elektrotherapeutischen Wellenform an den Patienten, um der spezifizierten Wellenform zu entsprechen, die Steuerung einer Schaltfrequenz des mindestens einen Stromsteuerungsschalters umfasst.

## Revendications

**1.** Système (103, 113, 200, 250, 275, 300, 1200, 1400) de génération d'une impulsion électrothérapeutique à administrer à un patient, le système comprenant :

un condensateur de stockage d'énergie (223, 302, 352, 382, 1004, 1012, 1208, 1406, 1702, 1904, 2018, 3812) permettant de fournir un courant électrothérapeutique au patient ;
un réseau de commande de courant thérapeutique (202, 286, 238, 303, 351, 381, 1202), couplé électriquement au condensateur de stockage d'énergie, pour commander le courant électrothérapeutique à administrer au patient, le réseau de commande de courant thérapeutique comprenant un circuit électrique résonant (283) et au moins un commutateur de commande de courant (203, 224, 282, 1214, 1710), dans lequel l'énergie fournie par le condensateur de stockage d'énergie circule à travers le circuit électrique résonant ; et
un dispositif de commande (212, 233, 285, 313, 1220) couplé électriquement au condensateur de stockage d'énergie et au réseau de commande de courant thérapeutique, dans lequel le dispositif de commande est configuré pour, en relation avec l'énergie fournie par le condensateur de stockage d'énergie qui circule à travers le circuit électrique résonant,
commander le fonctionnement de l'au moins un commutateur de commande de courant du réseau de commande de courant thérapeutique lors de l'administration d'une forme d'onde électrothérapeutique au patient pour correspondre à une forme d'onde spécifiée.

**2.** Système (103, 113, 200, 250, 275, 300, 1200, 1400) selon la revendication 1, comprenant au moins un capteur configuré pour détecter au moins un paramètre électrique à partir duquel le flux de courant vers le patient peut être déterminé ou estimé, dans lequel le dispositif de commande (212, 233, 285, 313, 1220) est configuré pour :

traiter un signal associé à l'au moins un paramètre électrique détecté ;
comparer le signal traité avec un second signal associé à la forme d'onde spécifiée ; et
commander le fonctionnement de l'au moins un commutateur de commande de courant (203, 224, 282, 1214, 1710) du réseau de commande de courant thérapeutique (202, 286, 238, 303, 351, 381, 1202) en ajustant l'administration de la forme d'onde électrothérapeutique au patient pour correspondre à la forme d'onde spécifiée.

**3.** Système (103, 113, 200, 250, 275, 300, 1200, 1400) selon la revendication 1 ou 2, dans lequel l'au moins un commutateur de commande de courant (203, 224, 282, 1214, 1710) comprend une pluralité de commutateurs, et dans lequel la commande du fonctionnement de l'au moins un commutateur de commande de courant du réseau de commande de courant thérapeutique (202, 286, 238, 303, 351, 381, 1202) dans l'ajustement de l'administration de la forme d'onde électrothérapeutique au patient pour correspondre à la forme d'onde spécifiée comprend la commande d'une configuration de chacun de la pluralité de commutateurs comme étant ouvert ou fermé.

**4.** Système (103, 113, 200, 250, 275, 300, 1200, 1400) selon la revendication 1 ou 2, comprenant une batterie (215, 301, 1002, 1008, 1022, 1902, 3810) et un réseau de commande de charge bidirectionnel (216, 1010, 1020), dans lequel :

la batterie et le réseau de commande de charge bidirectionnel sont reliés par l'intermédiaire d'un premier nœud et d'un deuxième nœud, et
le réseau de commande de charge bidirectionnel et le condensateur de stockage d'énergie (223, 302, 352, 382, 1004, 1012, 1208, 1406, 1702, 1904, 2018, 3812) sont reliés par l'intermédiaire d'un troisième nœud et d'un quatrième nœud, dans lequel :

le réseau de commande de charge bidirectionnel sert à commander le flux d'énergie de la batterie vers le condensateur de stockage d'énergie pour le stockage par le condensateur de stockage d'énergie, et du condensateur de stockage d'énergie vers la batterie pour le stockage par la batterie.

5. Système (103, 113, 200, 250, 275, 300, 1200, 1400) selon la revendication 4, dans lequel le réseau de commande de charge bidirectionnel comprend un second circuit électrique résonant (283) comprenant un réservoir résonant.

6. Système (103, 113, 200, 250, 275, 300, 1200, 1400) selon la revendication 1 ou 2, dans lequel le dispositif de commande (212, 233, 285, 313, 1220) est configuré pour commander le fonctionnement de l'au moins un commutateur de commande de courant (203, 224, 282, 1214, 1710) du réseau de commande de courant thérapeutique (202, 286, 238, 303, 351, 381, 1202) pour décaler un moment d'initiation de la commutation de courant par rapport à un moment d'initiation de la commutation de tension, la commutation de tension étant associée à la commutation de courant, de manière à réduire les pertes de commutation par rapport à l'initiation sensiblement simultanée de la commutation de courant et de la commutation de tension associée.

7. Système (103, 113, 200, 250, 275, 300, 1200, 1400) selon la revendication 1 ou 2, dans lequel le dispositif de commande (212, 233, 285, 313, 1220) est configuré pour commander le fonctionnement de l'au moins un commutateur de commande de courant pour fournir une commutation de tension sensiblement nulle (ZVS) et une commutation de courant sensiblement nulle (ZCS).

8. Système (103, 113, 200, 250, 275, 300, 1200, 1400) selon la revendication 1 ou 2, dans lequel la forme d'onde spécifiée est une forme d'onde de courant rectiligne biphasique, et le système comprend au moins un commutateur de commande de polarité, dans lequel l'au moins un commutateur de commande de polarité est utilisé pour commander la direction du flux de courant lors de l'administration de la forme d'onde de courant rectiligne biphasique au patient.

9. Système (103, 113, 200, 250, 275, 300, 1200, 1400) selon la revendication 1 ou 2, dans lequel le circuit électrique résonant (283) est un réservoir résonant à deux éléments.

10. Système (103, 113, 200, 250, 275, 300, 1200, 1400) selon la revendication 9, dans lequel le réservoir résonant à deux éléments comprend un inducteur et un condensateur.

11. Système (103, 113, 200, 250, 275, 300, 1200, 1400) selon la revendication 1 ou 2, dans lequel le circuit électrique résonant (283) est un réservoir résonant à trois éléments.

12. Système (103, 113, 200, 250, 275, 300, 1200, 1400) selon la revendication 1 ou 2, dans lequel le circuit électrique résonant (283) est un réservoir résonant à quatre éléments.

13. Système (103, 113, 200, 250, 275, 300, 1200, 1400) selon la revendication 1 ou 2, dans lequel le circuit électrique résonant (283) est au moins l'un : d'un réservoir résonant LCC, d'un réservoir résonant CLL, d'un réservoir résonant LLC et d'un réservoir résonant LCLC.

14. Système (103, 113, 200, 250, 275, 300, 1200, 1400) selon la revendication 1 ou 2, dans lequel la forme d'onde électrothérapeutique est destinée à la défibrillation.

15. Système (103, 113, 200, 250, 275, 300, 1200, 1400) selon la revendication 1 ou 2, dans lequel la commande du fonctionnement de l'au moins un commutateur de commande de courant (203, 224, 282, 1214, 1710) du réseau de commande de courant thérapeutique (202, 286, 238, 303, 351, 381, 1202) dans l'ajustement de l'administration de la forme d'onde électrothérapeutique au patient pour correspondre à la forme d'onde spécifiée comprend la commande d'une fréquence de commutation de l'au moins un commutateur de commande de courant.

100

Network
101

102
Defibrillator

106

109
110

108

107

103

Therapeutic current
control system

104

105

FIG. 1A

FIG. 1B

EP 4 157 439 B1

**Therapeutic current control system**

FIG. 2A

EP 4 157 439 B1

200

**Therapeutic current control system**

201

202

Power
Supply

Therapeutic current
control network

203

204

205

206

Current
control
switch(es)

Resonant
tank

Rectifier

Filter

. . .

207

208

Patient

Electrical
parameter
sensor(s)

216

Closed loop
current
control

214

Specified
current waveform
(for matching/generating)

212

Controller

209

Signal(s) indicative of
current output to
patient

# FIG. 2B

FIG. 2C

300

Therapeutic current control system

I Sense 309

V Sense 310

Patient 317

Polarity Control 308

N15 N16

Filter 307

N13 N14

Rectifier 306

N11 N12

Q5 Q6

N5 N6

LLC resonant tank based **therapeutic current control network** 303

Resonant tank 305

Closed loop current control

N3

Q3 Q4

Cr

N4

316

Current control switch network

N1 Q1 Q2 N2

FPGA /DSP 315

Waveform Request 311

A/D 314

Host 312

I Sense

V Sense

Energy storage capacitor 302

Inverter

Battery

**Controller** 313

# FIG. 3

FIG. 4A

EP 4 157 439 B1

Example resonant tank based **therapeutic current control network** 381

LP Filter 386

Polarity Control 387

Patient 390

Current0 = -1.76211

SCOPE3

V_PAT0

R_PAT

R_PAT

QM

QN

To Controller 391

$x^2$

$\times$

$\int Z$

Total_Energy0

FIG. 4A
CONTINUED

FIG. 4B

Example resonant tank based
**therapeutic current control network**
351

Polarity Control
357

LP Filter
356

Current0 = -1.76211

Patient
358

SCOPE3

R_PAT

V_PAT0

R_PAT

$x^a$

QM

QN

359
To Controller

$\int Z$

Total_Energy0

FIG. 4B
CONTINUED

EP 4 157 439 B1

Example Controller
of closed loop
therapeutic current control system

FIG. 4C

500

**FIG. 5**

600

Example three element resonant tank
of a therapeutic current control network

$L_P$ separate from transformer

601

$L_R$

$C_P$    $L_P$

FIG. 6A

EP 4 157 439 B1

605

Example three element resonant tank
of a current control network

$L_P$ effectively supplied by transformer

FIG. 6B

610

Example three element resonant tank
of a current control network

**LCC resonant tank**

FIG. 6C

EP 4 157 439 B1

615

# Example three element resonant tank of a current control network

## **CLL resonant tank**

$C_R$

$L_R$

$L_P$

## FIG. 6D

EP 4 157 439 B1

Example three element resonant tank of a
Therapeutic current control network

FIG. 6E

EP 4 157 439 B1

Example three element resonant tank of a
Therapeutic current control network

FIG. 6F

# Example three element resonant tank
# of a therapeutic current control network

## $L_P$ separate from transformer

### LLC resonant tank

## FIG. 6G

**635** Example three element resonant tank
of a therapeutic current control network

$L_P$ effectively supplied by transformer

**LLC resonant tank**

FIG. 6H

Example three element resonant tank of a
Therapeutic current control network

FIG. 6I

645

Example three element resonant tank of a
Therapeutic current control network

FIG. 6J

650

Example three element resonant tank of a
Therapeutic current control network

FIG. 6K

EP 4 157 439 B1

655

Example three element resonant tank of a
Therapeutic current control network

$C_P$

$L_P$

$C_R$

FIG. 6L

Example three element resonant tank of a
Therapeutic current control network

FIG. 6M

665

Example three element resonant tank of a
Therapeutic current control network

FIG. 6N

EP 4 157 439 B1

670

Example three element resonant tank of a
Therapeutic current control network

FIG. 6O

EP 4 157 439 B1

675

Example three element resonant tank of a
Therapeutic current control network

FIG. 6P

680

Example three element resonant tank of a
Therapeutic current control network

FIG. 6Q

EP 4 157 439 B1

685

Example two element resonant tank of a
therapeutic current control network

FIG. 6R

EP 4 157 439 B1

690

Example two element resonant tank of a
therapeutic current control network

$C_R$

$L_R$

FIG. 6S

Example two element resonant tank of a
therapeutic current control network

FIG. 6T

EP 4 157 439 B1

692

Example two element resonant tank of a
therapeutic current control network

FIG. 6U

EP 4 157 439 B1

693

An example of a four element of a therapeutic
current control network

**LCLC resonant tank**

FIG. 6V

EP 4 157 439 B1

EP 4 157 439 B1

700

# Example Q1 – Q4 current control
# switch configurations

## No requested current (over time T1 – T2)

Time T1 — 701

| Q1 | Q3 | Resonant tank | Q5 |
| Q2 | Q4 | | Q6 |

702

Time T2

| Q1 | Q3 | Resonant tank | Q5 |
| Q2 | Q4 | | Q6 |

703

| Q1 (closed) | Q1 (open) |
| Q2 (open) | Q2 (closed) |
| Q3 (closed) | Q3 (open) |
| Q4 (open) | Q4 (closed) |
| Q5 (closed) | Q5 (closed) |
| Q6 (closed) | Q6 (closed) |

FIG. 7A

750   Example current control
switch configurations
**Full requested current (over time T1 – T2)**

FIG. 7B

EP 4 157 439 B1

**775  Example Q1-Q4 signaling – partial requested current**

Switch closed /turned on

Switch open / turned off

Current

Q4  Q1

Q4  Q1

Q4  Q1

Q4  Q1

Current

Q3  Q2

Q3  Q3  Q2

Q3  Q2

Q3  Q2

Q3  Q2

Time ➔

FIG. 7C

**Example – partial requested current**

Current (amps)

Energy (Joules)

Current (amps)

Q4  Q1  Q4  Q1  Q1  Q4  Q1  Q4  Q1

Current (amps)

Q3  Q2  Q3  Q2  Q3  Q2  Q3  Q2  Q3  Q2

Time (milliseconds)

FIG. 7D

Example – no requested current

FIG. 7E

# FIG. 7F

**Example – full requested current**

Current (amps)

Energy (joules)

Current (amps)

Q4 signal

Q1 signal

Current (amps)

Q3 signal

Q2 signal

Time (milliseconds) ⟶

EP 4 157 439 B1

800

Hard switching – simplified depiction

FIG. 8

**Soft switching – simplified depiction**

FIG. 9

EP 4 157 439 B1

## Capacitor charging with energy "dump"

1000

1002

Battery

Energy storage Capacitor

1004

1006

Energy "dump" at resistor(s)

1014

## Capacitor charging / battery charging with bidirectionality

1008

Battery

Bidirectional charging control network

1010

Energy storage Capacitor

1012

# FIG. 10A

EP 4 157 439 B1

EP 4 157 439 B1

1020

**Bidirectional charging control network**

FIG. 10B

**Bidirectional charging control network**
**Secondary side – full bridge switching mode**

Switch set 1
(engaged)
1028

1030
Switch set 2
(not engaged)

# FIG. 10C

EP 4 157 439 B1

**Bidirectional charging control network
Secondary side – half bridge switching mode**

Switch set 1
(not engaged)
1028

1030
Switch set 2
(engaged)

FIG. 10D

Generated current waveform
to match a rectilinear biphasic current waveform
(**200 joules** at 50 ohms)

Current (I) - Reference/specified current waveform

Current (I) - Generated current waveform

FIG. 11A

Example - 200J at 50 ohms

1126

1125

Current (amps)

Energy (joules)

Time (msec)

FIG. 11B

99

Generated current waveform
to match a rectilinear biphasic current waveform
(**1 joule** at 50 ohms)

FIG. 11C

Example - 1J at 50 ohms

1138

1137

1136

1135

Current (amps)

Energy (joules)

Time (msec)

FIG. 11D

Generated current
waveform
to match a rectilinear
biphasic current
waveform
(**1 joule** at 50 ohms)

[Section Close-Up]

# FIG. 11E

Generated waveform
to match a pulsed biphasic current waveform
(200 joules at 50 ohms)

1104

Current (I) - Reference/specified current waveform

Current (I) - Generated current waveform

Time
(msec)

I
(amps)

FIG. 11F

Generated waveform
to match a pulsed biphasic current waveform
(200 joules at 50 ohms)

Current (I) - Reference/specified current waveform

Current (I) - Generated current waveform

FIG. 11G

Example - 200J at 200
ohms

Current
(amps)

1130

Energy
(joules)

Time
(msec)

FIG. 11H

EP 4 157 439 B1

EP 4 157 439 B1

Example - 200J at 50 ohms

FIG. 11I

Example: Reference signal Sine (30*sin(wt))

FIG. 11J

EP 4 157 439 B1

**Reference signal Ramp (a\*t))**

Current
(amps)

1155

Energy
(joules)

Time
(msec)

# FIG. 11K

1200

Quasi-resonant current control system

FIG. 12

EP 4 157 439 B1

1302

1300

Controller receives reference waveform from waveform generator

1304

Controller initiates delivery of current waveform to patient in accordance with reference waveform

1306

Controller controls switching frequency to adjust current waveform during delivery in accordance with reference waveform

1308

Current drawn from power supply is sensed

1310

Controller uses sensed current drawn from power supply in determining necessary adjustment to switching frequency

1312

Controller adjusts switching frequency according to determined adjustment

FIG. 13

FIG. 14

<u>1500</u>

1502 → MOSFET switch M1 switches on

1504 → Resonant tank inductor (Lres) current increases, sourced by current in filter inductor (Lfilt)

1506 → Resonant tank inductor (Lres) current continues increasing, sourced by resonant tank capacitor (Cres), until voltage on resonant tank capacitor (Cres) drops to zero

1508 → Current in resonant tank inductor (Lres) falls, reaching zero when voltage on resonant tank capacitor (Cres) is approx. equal and opposite to voltage on the energy storage capacitor

1510 → Current control switch (M1) switches off
(at zero current flow, for zero current switching)

1512 → Filter inductor (Lfilt) energy increases, sourced by energy in resonant tank capacitor (Cres), and then energy is transferred back from the filter inductor (Lfilt) to the resonant tank capacitor (Cres).

1514 → Current in filter inductor (Lfilt) persists, flowing through diode D1 and the patient load

## FIG. 15

FIG. 16

FIG. 17

1706 Filter

1708 Resonant tank

Current control HV MOSFET switch

1710 Switch driver (cascode config)

1712

1714 Zero current detector

Current 1716 Sense resistor

1718 Current sense amplifier

1700

1702 Patient load

1704 Polarity control switches

Energy storage capacitor 1702

1726 Switch timing latch

Low pass filter 1719

1720 Servo loop control (or other, e.g. FPGA, DSP based)

Reference 1722 Waveform generator

Voltage controlled 1724 oscillator

1800

FIG. 18

Example Patient relay circuit 3600

FIG. 19

Example
Patient relay
circuit
3700

FIG. 20

3702

3704

FIG. 21

FIG. 22

Example transformer circuit
2002

Cd 24pF

Rp 3m

LLp 120nH

Ce 7.7nF

Re 90

Le 1.94µH

Rs_dcr 3.6m

LLs_p 105nH

pri_top

sec_top

Bideal_pri
I=29*I(Bideal_sec)

Bideal_sec
V=-29*V(pri_top,pri_bot)

pri_bot

sec_bot

**FIG. 23**

# FIG. 24

Example primary side switch

<u>2004</u>

EP 4 157 439 B1

Example primary side
active clamp

2006

FIG. 25

Example primary side gate driver 2008

FIG. 26

EP 4 157 439 B1

Example secondary
side switch
2010

# FIG. 27

Example secondary side gate
driver

2012

# FIG. 28

Example secondary side
passive clamp

2014

FIG. 29

EP 4 157 439 B1

Forward charging path

FIG. 30

EP 4 157 439 B1

Forward discharging path

FIG. 31

Reverse charging path

FIG. 32

Reverse discharging path

FIG. 33

FIG. 34

EP 4 157 439 B1

FIG. 35

## CCM / BCM

3402

FIG. 36

EP 4 157 439 B1

supply_i_sense

R5

2Ω

C9 10µF

Htmcs1100 50m

Vtmcs1100 0

Example
current
sensing circuit
2010

FIG. 37

FIG. 38

FIG. 39

EP 4 157 439 B1

FIG. 40

FIG. 41

FIG. 42

EP 4 157 439 B1

FIG. 43

FIG. 44

EP 4 157 439 B1

FIG. 45

Forward mode

4602

Reverse mode

4604

Battery
current
**4606**

Alternating
Current
through **I**
transformer
**4608**

Energy
storage **I**
capacitor
voltage
**4610**

**V**

**Time**

FIG. 46

EP 4 157 439 B1

4700

FIG. 47

FIG. 48

**EP 4 157 439 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1535645 A1
- US 2001031991 A1
- EP 0712321 A1
- US 2003074025 A1
- JP H1057508 A